# EUROPEAN PATENT APPLICATION

(11) **EP 1 600 452 A2**
(43) Date of publication of application: **30.11.2005**
(21) Application number: 05076621.1
(22) Date of filing: 13.11.2000
(51) Int. Cl.: C07H 1/06, C07H 19/06, C07H 19/16, C12N 15/10, A61K 31/7052, A61P 31/12

(54) **Synthesis of 2'-deoxy-L-nucleosides**

(30) Priority: 12.11.1999 US 165087 P
(62) Divisional of application: 00977183.3
(71) Applicant: Pharmasset Limited, Tucker, GA 30084 (US)
(72) Inventor: Watanabe, Kyoichi A., Stone Mountain, GA 30087 (US); Choi, Woo-Baeg, Atlanta, GA 30339 (US)
(74) Representative: Hallybone, Huw George

(57) **Abstract**

The present invention provides a process for the preparation of a 2'-deoxy-L-nucleoside comprising the following steps:
a) preparing from a suitably protected and activated L-furanose a 2-S-substituted-2-deoxy-L-furanose of the following formula: 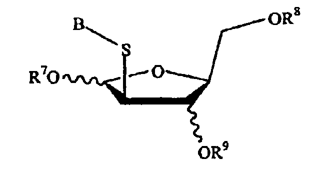 wherein B is a heterocyclic or heteroaromatic base, R⁸ and R⁹ are independently hydrogen or a suitable protecting group; R⁷ is a suitable protecting group;
b) cyclizing the 2-S-substituted-2-deoxy-L-furanose to form a cyclonucleoside of the following formula: 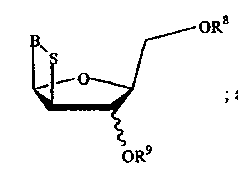 and
c) reducing the cyclonucleoside to a 2'-deoxy-L-nucleoside.

## Description

### Background of the Invention

This application is in the area of pharmaceutical chemistry and is a process for producing 2'-deoxy-L-nucleosides that have activity against human immunodeficiency virus, hepatitis B virus, hepatitis C virus and abnormal cell proliferation, and products and compositions prepared according to this process.

This application claims priority to U.S.S.N. 60/165,087, entitled "Synthesis of 2'-Deoxy-L- Nucleosides" by Woo-Baeg Choi and Kyoihi A. Watanabe, filed on November 12, 1999.

### Human Immunodeficiency Virus

A virus that causes a serious human health problem is the human immunodeficiency virus (HIV). In 1981, acquired immune deficiency syndrome (AIDS) was identified as a disease that severely compromises the human immune system, and that almost without exception leads to death. In 1983, the etiological cause of AIDS was determined to be the human immunodeficiency virus (HIV). In 1985, it was reported that the synthetic nucleoside 3'-azido-3'-deoxythymidine (AZT) inhibits the replication of human immunodeficiency virus. Thereafter, a number of other synthetic nucleosides, including 2',3'-dideoxyinosine (DDI), 2',3'-dideoxycytidine (DDC), and 2',3'-dideoxy-2',3'-didehydrothymidine (D4T), have been proven to be effective against HIV. After cellular phosphorylation to the 5'-triphosphate by cellular kinases, these synthetic nucleosides are incorporated into a growing strand of viral DNA, causing chain termination due to the absence of the 3'-hydroxyl group. They can also inhibit the viral enzyme reverse transcriptase.

The success of various synthetic nucleosides in inhibiting the replication of HIV *in vivo* or *in vitro* has led a number of researchers to design and test nucleosides that substitute a heteroatom for the carbon atom at the 3'-position of the nucleoside. European Patent Application Publication No. 0 337 713 and U.S. Patent No. 5,041,449, assigned to BioChem, Pharma, Inc., disclose racemic 2-substituted-4-substituted-1,3-dioxolanes that exhibit antiviral activity. U.S. Patent No. 5,047,407 and European Patent Application No. 0 382 526, also assigned to BioChem Pharma, Inc., disclose that a number of racemic 2-substituted-5-substituted-1,3-oxathiolane nucleosides have antiviral activity, and specifically report that the racemic mixture of 2-hydroxymethyl-5-(cytosin-1-yl)-1,3-oxathiolane (referred to below as BCH-189) has approximately the same activity against HIV as AZT, with little toxicity. The (-)-enantiomer of the racemate BCH-189, known as 3TC, which is covered by U.S. Patent No. 5,539,116 to Liotta *et al*., is currently sold for the treatment of HIV in combination with AZT in humans in the U.S.

It has also been disclosed that cis-2-hydroxymethyl-5-(5-fluorocytosin-1-yl)-1,3-oxathiolane ("FTC") has potent HIV activity. Schinazi, *et al*., "Selective Inhibition of Human Immunodeficiency viruses by Racemates and Enantiomers of cis-5-Fluoro-1-[2-(Hydroxymethyl)-1,3-Oxathiolane-5-yl]Cytosine" *Antimicrobial Agents and Chemotherapy,* **1992**,2423-2431. See also U.S. Patent No. 5,210,085; WO 91/11186, and WO 92/14743.

### Hepatitis B

In western industrialized countries, high risk groups for HBV infection include those in contact with HBV carriers or their blood samples. The epidemiology of HBV is very similar to that of acquired immune deficiency syndrome, which accounts for why HBV infection is common among patients infected with HIV or AIDS. However, HBV is more contagious than HIV.

Infection by hepatitis B virus is a problem of enormous dimensions. It is estimated that as many as 300 million people worldwide are persistently infected with HBV, many of whom develop associated pathologies such as chronic hepatic insufficiency, cirrhosis, and hepatocellular carcinoma. In the United States 200,000 new cases of HBV infection occur annually (Zakim, D.; Doyer, T.D. Eds, "Hepatology: A Textbook of Liver Disease", W. B. Saunders Publ., Philadelphia, **1982;** Vyas, G., Ed., "Viral Hepatitis and Liver Disease", Grune and Stratton Publ., **1984).** About 1-2% of these develop fulminant hepatitis with a mortality rate of 60-70%. Six to ten percent of infected patients progress to chronic active hepatitis. The virus has been the target of extensive investigation into the basic biology and molecular biology. Recent years have seen vigorous activity directed towards the development of effective therapeutic agents. However certain unusual features of the biology of the virus make this a particularly challenging problem.

The primary goal of treatment of patients with persistent viral replication (the most likely to die of liver disease) is the inhibition of replication. The presence of a viral reservoir in the form of non replicating mini-chromosomes, which cannot be directly attacked, makes complete cures of the infection quite difficult, and necessitates fairly lengthy times of treatment. The hope of the therapy is to suppress viral replication for a sufficiently long period such that the minichromosome reservoir might be depleted by natural turnover in the absence of replenishment. Treatments that fail to reduce the reservoir are marked by a rapid rebound in viral burden upon termination of treatment. A number of therapies, many of them anti replicative nucleoside analogues, have been tested in experimental animal models and/or in human clinical trials.

Both FTC and 3TC exhibit activity against HBV. Furman, *et al*., "The Anti-Hepatitis B Virus Activities, Cytotoxicities, and Anabolic Profiles of the (-) and (+) Enantiomers of cis-5-Fluoro-1-[2-(Hydroxymethyl)-1,3-oxathiolane-5-yl]-Cytosine" *Antimicrobial Agents and Chemotherapy,* December 1992, pp. 2686-2692; and Cheng, *et al*., *Journal of Biological Chemistry,* Volume 267(20), pp.13938-13942 (1992).

Alpha interferon has received extensive clinical application in HBV treatment (Perrillo, R. P.; Schiff, E. R.; Davis, G. L.; Bodenheimer, H. C.; Lindsay, K.; Payne, J.; Dienstag, J. L.; O'Brien, C.; Tamburro, C.; Jacobson, 1. M.; Sampliner, R.; Feit, D.; Lefkovwitch, J.; Kuhns, M.; Meschievitz, C.; Sanghvi, B.; Albrecht, J.; Gibas, A. *N. Eng. J. Med*. **1990,** *323,* 295-301). However, efficacy has been shown largely in patients with a low HBV level, lack of cirrhosis, and less than 10 years of infection (Perrillo, R. P.; Schiff, E. R.; Davis, G. L.; Bodenheimer, H. C.; Lindsay, K.; Payne, J.; Dienstag, J. L.; O'Brien, C.; Tamburro, C.; Jacobson, I. M.; Sampliner, R.; Feit, D.; Lefkovwitch, J.; Kuhns, M.; Meschievitz, C.; Sanghvi, B.; Albrecht, J.; Gibas, A. *N*. *Eng. J. Med.* **1990,** 323, 295-301). Consequently, the majority of patients do not benefit and there are limiting side effects as well.

The fluorinated D-nucleoside FIAU was found to have potent activity against HBV (Hantz, O. Allaudeen, H. S.; Ooka, T.; De Clercq, E.; Trepo, C. *Antiviral Res.* **1984,** *4,* 187-199; Hantz, O.; Ooka, T.; Vitvitski, L.; Pichoud, C.; Trepo, C. *Antimicrob. Agents* *Chemother*. **1984,** *25,* 242-246). FIAU was administered to HBV patients in three clinical trials. In the first two, with two and four week courses of FIAU there was a quick suppression of serum HBV DNA levels by as much as 95% (Paar, D. P.; Hooten, T. M.; Smiles, K. A.; Abstracts of the 32^{nd} Interscience Conference on Antimicrobial Agents and Chemotherapy **1992**, Abstract #264). In several of the patients, there was a sustained loss of viral DNA (Fried, M. W.; DiBisceglie, A. M.; Straus, S. E.; Savalese, B.; Beames, M. P.; Hoofnagle, J. H. *Hematology* **1992,** *16*, 127A) after termination of the trial. Thus, in these patients, the drug appeared to clear the virus without the rebound that customarily follows other treatments.

However, in a prolonged treatment trial with 15 patients (Macilwain, C. *Nature,* **1993**, *364*, 275; Touchette, N. *J. NIH Res*., **1993**, *5*, 33-35.; McKenzie, R.; Fried, M. W.; Sallie, R.; Conjeevaram, H.; Di Bisceglie, A. M.; Park, Y.; Savarese, B.; Kleiner, D.; Tsokos, M.; Luciano, C.; Pruett, T.; Stotka, J. L.; Straus, S. E.; Hoofnage, J. H. *New Eng. J. Med*., **1995,** *333*, 1099-1105), delayed hepatotoxicity was recognized in 7 patients, 5 of whom died from hepatic failure. The accompanying lactic acidosis, peripheral neuropathy, myopathy, as well as subsequent cellular studies showed that the toxicity was due to mitochondrial injury (Parker, W. B.; Cheng, Y-C. *J. NIH Res*. **1994,** *6*, 57-61.; Lewis, W.; Dalakas, M. C. Mitochondrial toxicity of antiviral drugs *Nature Medicine,* **1995,** *1*, 417-422). Experiments with purified mitochondrial polymerase γ demonstrated that the polymerase had a higher affinity for the drug than other cellular polymerases, and that FIAU was incorporated into mitochondrial DNA (Lewis, W.; Meyer, R. R.; Simpson, J. F.; Colacino, J. M.; Perrino, F. M. *Biochemistry* **1994,** *33*, 14620-14624.). Since there was no mechanism for removal (Klecker, R. W.; Katki, A. G.; Collins, J. M. *Mol. Pharmacol*. **1994,** *46,* 1204-1209.), it is generally believed that the toxicity was due to either effects on mitochondrial DNA transcription or, perhaps, the formation of mutant proteins encoded by the substituted mitochondrial DNA (Parker, W. B.; Cheng, Y-C. *J. NIH Res.* **1994,** *6*, 57-61.; Lewis, W.; Dalakas, M. C. Mitochondrial toxicity of antiviral drugs *Nature Medicine*, **1995,** *1*, 417-422.).

In order to reduce the toxicity of 2'-fluorinated D-nucleosides while maintaining the antiviral activity, Chu et al. (Chu, C. K.; Ma., T-W.; Shanmuganathan, K.; Wang, C-G.; Xiang, Y-J.; Pai, S. B.; Yao, G-Q.; Sommadossi, J-P.; Cheng, Y-C. *Antimicrob. Agents Chemother.* **1995,** *39,* 979-981) synthesized L-FMAU and found that it exhibited potent in vivo activity against woodchuck hepatitis virus (WHV) (Tennant, B.; Jacob, J.; Graham, L. A.; Peek, S.; Du, J.; Chu, C. K. *Antiviral Res.* **1996,** *34 (A52),* 36) and duck hepatitis B virus (Aguesse-Germon, S.; Liu, S-H.; Chevallier, M.; Pichaud, C.; Jamard, C.; Borel, C.; Chu, C. K.; Trepo, C.; Cheng, Y-C., Zoulim, F. *Antimicrob. Agents. Chemother.* **1998,** *42***.** 369-376). The toxic effects of L-FMAU were far less than those of the D-counterpart. L-FMAU did not adversely affect mitochondrial function at a concentration of 200 µM in hepatoma cell lines and no significant lactic acid production was observed (Pai, S. P.; Liu, S-H.; Zhu, Y-L.; Chu, C. K.; Cheng, Y-C. *Antimicrob. Agents. Chemother.* **1996***, 40,* 380-386)*.*

Very recently L-counterparts of all four DNA constituents were tested for their anti-HBV activity in HBV-transfected HepG2 cells (2.2.15 cells) at Novirio Pharmaceuticals, Inc. See WO 00/09531 entitled *β*-L-2'-Deoxynucleosides for the Treatment of Hepatitis B," by Novirio Limited and Centre National Da La Recherche Scientifique. 2'-Deoxy-β-L-thymidine (L-dThd), 2'-deoxy-β-L-cytidine (L-dCyd) and 2'-deoxy-β-L-adenosine (L-dAdo) were active at sub-micromolar concentrations (ED₅₀ ≤ 0.01 µM). No toxicity was observed in uninfected HepG2 cells when these L-nucleosides were tested at up to 200 µM (ID₅₀ > 200 µM, making the therapeutic index of > 20,000). Lamivudine or 3TC, used as the positive control in the assay, has a median effective concentration (EC₅₀) of 0.05 µM. The three L-nucleosides described above have comparable activity to 3TC. Also, these L-nucleosides exhibit specific activity against HBV, and not HIV. L-dThd, L-dCyd and L-dAdo have no effect on mitochondrial DNA synthesis and lactic acid production. Additionally, these L-nucleosides demonstrated no morphological changes in HepG2 cells when treated at up to 100 µM*.*

L-dThd is phosphorylated by thymidine kinase and deoxycytidine kinase, L-dCyd is phosphorylated by deoxycytidine kinase, and L-dAdo is phosphorylated by an unknown kinase. At Novirio, it was discovered that though substrates of cellular kinases, these nucleosides seem to have little, if any, substrate activity for polymerase γ which is responsible for mitochondrial DNA chain elongation. Thus, they showed no effect on mitochondrial functions.

L-Thymidine (L-dThd) was originally synthesized in 1964 (Smejkal, J.; Sorm, F. *Coll. Czech. Chem. Commun*. **1964,** *29,* 2809-2813) by a Czech group. Later, Holy *et al* synthesized several 2'-deoxy-L-nucleosides including L-dThd (Holy, A. *Coll. Czech. Chem*. *Commun*. **1972,** *37*,4072-4082).

### Hepatitis C

Hepatitis C virus ("HCV") is the major causative agent of post-transfusion and of sporadic non A, non B hepatitis (Alter, H. J. *J. Gastro. Hepatol.* (**1990**) *1*, 78-94; Dienstag, J. L*. Gastro* (1983) 85, 439-462). Despite improved screenings, HCV still accounts for at least 25% of the acute viral hepatitis in many countries (Alter, H. J. (1990) *supra*; Dienstag, J. L. (1983) *supra*; Alter M. J. *et al*. (1990a) *J.A.M.A*. 264:2231-2235; Alter M. J. *et al* (1992) *N. Engl. J. Med.* 327:1899-1905; Alter, M. J. *et al*. (1990b) *N. Engl. J. Med.* 321:1494-1500). Infection by HCV is insidious in that a high proportion of chronically infected (and infectious) carriers may not experience clinical symptoms for many years. The high rate of progression of acute infection to chronic infection (70-100%) and liver disease (>50%), its world-wide distribution and lack of a vaccine make HCV a significant cause of morbidity and mortality.

### Tumors

A tumor is an unregulated, disorganized proliferation of cell growth. A tumor is malignant, or cancerous, if it has the properties of invasiveness and metastasis. Invasiveness refers to the tendency of a tumor to enter surrounding tissue, breaking through the basal laminas that define the boundaries of the tissues, thereby often entering the body's circulatory system. Metastasis refers to the tendency of a tumor to migrate to other areas of the body and establish areas of proliferation away from the site of initial appearance.

Cancer is now the second leading cause of death in the United States. Over 8,000,000 persons in the United States have been diagnosed with cancer, with 1,208,000 new diagnoses expected in 1994. Over 500,000 people die annually from the disease in this country.

Cancer is not fully understood on the molecular level. It is known that exposure of a cell to a carcinogen such as certain viruses, certain chemicals or radiation, leads to DNA alteration that inactivates a "suppressive" gene or activates an "oncogene." Suppressive genes are growth regulatory genes which, upon mutation, can no longer control cell growth. Oncogenes are initially normal genes (called prooncongenes) that, by mutation or altered context of expression, become transforming genes. The products of transforming genes cause inappropriate cell growth. More than twenty different normal cellular genes can become oncogenes by genetic alteration. Transformed cells differ from normal cells in many ways, including cell morphology, cell-to-cell interactions, membrane content, cytoskeletal structure, protein secretion, gene expression and mortality (transformed cells can grow indefinitely).

All of the various cell types of the body can be transformed into benign or malignant tumor cells. The most prevalent type of cancer is lung, followed by colorectal, breast, prostate, bladder, pancreas, and then ovarian cancers. Other prevalent types of cancer include leukemia, central nervous system cancers, including brain cancer, melanoma, lymphoma, erythroleukemia, uterine cancer and head and neck cancer.

Cancer is now primarily treated with one or a combination of therapies, including surgery, radiation, and chemotherapy. Surgery involves the bulk removal of diseased tissue. While surgery is sometimes effective in removing tumors located at certain sites, for example, in the breast, colon and skin, it cannot be used in the treatment of tumors located in other areas such as the backbone, nor in the treatment of disseminated neoplastic conditions such as leukemia.

Chemotherapy involves the disruption of cell replication or cell metabolism. It is used most often in the treatment of leukemia, as well as breast, lung and testicular cancer.

There are five major classes of chemotherapeutic agents currently in use for the treatment of cancer are natural products and their derivatives, anthacyclines, alkylating agents, antiproliferatives (also called antimetabolites) and hormonal agents. Chemotherapeutic agents are often referred to as antineoplastic agents.

It is an object of the present invention to provide methods for the manufacture of the pharmaceutically important 2'-deoxy-L-nucleosides from readily available sugars. In addition, this invention discloses methods to prepare β-L-nucleosides from α-D-nucleosides.

It is a further objective of the present invention to provide new compounds and methods for the treatment of HIV.

It is another objective of the present invention to provide new compounds and methods for the treatment of HBV.

It is still another object of the present invention to provide new compounds and methods for the treatment of HCV.

It is yet a further objective of the present invention to provide new compounds and methods for the treatment of tumors, including cancer.

### Summary of the Invention

A process for the manufacture of a compound of formula (A) is provided, wherein (A) has the formula: 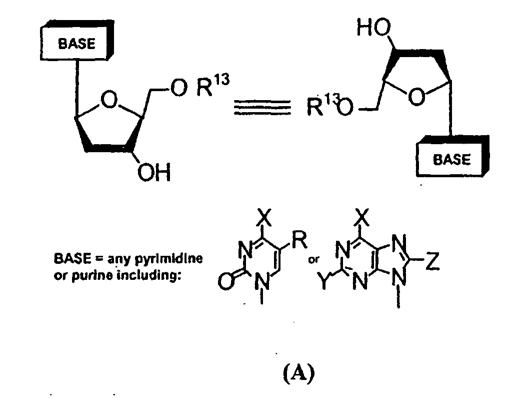 wherein
X and Y are independently hydrogen, OH, OR¹, SH, SR¹, NH₂, NHR¹ or NR¹R²;
Z is hydrogen, halogen, CN or NH₂;
R is hydrogen, lower alkyl, aralkyl, halogen, NO₂, NH₂, NHR³, NR³R⁴, OH, OR³, SH, SR³, CN, CONH₂, CSNH₂, CO₂H, CO₂R³, CH₂CO₂H, CH₂CO₂R³, CH=CHR³, CH₂CH=CHR³ or C≡CR³;
R¹, R², R³ and R⁴ are independently a lower alkyl, e.g., methyl, ethyl, propyl, butyl, and alkyl possessing 6 or less carbons, in cyclic, branched or straight chains, unsubstituted or substituted wherein the alkyl bears one, two, or more substituents, including but not limited to, amino, carboxyl, hydroxy and phenyl;
R¹³ is hydrogen, alkyl, acyl, phosphate (monophosphate, diphosphate, triphosphate, or stabilized phosphate) or silyl; and
which can be prepared from one of the following starting materials: L-ribose, L-xylose, L-arabinose, D-arabinose or a nucleoside with a natural β-D-glycosyl configuration.

In one embodiment, the synthesis of a 2'-deoxy-L-nucleoside includes selectively activating the 2'-position of a L-nucleoside to O-LG, halogen or S(=O)ₘR⁶ wherein O-LG is the following: 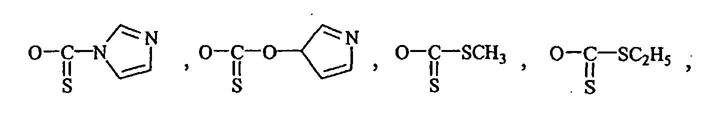 O-S(=O)ₙ-R⁵ or O-C(=O)-R⁵;
R⁵ is a hydrogen, an alkyl or aryl moiety;
R⁶ is an alkyl or aryl;
n is 1 or 2; and m is 0, 1 or 2; and
then subsequently reducing the formed product to give the desired 2'-deoxy-L-nucleoside.

In another embodiment, the synthesis of a 2'-deoxy-L-nucleoside includes preparing a 2-S-substituted-2-deoxy-L-furanose of the following formula: 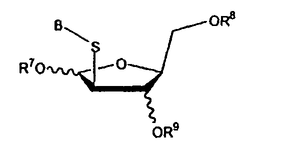 wherein B is a heterocyclic or heteroaromatic base;
R⁷, R⁸ and R⁹ are independently hydrogen or a suitable protecting group;
that includes cyclizing the 2-S-substituted-2-deoxy-L-furanose to form a cyclonucleoside of the following formula: 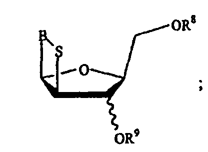 and
then reducing the cyclonucleoside to a 2'-deoxy-L-nucleoside.

In yet another embodiment, the synthesis of a 2'-deoxy-L-nucleoside includes preparing from a suitably protected and activated L-nucleoside a 2'-carbonyl-L-nucleoside of the following formula: 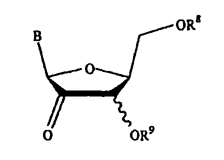 wherein B, R⁸ and R⁹ are defined above; and
reducing the 2'-carbonyl-L-nucleoside to a 2'-deoxy-nucleoside.

In an alternate embodiment, the synthesis of a 2'-deoxy-L-nucleoside includes epimerizing the 4' moiety of a 2'-deoxy-α-D-nucleoside, using a process described in detail below.

In a further embodiment, the synthesis of a 2'-deoxy-α-D-nucleoside includes selectively activating the 2'-position of a L-nucleoside to O-LG, halogen or S(=O)ₘR⁶ and subsequently reducing the formed compound to give the corresponding 2'-deoxy-α-D-nucleoside.

In another embodiment, the synthesis of a 2'-deoxy-L-nucleoside containing a purine or pyrimidine base is presented that includes base substitution of a β-L-nucleoside containing a different base.

### Detailed Description of the Invention

The invention as disclosed herein is a process to produce compounds of formula (A). 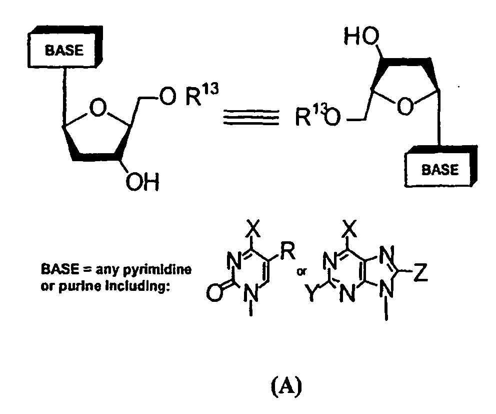 wherein
X and Y are independently hydrogen, OH, OR¹, SH, SR¹, NH₂, NHR¹ or NR¹R²;
Z is hydrogen, halogen, CN or NH₂;
R is hydrogen, lower alkyl, aralkyl, halogen, NO₂, NH₂, NHR³, NR³R⁴, OH, OR³, SH, SR³, CN, CONH₂, CSNH₂, CO₂H, CO₂R³, CH₂CO₂H, CH₂CO₂R³, CH=CHR³, CH₂CH=HR³ or C≡CR³;

R¹, R², R³ and R⁴ are independently a lower alkyl, e.g., methyl, ethyl, propyl, butyl, and alkyl possessing 6 or less carbons, in cyclic, branched or straight chains, unsubstituted or substituted wherein the alkyl bears one, two, or more substituents, including but not limited to, amino, carboxyl, hydroxy and phenyl; and

R¹³ is hydrogen, alkyl, acyl, phosphate (monophosphate, diphosphate, triphosphate, or stabilized phosphate) or silyl.

In one embodiment, the use of these compounds for the treatment of HIV, hepatitis (B or C), or abnormal cellular proliferation, in humans or other host animals is provided, that includes administering an effective amount of a 2'-deoxy-L-nucleoside. The compounds of this invention either possess antiviral (i.e., anti-HIV-1, anti-HIV-2, or anti-hepatitis (B or C)) activity, or antiproliferative activity, or are metabolized to a compound that exhibits such activity.

In summary, the present invention includes the following features:
(a) processes for the production of 2'-deoxy-L-nucleosides, as described herein, and pharmaceutically acceptable prodrugs and salts thereof;
(b) certain 2'-deoxy-L-nucleosides, as described herein, and pharmaceutically acceptable prodrugs and salts thereof for use of medical therapy, for example for the treatment or prophylaxis of an HIV or hepatitis (B or C) infection or for the treatment of abnormal cellular proliferation;
(c) use of certain 2'-deoxy-L-nucleosides, and pharmaceutically acceptable prodrugs salts thereof in the manufacture of a medicament for treatment of an HIV or hepatitis infection (B or C) or for the treatment of abnormal cellular proliferation; and
(d) pharmaceutical formulations comprising certain 2'-deoxy-L-nucleosides or a pharmaceutically acceptable derivative or salt thereof together with a pharmaceutically acceptable carrier or diluent.

Specifically, this invention provides processes for the preparation of a compound having the structure: 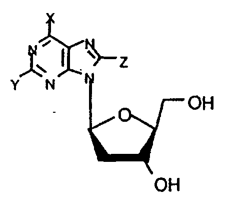 wherein
X and Y are independently hydrogen, OH, OR¹, SH, SR¹, NH₂, NHR¹ or NR¹R².
Z is hydrogen, halogen, OH, OR⁵, SH, SR⁵, CN, NH₂, NHR⁵ or NR⁵R⁶.
R¹, R², R⁵ and R⁶ are independently a lower alkyl, e.g., methyl, ethyl, propyl, butyl, and alkyl possessing 6 or less carbons, in cyclic, branched or straight chains, unsubstituted or substituted wherein the alkyl bears one, two, or more substituents, including but not limited to, amino, carboxyl, hydroxy and phenyl.

The present invention also provides processes for synthesizing a compound having the structure: 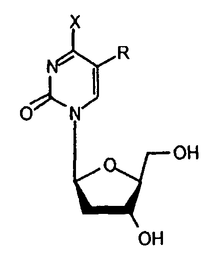 wherein X is defined above.
R is hydrogen, lower alkyl, aralkyl, halogen, NO₂, NH₂, NHR³, NR³R⁴, OH, OR³, SH, SR³, CN, CONH₂, CSNH₂, CO₂H, CO₂R³, CH₂CO₂H, CH₂CO₂R³, CH=CHR³, CH₂CH=CHR³ or C≡CR³.

R¹, R², R³ and R⁴ are independently a lower alkyl, e.g., methyl, ethyl, propyl, butyl, and alkyl possessing 6 or less carbons, in cyclic, branched or straight chains, unsubstituted or substituted wherein the alkyl bears one, two, or more substituents, including but not limited to, amino, carboxyl, hydroxy and phenyl.

This invention further provides a method of treating a mammal having a virus associated disorder, and in particular HIV or hepatitis (B or C) which comprises administering to the mammal a pharmaceutically effective amount of a compound having the structure: 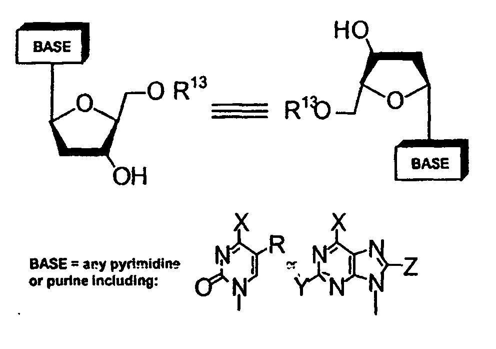 wherein X, Y, Z, R, R¹, R², R³, R⁴, R⁵, R⁶ and R¹³ are defined above.
Formula (A) includes but is not limited to the following compounds:
1-(2-deoxy-*β*-L-*erythro*pentofuranosyl)-5-ethyluracil,
1-(2-deoxy-*β*-L-*erythro*pentofuranosyl)-5-propyluracil,
1-(2-deoxy-*β*-L-*erythro*pentofuranosyl)-5-phenyluracil,
1-(2-deoxy-β-L-*erythro*pentofuranosyl)-5-benzyluracil,
1-(2-deoxy-β-L-*erythro*pentofuranosyl)-5-fluorouracil,
1-(2-deoxy-β-L-*erythro*pentofuranosyl)-5-chlorouracil,
1-(2-deoxy-*β*-L-*erythro*pentofuranosyl)-5-bromouracil,
1-(2-deoxy-*β*-L-*erythro*pentofuranosyl)-5-iodouracil,
1-(2-deoxy-*β*-L-*erythro*pentofuranosyl)-5-nitrouracil,
1-(2-deoxy-*β*-L-*erythro*pentofuranosyl)-5-aminouracil,
1-(2-deoxy-*β*-L-*erythro*pentofuranosyl)-5-methylaminouracil,
1-(2-deoxy-*β*-L-*erythro*pentofuranosyl)-5-ethylaminouracil,
1-(2-deoxy-β-L-*erythro*pentofuranosyl)-5-dimethylaminouracil,
1-(2-deoxy-β-L-*erythro*pentofuranosyl)-5-methoxyuracil,
1-(2-deoxy-β-L-*erythro*pentofuranosyl)-5-benzyloxyuracil,
1-(2-deoxy-β-L-*erythro*pentofuranosyl)-5-ethoxyuracil,
1-(2-deoxy-β-L-*erythro*pentofuranosyl)-5-thiouracil,
1-(2-deoxy-β-L-*erythro*pentofuranosyl)-5-methylthiouracil,
1-(2-deoxy-β-L-*erythro*pentofuranosyl)-5-ethylthiouracil,
1-(2-deoxy-β-L-*erythro*pentofuranosyl)-5-benzylthiouracil,
1-(2-deoxy-β-L-*erythro*pentofuranosyl)-5-cyanouracil,
1-(2-deoxy-β-L-*erythro*pentofuranosyl)uracil-5-carboxamide,
1-(2-deoxy-β-L-*erythro*pentofuranosyl)uracil-5-thiocaboxamide,
1-(2-deoxy-β-L-*erythro*pentofuranosyl)uracil-5-carboxylic acid,
1-(2-deoxy-β-L-*erythro*pentofuranosyl)-5-methoxycarbonyluracil,
1-(2-deoxy-β-L-*erythro*pentofuranosyl)-5-ethoxycarbonyluracil,
1-(2-deoxy-β-L-*erythro*pentofuranosyl)-5-phenoxycarbonyluracil,
1-(2-deoxy-β-L-*erythro*pentofuranosyl)-5-benzyloxycarbonyluracil,
1-(2-deoxy-β-L-*erythro*pentofuranosyl)-5-carboxymethyluracil,
1-(2-deoxy-β-L-*erythro*pentofuranosyl)-5-methoxycarbonylmethyluracil,
1-(2-deoxy-β-L-*erythro*pentofuranosyl)-5-ethoxycarbonylmethyluracil,
1-(2-deoxy-β-L-*erythro*pentofuranosyl)-5-benzyloxycarbonylmethyluracil,
1-(2-deoxy-β-L-*erythro*pentofuranosyl)-5-vinyluracil,
1-(2-deoxy-β-L-*erythro*pentofuranosyl)-5-β-carboxyvinyluracil,
1-(2-deoxy-β-L-*erythro*pentofuranosyl)-5-β-ethoxycarbonylvinyluracil,
1-(2-deoxy-β-L-*erythro*pentofuranosyl)-5-β-fluorovinyluracil,
1-(2-deoxy-β-L-*erythro*pentofuranosyl)-5-β-chlorovinyluracil,
1-(2-deoxy-β-L-*erythro*pentofuranosyl)-5-β-bromovinyluracil,
1-(2-deoxy-β-L-*erythro*pentofuranosyl)-5-β-iodovinyluracil,
1-(2-deoxy-β-L-*erythro*pentofuranosyl)-5-β-methylvinyluracil,
1-(2-deoxy-β-L-*erythro*pentofuranosyl)-5-β-methylvinyluracil,
1-(2-deoxy-β-L-*erythro*pentofuranosyl)-5-allyluracil,
1-(2-deoxy-β-L-*erythro*pentofuranosyl)-5-ethynyluracil,
1-(2-deoxy-β-L-*erythro*pentofuranosyl)-5-β-methylethynyluracil,
1-(2-deoxy-β-L-*erythro*pentofuranosyl)-5-methyl-4-thiouracil,
1-(2-deoxy-β-L-*erythro*pentofuranosyl)-5-ethyl-4-thiouracil,
1-(2-deoxy-β-L-*erythro*pentofuranosyl)-5-propyl-4-thiouracil,
1-(2-deoxy-β-L-*erythro*pentofuranosyl)-5-phenyl-4-thiouracil,
1-(2-deoxy-β-L-*erythro*pentofuranosyl)-5-benzyl-4-thiouracil,
1-(2-deoxy-β-L-*erythro*pentofuranosyl)-5-fluoro-4-thiouracil,
1-(2-deoxy-β-L-*erythro*pentofuranosyl)-5-methoxy-4-thiouracil,
1-(2-deoxy-β-L-*erythro*pentofuranosyl)-5-benzyloxy-4-thiouracil,
1-(2-deoxy-β-L-*erythro*pentofuranosyl)-5-ethoxy-4-thiouracil,
1-(2-deoxy-β-L-*erythro*pentofuranosyl)-4,5-dithiouracil,
1-(2-deoxy-β-L-*erythro*pentofuranosyl)-5-methylthio-4-thiouracil,
1-(2-deoxy-β-L-*erythro*pentofuranosyl)-5-ethylthio-4-thiouracil,
1-(2-deoxy-β-L-*erythro*pentofuranosyl)-5-benzylthio-4-thiouracil,
1-(2-deoxy-β-L-*erythro*pentofuranosyl)-4-thiouracil-5-thiocaboxamide,
1-(2-deoxy-β-L-*erythro*pentofuranosyl)-4-thiouracil-5-carboxylic acid,
1-(2-deoxy-β-L-*erythro*pentofuranosyl)-5-methoxycarbonyl-4-thiouracil,
1-(2-deoxy-β-L-*erythro*pentofuranosyl)-5-ethoxycarbonyl-4-thiouracil,
1-(2-deoxy-β-L-*erythro*pentofuranosyl)-5-phenoxycarbonyl-4-thiouracil,
1-(2-deoxy-β-L-*erythro*pentofuranosyl)-5-benzyloxycarbonyl-4-thiouracil,
1-(2-deoxy-β-L-*erythro*pentofuranosyl)-5-carboxymethyl-4-thiouracil,
1-(2-deoxy-β-L-*erythro*pentofuranosyl)-5-methoxycarbonylmethyl-4-thiouracil,
1-(2-deoxy-β-L-*erythro*pentofuranosyl)-5-ethoxycarbonylmethyl-4-thiouracil,
1-(2-deoxy-β-L-*erythro*pentofuranosyl)-5-methylcytosine,
1-(2-deoxy-β-L-*erythro*pentofuranosyl)-5-ethylcytosine,
1-(2-deoxy-β-L-*erythro*pentofuranosyl)-5-propycytosine,
1-(2-deoxy-β-L-*erythro*pentofuranosyl)-5-phenylcytosine,
1-(2-deoxy-*β*-L-*erythro*pentofuranosyl)-5-benzylcytosine,
1-(2-deoxy-β-L-*erythro*pentofuranosyl)-5-fluorocytosine,
1-(2-deoxy-β-L-*erythro*pentofuranosyl)-5-chlorocytosine,
1-(2-deoxy-β-L-*erythro*pentofuranosyl)-5-bromocytosine,
1-(2-deoxy-β-L-*erythro*pentofuranosyl)-5-iodocytosine,
1-(2-deoxy-β-L-*erythro*pentofuranosyl)-5-nitrocytosine,
1-(2-deoxy-β-L-*erythro*pentofuranosyl)-5-aminocytosine,
1-(2-deoxy-β-L-*erythro*pentofuranosyl)-5-methylaminocytosine,
1-(2-deoxy-β-L-*erythr*opentofuranosyl)-5-ethylaminocytosine,
1-(2-deoxy-β-L-*erythro*pentofuranosyl)-5-dimethylaminocytosine,
1-(2-deoxy-β-L-*erythro*pentofuranosyl)-5-methoxycytosine,
1-(2-deoxy-β-L-*erythro*pentofuranosyl)-5-benzyloxycytosine,
1-(2-deoxy-β-L-*erythro*pentofuranosyl)-5-ethoxycytosine,
1-(2-deoxy-β-L-*erythr*opentofuranosyl)-5-thioucytosine,
1-(2-deoxy-β-L-*erythro*pentofuranosyl)-5-methylthiocytosine,
1-(2-deoxy-β-L-*erythro*pentofuranosyl)-5-ethylthiocytosine,
1-(2-deoxy-β-L-*erythro*pentofuranosyl)-5-benzylthiocytosine,
1-(2-deoxy-β-L-*erythro*pentofuranosyl)-5-cyanocytosine,
1-(2-deoxy-β-L-*erythro*pentofuranosyl)cytosine-5-carboxamide,
1-(2-deoxy-β-L-*erythro*pentofuranosyl)cytosine-5-thiocaboxamide,
1-(2-deoxy-β-L-*erythro*pentofuranosyl)cytosine-5-carboxylic acid,
1-(2-deoxy-β-L-*erythro*pentofuranosyl)-5-methoxycarbonylcytosine,
1-(2-deoxy-β-L-*erythro*pentofuranosyl)-5-ethoxycarbonylcytosine,
1-(2-deoxy-β-L-*erythro*pentofuranosyl)-5-phenoxycarbonylcytosine,
1-(2-deoxy-β-L-*erythro*pentofuranosyl)-5-benzyloxycarbonylcytosine,
1-(2-deoxy-β-L-*erythro*pentofuranosyl)-5-carboxymethylcytosine,
1-(2-deoxy-β-L-*erythro*pentofuranosyl)-5-methoxycarbonylmethylcytosine,
1-(2-deoxy-β-L-*erythro*pentofuranosyl)-5-ethoxycarbonylmethylcytosine,
1-(2-deoxy-β-L-*erythro*pentofuranosyl)-5-benzyloxycarbonylmethylcytosine,
1-(2-deoxy-*β*-L-*erythro*pentofuranosyl)-5-vinylcytosine,
1-(2-deoxy-*β*-L-*erythro*pentofuranosyl)-5-*β*-carboxyvinylcytosine,
1-(2-deoxy-*β*-L-*erythro*pentofuranosyl)-5-β-ethoxycarbonylvinylcytosine,
1-(2-deoxy-*β*-L-*erythro*pentofuranosyl)-5-β-fluorovinylcytosine,
1-(2-deoxy-*β*-L-*erythro*pentofuranosyl)-5-β-chlorovinylcytosine,
1-(2-deoxy-β-L-*erythro*pentofuranosyl)-5-β-bromovinylcytosine,
1-(2-deoxy-*β*-L-*erythro*pentofuranosyl)-5-β-iodovinylcytosine,
1-(2-deoxy-*β*-L-*erythro*pentofuranosyl)-5-β-methylvinylcytosine,
1-(2-deoxy-*β*-L-*erythro*pentofuranosyl)-5-β-methylvinylcytosine,
1-(2-deoxy-*β*-L-*erythro*pentofuranosyl)-5-allylcytosine,
1-(2-deoxy-*β*-L-*erythro*pentofuranosyl)-5-ethynylcytosine,
1-(2-deoxy-β-L-*erythro*pentofuranosyl)-5-β-methylethynylcytosine,
1-(2-deoxy-β-L-e*rythro*pentofuranosyl)-*N*4-methylcytosine,
1-(2-deoxy-β-L-*erythro*pentofuranosyl)-*N*4-ethylcytosine,
1-(2-deoxy-*β*-L-*erythro*pentofuranosyl)-*N*4-benzylcytosine,
1-(2-deoxy-β-L-e*rythro*pentofuranosyl)-*N*4,*N*4-dimethylcytosine,
1-(2-deoxy-*β*-L-*erythro*pentofuranosyl)-*N*4-methyl-5-methylcytosine,
1-(2-deoxy-*β*-L-*erythro*pentofuranosyl)-*N*4-benzyl-5-methylcytosine,
1-(2-deoxy-β-L-*erythro*pentofuranosyl)-*N*4-ethyl-5-methylcytosine,
1-(2-deoxy-*β*-L-*erythro*pentofuranosyl)-*N*4,*N*4-dimethyl-5-methylcytosine,
1-(2-deoxy-*β*-L-*erythro*pentofuranosyl)-*N*4-ethyl-5-methylcytosine,
9-(2-deoxy-*β*-L-*erythro*pentofuranosyl)purine,
9-(2-deoxy-*β*-L-*erythro*pentofuranosyl)-2-fluoropurine,
9-(2-deoxy-*β*-L-*erythro*pentofuranosyl)-2-chloropurine,
9-(2-deoxy-*β*-L-*erythro*pentofuranosyl)-2-bromopurine
9-(2-deoxy-*β*-L-*erythro*pentofuranosyl)-2-iodopurine,
9-(2-deoxy-β-L-e*rythro*pentofuranosyl)-2-aminopurine,
9-(2-deoxy-*β*-L-*erythro*pentofuranosyl)-2-methylaminopurine,
9-(2-deoxy-*β*-L-*erythro*pentofuranosyl)-2-dimethylaminopurine,
9-(2-deoxy-*β*-L-*erythro*pentofuranosyl)-2-trimethylammoniumpurine,
9-(2-deoxy-*β*-L-*erythro*pentofuranosyl)-2-hydroxypurine,
9-(2-deoxy-*β*-L-*erythro*pentofuranosyl)-2-methoxypurine,
9-(2-deoxy-β-L-*erythro*pentofuranosyl)-2-thiopurine,
9-(2-deoxy-β-L-*erythro*pentofuranosyl)-2-methylthiopurine,
9-(2-deoxy-β-L-*erythro*pentofuranosyl)-6-fluoropurine,
9-(2-deoxy-β-L-*erythro*pentofuranosyl)-6-chloropurine,
9-(2-deoxy-β-L-*erythro*pentofuranosyl)-6-bromopurine
9-(2-deoxy-β-L-*erythro*pentofuranosyl)hypoxanthine,
9-(2-deoxy-β-L-*erythro*pentofuranosyl)-6-methoxypurine,
9-(2-deoxy-β-L-*erythro*pentofuranosyl)-6-thiopurine,
9-(2-deoxy-β-L-*erythro*pentofuranosyl)-6-methylthiopurine
9-(2-deoxy-β-L-*erythro*pentofuranosyl)-6-methylaminopurine,
9-(2-deoxy-β-L-*erythro*pentofuranosyl)-6-dimethylaminopurine,
9-(2-deoxy-β-L-*erythro*pentofuranosyl)-8-methylpurine,
9-(2-deoxy-β-L-*erythro*pentofuranosyl)-8-chloropurine,
9-(2-deoxy-β-L-*erythro*pentofuranosyl)-8-bromopurine,
9-(2-deoxy-β-L-*erythro*pentofuranosyl)-8-oxopurine,
9-(2-deoxy-β-L-*erythro*pentofuranosyl)-8-methoxyurine
9-(2-deoxy-β-L-*erythro*pentofuranosyl)-8-thiopurine,
9-(2-deoxy-β-L-*erythro*pentofuranosyl)-8-methylthiourine
9-(2-deoxy-β-L-*erythro*pentofuranosyl)-8-aminopurine
9-(2-deoxy-β-L-*erythro*pentofuranosyl)-8-methylaminopurine,
9-(2-deoxy-β-L-*erythro*pentofuranosyl)-8-dimethylaminopurine,
9-(2-deoxy-β-L-*erythro*pentofuranosyl)-2,6-dichloropurine,
9-(2-deoxy-β-L-*erythro*pentofuranosyl)-2,6-dibromopurine
9-(2-deoxy-β-L-*erythro*pentofuranosyl)-2-amino-6-chloropurine,
9-(2-deoxy-β-L-*erythro*pentofuranosyl)-2-methylamino-6-chloropurine,
9-(2-deoxy-β-L-*erythro*pentofuranosyl)-2-dimethylamino-6-chloropurine,
9-(2-deoxy-β-L-*erythro*pentofuranosyl)-2-hydroxy-6-chloropurine,
9-(2-deoxy-β-L-*erythro*pentofuranosyl)-2-methoxy-6-chloropurine,
9-(2-deoxy-β-L-*erythro*pentofuranosyl)-2-fluoroadenine,
9-(2-deoxy-β-L-*erythro*pentofuranosyl)-2-chloroadenine,
9-(2-deoxy-β-L-*erythro*pentofuranosyl)-2-bromoadenine,
9-(2-deoxy-β-L-*erythro*pentofuranosyl)-2-iodoadenine,
9-(2-deoxy-β-L-*erythro*pentofuranosyl)-2,6-diaminopurine,
9-(2-deoxy-β-L-*erythro*pentofuranosyl)-2-methylaminoadenine,
9-(2-deoxy-β-L-*erythro*pentofuranosyl)-2-dimethylaminoadenine,
9-(2-deoxy-β-L-*erythro*pentofuranosyl)-2-trimethylammoniumadenine,
9-(2-deoxy-β-L-*erythro*pentofuranosyl)-isoguanine,
9-(2-deoxy-β-L-*erythro*pentofuranosyl)-2-methoxyadenine,
9-(2-deoxy-β-L-*erythro*pentofuranosyl)-2-thioadenine,
9-(2-deoxy-β-L-*erythro*pentofuranosyl)-2-methylthioadenine,
9-(2-deoxy-β-L-*erythro*pentofuranosyl)-2,6-bis(methylamino)purine,
9-(2-deoxy-β-L-*erythro*pentofuranosyl)-2-fluoro-6-methylaminopurine,
9-(2-deoxy-β-L-*erythro*pentofuranosyl)-2-chloro-6-methylaminopurine,
9-(2-deoxy-β-L-*erythro*pentofuranosyl)-2-bromo-6-methylaminopurine
9-(2-deoxy-β-L-*erythro*pentofuranosyl)-2-amino-6-methylaminopurine,
9-(2-deoxy-β-L-*erythro*pentofuranosyl)-2,6-bis(methylamino)purine,
9-(2-deoxy-β-L-*erythro*pentofuranosyl)-2-dimethylamino-6-methylaminopurine,
9-(2-deoxy-β-L-*erythro*pentofuranosyl)-2-trimethylammonium-6-methylaminopurine,
9-(2-deoxy-β-L-*erythro*pentofuranosyl)-2-hydroxy-6-methylaminopurine,
9-(2-deoxy-β-L-*erythro*pentofuranosyl)-2-methoxy-6-methylaminopurine,
9-(2-deoxy-β-L-*erythro*pentofuranosyl)-2-thiopurine-6-methylaminopurine,
9-(2-deoxy-β-L-*erythro*pentofuranosyl)-2-methylthio-6-methylaminopurine,
9-(2-deoxy-β-L-*erythro*pentofuranosyl)-2-fluorohypoxanthine,
9-(2-deoxy-β-L-*erythro*pentofuranosyl)-2-chlorohypoxanthine,
9-(2-deoxy-β-L-*erythro*pentofuranosyl)-2-bromohypoxanthine,
9-(2-deoxy-β-L-*erythro*pentofuranosyl)-2-iodohypoxanthine,
9-(2-deoxy-β-L-*erythro*pentofuranosyl)-2-methylaminohypoxanthine,
9-(2-deoxy-β-L-*erythro*pentofuranosyl)-2-dimethylaminohypoxanthine,
9-(2-deoxy-β-L-*erythro*pentofuranosyl)-2-trimethylammoniumhypoxanthine,
9-(2-deoxy-β-L-*erythro*pentofuranosyl)xanthine,
9-(2-deoxy-β-L-*erythro*pentofuranosyl)-2-methoxyhypoxanthine,
9-(2-deoxy-β-L-*erythro*pentofuranosyl)-2-thiohypoxanthine,
9-(2-deoxy-β-L-*erythro*pentofuranosyl)-2-methylthiohypoxanthine,
9-(2-deoxy-β-L-*erythro*pentofuranosyl)-2-fluoro-6-methoxypurine,
9-(2-deoxy-β-L-*erythro*pentofuranosyl)-2-chloro-6-methoxypurine,
9-(2-deoxy-β-L-*erythro*pentofuranosyl)-2-bromo-6-methoxypurine,
9-(2-deoxy-β-L-*erythro*pentofuranosyl)-2-iodo-6-methoxypurine,
9-(2-deoxy-β-L-*erythro*pentofuranosyl)-2-methylamino-6-methoxypurine,
9-(2-deoxy-β-L-*erythro*pentofuranosyl)-2-dimethylamino-6-methoxypurine,
9-(2-deoxy-β-L-*erythro*pentofuranosyl)-2-trimethylammonium-6-methoxypurine,
9-(2-deoxy-β-L-*erythro*pentofuranosyl)-2,6-dimethoxypurine,
9-(2-deoxy-β-L-*erythro*pentofuranosyl)-2-fluoro-6-thiopurine,
9-(2-deoxy-β-L-*erythro*pentofuranosyl)-2-chloro-6-thiopurine,
9-(2-deoxy-β-L-*erythro*pentofuranosyl)-2,6-dithiopurine,
9-(2-deoxy-β-L-*erythro*pentofuranosyl)-2-methylamino-6-thiopurine,
9-(2-deoxy-β-L-*erythro*pentofuranosyl)-2-dimethylamino-6-thiopurine,
9-(2-deoxy-β-L-*erythro*pentofuranosyl)-2-trimethylammonium-6-thiopurine,
9-(2-deoxy-β-L-*erythro*pentofuranosyl)-2,6-bis(methylthio)purine,
9-(2-deoxy-β-L-*erythro*pentofuranosyl)-2-thiohypoxanthine,
9-(2-deoxy-β-L-*erythro*pentofuranosyl)-2-methylthiohypoxanthine,
9-(2-deoxy-β-L-*erythro*pentofuranosyl)-2-fluoro-6-methylamine,
9-(2-deoxy-β-L-*erythro*pentofuranosyl)-2-chloro-6-methylaminopurine,
9-(2-deoxy-β-L-*erythro*pentofuranosyl)-2-bromo-6-methylaminopurine
9-(2-deoxy-β-L-*erythro*pentofuranosyl)-2-amino-6-methylaminopurine,
9-(2-deoxy-β-L-*erythro*pentofuranosyl)-2,6-bis(methylamino)purine,
9-(2-deoxy-β-L-*erythro*pentofuranosyl)-2-dimethylamino-6-methylaminopurine,
9-(2-deoxy-β-L-*erythro*pentofuranosyl)-2-trimethylammonium-6-methylaminopurine,
9-(2-deoxy-β-L-*erythro*pentofuranosyl)-2-hydroxy-6-methylaminopurine,
9-(2-deoxy-β-L-*erythro*pentofuranosyl)-2-methoxy-6-methylaminopurine,
9-(2-deoxy-β-L-*erythro*pentofuranosyl)-2-thiopurine-6-methylaminopurine,
9-(2-deoxy-β-L-*erythro*pentofuranosyl)-2-methylthio-6-methylaminopurine,
9-(2-deoxy-β-L-*erythro*pentofuranosyl)-2,8-dichloropurine,
9-(2-deoxy-β-L-*erythro*pentofuranosyl)-2,8-dibromopurine
9-(2-deoxy-β-L-*erythro*pentofuranosyl)-2-amino-8-chloropurine,
9-(2-deoxy-β-L-*erythro*pentofuranosyl)-2-methylamino-8-chloropurine,
9-(2-deoxy-β-L-*erythro*pentofuranosyl)-2-dimethylamino-8-chloropurine,
9-(2-deoxy-β-L-*erythro*pentofuranosyl)-2-hydroxy-8-chloropurine,
9-(2-deoxy-β-L-*erythro*pentofuranosyl)-2-methoxy-8-chloropurine,
9-(2-deoxy-β-L-*erythro*pentofuranosyl)-2-fluoro-8-aminopurine,
9-(2-deoxy-β-L-*erythro*pentofuranosyl)-2-chloro-8-aminopurine,
9-(2-deoxy-β-L-*erythro*pentofuranosyl)-2,8-diaminopurine,
9-(2-deoxy-β-L-*erythro*pentofuranosyl)-2-hydorxy-8-aminopurine,
9-(2-deoxy-β-L-*erythro*pentofuranosyl)-2-methoxy-8-aminopurine,
9-(2-deoxy-β-L-*erythro*pentofuranosyl)-2,8-bis(methylamino)purine,
9-(2-deoxy-β-L-*erythro*pentofuranosyl)-2-fluoro-8-methylaminopurine,
9-(2-deoxy-β-L-*erythro*pentofuranosyl)-2-chloro-8-methylaminopurine,
9-(2-deoxy-β-L-*erythro*pentofuranosyl)-2-bromo-8-methylaminopurine
9-(2-deoxy-β-L-*erythro*pentofuranosyl)-2-hydroxy-8-methylaminopurine,
9-(2-deoxy-β-L-*erythro*pentofuranosyl)-2-methoxy-8-methylaminopurine,
9-(2-deoxy-β-L-*erythro*pentofuranosyl)-2-thiopurine-8-methylaminopurine,
9-(2-deoxy-β-L-*erythro*pentofuranosyl)-2-methylthio-8-methylaminopurine,
9-(2-deoxy-β-L-*erythro*pentofuranosyl)-2-fluoro-8-oxopurine,
9-(2-deoxy-β-L-*erythro*pentofuranosyl)-2-chloro-8-oxopurine,
9-(2-deoxy-β-L-*erythro*pentofuranosyl)-2-amino-8-oxopurine,
9-(2-deoxy-β-L-*erythro*pentofuranosyl)-2-methylamino-8-oxopurine,
9-(2-deoxy-β-L-*erythro*pentofuranosyl)-2-dimethylamino-8-oxopurine,
9-(2-deoxy-β-L-*erythro*pentofuranosyl)-2-trimethylammonium-8-oxopurine,
9-(2-deoxy-β-L-*erythro*pentofuranosyl)-2,8-dihydroxypurine,
9-(2-deoxy-β-L-*erythro*pentofuranosyl)-2,8-dimethoxypurine,
9-(2-deoxy-β-L-*erythro*pentofuranosyl)-2,8-dithiopurine,
9-(2-deoxy-β-L-*erythro*pentofuranosyl)-2,8-dimethylthiopurine,
9-(2-deoxy-β-L-*erythro*pentofuranosyl)-2-fluoro-8-methoxypurine,
9-(2-deoxy-β-L-*erythro*pentofuranosyl)-2-chloro-8-methoxypurine,
9-(2-deoxy-β-L-*erythro*pentofuranosyl)-2-methylamino-8-methoxypurine,
9-(2-deoxy-β-L-*erythro*pentofuranosyl)-2-dimethylamino-8-methoxypurine,
9-(2-deoxy-β-L-*erythro*pentofuranosyl)-2-fluoro-8-thiopurine,
9-(2-deoxy-β-L-*erythro*pentofuranosyl)-2-chloro-8-thiopurine,
9-(2-deoxy-β-L-*erythro*pentofuranosyl)-2-methylamino-8-thiopurine,
9-(2-deoxy-β-L-*erythro*pentofuranosyl)-2-dimethylamino-8-thiopurine,
9-(2-deoxy-β-L-*erythro*pentofuranosyl)-2-thio-8-oxopurine,
9-(2-deoxy-β-L-*erythro*pentofuranosyl)-2-methylthio-8-oxopurine,
9-(2-deoxy-β-L-*erythro*pentofuranosyl)-6,8-dichloropurine,
9-(2-deoxy-β-L-*erythro*pentofuranosyl)-6,8-dibromopurine
9-(2-deoxy-β-L-*erythro*pentofuranosyl)-8-chloroadenine,
9-(2-deoxy-β-L-*erythro*pentofuranosyl)-6-methylamino-8-chloropurine,
9-(2-deoxy-β-L-*erythro*pentofuranosyl)-6-dimethylamino-8-chloropurine,
9-(2-deoxy-β-L-*erythro*pentofuranosyl)-8-chlorohypoxanthine,
9-(2-deoxy-β-L-*erythro*pentofuranosyl)-6-methoxy-8-chloropurine,
9-(2-deoxy-β-L-*erythro*pentofuranosyl)-6-fluoro-8-aminopurine,
9-(2-deoxy-β-L-*erythro*pentofuranosyl)-6-chloro-8-aminopurine,
9-(2-deoxy-β-L-*erythro*pentofuranosyl)-6,8-diaminopurine,
9-(2-deoxy-β-L-*erythro*pentofuranosyl)-6,8-bis(methylamino)purine,
9-(2-deoxy-β-L-*erythro*pentofuranosyl)-6-chloro-8-methylaminopurine,
9-(2-deoxy-β-L-*erythro*pentofuranosyl)-6-bromo-8-methylaminopurine
9-(2-deoxy-β-L-*erythro*pentofuranosyl)-8-methylaminohypoxanthine,
9-(2-deoxy-β-L-*erythro*pentofuranosyl)-6-methoxy-8-methylaminopurine,
9-(2-deoxy-β-L-*erythro*pentofuranosyl)-6-thiopurine-8-methylaminopurine,
9-(2-deoxy-β-L-*erythro*pentofuranosyl)-6-methylthio-8-methylaminopurine,
9-(2-deoxy-β-L-*erythro*pentofuranosyl)-6-chloro-8-oxopurine,
9-(2-deoxy-β-L-*erythro*pentofuranosyl)-8-oxoadenine,
9-(2-deoxy-β-L-*erythro*pentofuranosyl)-6-methylamino-8-oxopurine,
9-(2-deoxy-β-L-*erythro*pentofuranosyl)-6-dimethylamino-8-oxopurine,
9-(2-deoxy-β-L-*erythro*pentofuranosyl)-6,8-dihydroxypurine,
9-(2-deoxy-β-L-*erythro*pentofuranosyl)-6,8-dimethoxypurine,
9-(2-deoxy-β-L-*erythro*pentofuranosyl)-6,8-dithiopurine,
9-(2-deoxy-β-L-*erythro*pentofuranosyl)-6,8-dimethylthiopurine,
9-(2-deoxy-β-L-*erythro*pentofuranosyl)-6-dimethylamino-8-thiopurine,
9-(2-deoxy-β-L-*erythro*pentofuranosyl)-6-thio-8-oxopurine,
9-(2-deoxy-β-L-*erythro*pentofuranosyl)-6-methylthio-8-oxopurine,
9-(2-deoxy-β-L-*erythro*pentofuranosyl)-2,6,8-trichloropurine,
9-(2-deoxy-β-L-*erythro*pentofuranosyl)-2,6,8-tribromopurine
9-(2-deoxy-β-L-*erythro*pentofuranosyl)-2-amino-6,8-dichloropurine,
9-(2-deoxy-β-L-*erythro*pentofuranosyl)-2,8-dithioadenine,
9-(2-deoxy-β-L-*erythro*pentofuranosyl)-2,8-dimethylthioadenine,
9-(2-deoxy-β-L-*erythro*pentofuranosyl)-2,6,8-tris(methylamino)purine,
9-(2-deoxy-β-L-*erythro*pentofuranosyl)-8-methylxanthine,
9-(2-deoxy-β-L-*erythro*pentofuranosyl)-2-dimethylaminohypoxanthine,

This invention also provides a pharmaceutical composition which comprises any of the above-identified compounds and a pharmaceutically acceptable carrier. In the preferred embodiment of this invention, the compounds are administered to the mammal, including a human, as a pharmaceutical composition.

### Definitions

The term "alkyl," as used herein, unless otherwise specified, refers to a saturated straight, branched, or cyclic, primary, secondary, or tertiary hydrocarbon, typically of C₁ to C₁₈, and specifically includes methyl, ethyl, propyl, isopropyl, butyl, isobutyl, t-butyl, pentyl, cyclopentyl, isopentyl, neopentyl, hexyl, isohexyl, cyclohexyl, cyclohexylmethyl, 3-methylpentyl, 2,2-dimethylbutyl, and 2,3-dimethylbutyl. The alkyl group can be optionally substituted with one or more moieties selected from the group consisting of hydroxyl, amino, alkylamino, arylamino, alkoxy, aryloxy, nitro, cyano, sulfonic acid, sulfate, phosphonic acid, phosphate, or phosphonate, either unprotected, or protected as necessary, as known to those skilled in the art, for example, as taught in Greene, *et al*., "Protective Groups in Organic Synthesis," John Wiley and Sons, Second Edition, 1991, hereby incorporated by reference.

The term "lower alkyl," as used herein, and unless otherwise specified, refers to a C₁ to C₆ saturated straight or branched alkyl group.

The term "aryl," as used herein, and unless otherwise specified, refers to phenyl, biphenyl, or naphthyl, and preferably phenyl. The aryl group can be optionally substituted with one or more moieties selected from the group consisting of hydroxyl, amino, alkylamino, arylamino, alkoxy, aryloxy, nitro, cyano, sulfonic acid, sulfate, phosphonic acid, phosphate, or phosphonate, either unprotected, or protected as necessary, as known to those skilled in the art, for example, as taught in Greene, *et al*., "Protective Groups in Organic Synthesis," John Wiley and Sons, Second Edition, 1991.

The term "alkaryl" or "alkylaryl" refers to an alkyl group with an aryl substituent.

The term "aralkyl" or "arylalkyl" refers to an aryl group with an alkyl substituent.

The term "halogen," as used herein, includes fluorine, chlorine, bromine and iodine.

The term "acyl" refers to moiety of the formula -C(=O)R', wherein R' is alkyl; aryl, alkaryl, aralkyl, heteroaromatic, alkoxyalkyl including methoxymethyl; arylalkyl including benzyl; aryloxyalkyl such as phenoxymethyl; aryl including phenyl optionally substituted with halogen, C₁ to C₄ alkyl or C₁ to C₄ alkoxy, or the residue of an amino acid.

The term "reducing agent," as used herein refers to a reagent that substitutes at least one hydrogen for a functional group on a carbon atom. Non-limiting examples of reducing agents include NaH, KH, LiH, NH₂/NH₃, NaBH₂S₃, tributyltin hydride, optionally in the presence of AIBN, Raney nickel; hydrogen gas over palladium; hydrazine hydrate and KOH, catecholborane and sodium acetate, BH₃-THF, BH₃-etherate, NaBH₄, NaBH₃CN, disiamylborane, LiAlH₄, LiAlH(OMe)₃, LiAlH(O-*t*-Bu)₃, AlH₃, LiBEt₃H, NaAlEt₂H₂, zinc (with acid or base), SnCl₂, Chromium (II) ion, optionally complexed with ethylenediamine or ethanolamine, (Me₃Si)₃Si-H-NaBH₄, SmI₂-THF-HMPA, Et₃SiH in the presence of AlCl₃, titanium, (C₅H₅)₂TiCl₂, Zn-Hg, Lindlar's Catalyst, rhodium, ruthenium, chlorotris(triphenylphosphine)rhodium (Wilkinson's Catalyst), chlorotris(triphenylphosphine)hydridoruthenium (II), H₂PtCl₆, RhCl₃, sodium in alcohol, DIBALH, Li/NH₃, 9-BBN, NaBH₃(OAc), Et₃SiH, SiHCl₃, Pb(OAc)₄, Cu(OAc)₂, lithium *n*-butylborohydride, Alpine-Borane and SeO₂.

The term "amino acid" includes naturally occurring and synthetic amino acids, and includes but is not limited to, alanyl, valinyl, leucinyl, isoleucinyl, prolinyl, phenylalaninyl, tryptophanyl, methioninyl, glycinyl, serinyl, threoninyl, cysteinyl, tyrosinyl, asparaginyl, glutaminyl.

The term "purine or pyrimidine base" includes, but is not limited to, adenine, N⁶-alkylpurines, N⁶-acylpurines (wherein acyl is C(=O)(alkyl, aryl, alkylaryl, or arylalkyl), N⁶-benzylpurine, N⁶-halopurine, N⁶-vinylpurine, N⁶-acetylenic purine, N⁶-acyl purine, N⁶-hydroxyalkyl purine, N⁶-thioalkyl purine, N²-alkylpurines, N²-alkyl-6-thiopurines, thymine, cytosine, 5-fluorocytosine, 5-methylcytosine, 6-azapyrimidine, including 6-azacytosine, 2- and/or 4-mercaptopyrmidine, uracil, 5-halouracil, including 5-fluorouracil, C⁵-alkylpyrimidines, C⁵-benzylpyrimidines, C⁵-halopyrimidines, C⁵-vinylpyrimidine, C⁵-acetylenic pyrimidine, C⁵-acyl pyrimidine, C⁵-hydroxyalkyl purine, C⁵-amidopyrimidine, C⁵-cyanopyrimidine, C⁵-nitropyrimidine, C⁵-aminopyrimidine, N²-alkylpurines, N²-alkyl-6-thiopurines, 5-azacytidinyl, 5-azauracilyl, triazolopyridinyl, imidazolopyridinyl, pyrrolopyrimidinyl, and pyrazolopyrimidinyl. Purine bases include, but are not limited to, guanine, adenine, hypoxanthine, 2,6-diaminopurine, and 6-chloropurine. Functional oxygen and nitrogen groups on the base can be protected as necessary or desired. Suitable protecting groups are well known to those skilled in the art, and include trimethylsilyl, dimethylhexylsilyl, *t*-butyldimethylsilyl, and *t*-butyldiphenylsilyl, trityl, alkyl groups, acyl groups such as acetyl and propionyl, methanesulfonyl, and p-toluenesulfonyl.

The term "heteroaryl" or "heteroaromatic," as used herein, refers to an aromatic that includes at least one sulfur, oxygen, nitrogen or phosphorus in the aromatic ring. The term "heterocyclic" refers to a nonaromatic cyclic group wherein there is at least one heteroatom, such as oxygen, sulfur, nitrogen, or phosphorus in the ring. Nonlimiting examples of heteroaryl and heterocyclic groups include furyl, furanyl, pyridyl, pyrimidyl, thienyl, isothiazolyl, imidazolyl, tetrazolyl, pyrazinyl, benzofuranyl, benzothiophenyl, quinolyl, isoquinolyl, benzothienyl, isobenzofuryl, pyrazolyl, indolyl, isoindolyl, benzimidazolyl, purinyl, carbazolyl, oxazolyl, thiazolyl, isothiazolyl, 1,2,4-thiadiazolyl, isooxazolyl, pyrrolyl, quinazolinyl, cinnolinyl, phthalazinyl, xanthinyl, hypoxanthinyl, thiophene, furan, pyrrole, isopyrrole, pyrazole, imidazole, 1,2,3-triazole, 1,2,4-triazole, oxazole, isoxazole, thiazole, isothiazole, pyrimidine or pyridazine, and pteridinyl, aziridines, thiazole, isothiazole, 1,2,3-oxadiazole, thiazine, pyridine, pyrazine, piperazine, pyrrolidine, oxaziranes, phenazine, phenothiazine, morpholinyl, pyrazolyl, pyridazinyl, pyrazinyl, quinoxalinyl, xanthinyl, hypoxanthinyl, pteridinyl, 5-azacytidinyl, 5-azauracilyl, tfiazolopyridinyl, imidazolopyridinyl, pyrrolopyrimidinyl, pyrazolopyrimidinyl, adenine, N⁶-alkylpurines, N⁶-benzylpurine, N⁶-halopurine, N⁵-vinypurine, N⁶-acetylenic purine, N⁶-acyl purine,N⁶-hydroxyalkyl purine, N⁶-thioalkyl purine, thymine, cytosine, 6-azapyrimidine, 2-mercaptopyrmidine, uracil, N⁵-alkylpyrimidines, N⁵-benzylpyrimidines, N⁵-halopyrimidines, N⁵-vinylpyrimidine, N⁵-acetylenic pyrimidine, N⁵-acyl pyrimidine, N⁵-hydroxyalkyl purine, N⁶-thioalkyl purine, isoxazolyl, pyrrolidin-2-yl, pyrrolidin-2-on-5-yl, piperidin-2-yl, piperidin-2-on-l-yl, piperidin-2-on-6-yl, quinolin-2-yl, isoquinolin-1-yl, isoquinolin-3-yl, pyridin-2-yl, 4-methylimidazol-2-yl, 1-methylimidazol-4-yl, 1-methylimidazol-5-yl, 1-n-hexylimidazol-4-yl, 1-*N*-hexylimidazol-5-yl, 1-benzylimidazol-4-yl, 1-benzylimidazol-5-yl, 1,2-dimethyl-imidazol-4-yl, 1,2-dimethylimidazol-5-yl, 1-n-pentyl-2-methyl-imidazol-4-yl, 1-n-pentyl-2-methyl-imidazol-5-yl, 1-n-butyl-2-methyl-imidazol-4-yl, 1-n-butyl-2-methyl-imidazol-5-yl, 1-benzyl-2-methyl-imidazol-4-yl, 1-benzyl-2-methyl-imidazol-5-yl, benz-imidazol-2-yl, 1-methylbenzimidazol-2-yl, 1-ethylbenzimidazol-2-yl, 1-n-propylbenz-imidazol-2-yl, 1-isopropyl-benzimidazol-2-yl, 1-*n*-butylbenzimidazol-2-yl, 1-isobutylbenz-imidazol-2-yl, 1-*n*-pentylbenzimidazol-2-yl, 1-*n*-hexylbenzimidazol-2-yl, 1-cyclopropylbenz-imidazol-2-yl, 1-cyclobutylbenzimidazol-2-yl, 1-cyclopentylbenzimidazol-2-yl, 1-cyclo-hexylbenzimidazol-2-yl, 5-nitro-benzimidazol-2-yl, 5-amino-benzimidazol-2-yl, 5-acet-amidobenzimidazol-2-yl, 5-methyl-benzimidazol-2-yl, 5-methoxy-benzimidazol-2-yl, 5-ethoxy-benzimidazol-2-yl, 1-methyl-5-methoxy-benzimidazol-2-yl, 1,5-dimethyl-benz-imidazol-2-yl, 1,6-dimethyl-benzimidazol-2-yl, 1,4-dimethyl-benzimidazol-2-yl, 5,6-di-methyl-benzimidazol-2-yl, 1,5,6-trimethyl-benzimidazol-2-yl, 5-chloro-benzimidazol-2-yl, 5-chloro-1-methyl-benzimidazol-2-yl, 6-chloro-1-methyl-benzimidazol-2-yl, 5,6-dichloro-1-methyl-benzimidazol-2-yl, 5-dimethylamino-benzimidazol-2-yl, 5-dimethylamino-1-ethyl-benzimidazol-2-yl, 5,6-dimethoxy-1-methyl-benzimidazol-2-yl, 5,6-dimethoxy-1-ethyl-benzimidazol-2-yl, 5-fluoro-1-methyl-benzimidazol-2-yl, 6-fluoro-1-methyl-benzimidazol-2-yl, 5-trifluoromethyl-benzimidazol-2-yl, 5-trifluoromethyl-1-methyl-benzimidazol-2-yl, 4-cyano-1-methyl-benzimidazol-2-yl, 5-carboxy-1-methyl-benzimidazol-2-yl, 5-amino-carbonyl-benzimidazol-2-yl, 5-aminocarbonyl-l-methyl-benzimidazol-2-yl, 5-dimethyl-aminosulphonyl-1-methyl-benzimidazol-2-yl, 5-methoxycarbonyl-1-methyl-benzimidazol-2-yl, 5-methylaminocarbonyl-1-methyl-benzimidazol-2-yl, 5-dimethylaminocarbonyl-1-methyl-benzimidazol-2-yl, 4,6-di-fluoro-1-methyl-benzimidazol-2-yl, 5-acetyl-1-methyl-benz-imidazol-2-yl, 5,6-dihydroxy-1-methyl-benzimidazol-2-yl, imidazo[1,2-a]pyridin-2-yl, 5-methyl-imidazo[1,2-a]pyridin-2-yl, 6-methyl-imidazo[1,2-a]-pyridin-2-yl, 7-methyl-imidazo-[1,2-a]-pyridin-2-yl, 8-methyl-imidazo-[1,2-a]-pyridin-2-yl, 5,7-dimethyl-imidazo-[1,2-a]-pyridin-2-yl, 6-aminocarbonyl-imidazo-[1,2-a]-pyridin-2-yl, 6-chloro-imidazo[1,2-a]-pyridin-2-yl, 6-bromo-imidazo[1,2-a]pyridin-2-yl, 5,6,7,8-tetrahydro-imidazo[1,2-a]pyrimidin-2-yl, imidazo[1,2-a]pyrimidin-2-yl, 5,7-dimethyl-imidazo[1,2-a]pyrimidin-2-yl, imidazo[4,5-b]pyridin-2-yl, 1-methyl-imidazo[4,5-b]pyridin-2-yl, 1-n-hexyl-imidazo[4,5-b]pyridin-2-yl, 1-cyclopropyl-imidazo-[4,5-b]-pyridin-2-yl, 1-cyclohexyl-imidazo[4,5-b]pyridin-2-yl, 4-methyl-imidazo-[4,5-b]-pyridin-2-yl, 6-methyl-imidazo[4,5-b]pyridin-2-yl, 1,4-dimethyl-imidazo-[4,5-b]-pyridin-2-yl, 1,6-dimethyl-imidazo[4,5-b]pyridin-2-yl, imidazo[4,5-c]pyridin-2-yl, 1-methyl-imidazo-[4,5-c]-pyridin-2-yl, 1-n-hexyl-imidazo[4,5-c]-pyridin-2-yl, 1-cyclo-propyl-imidazo[4,5-c]-pyridin-2-yl, 1-cyclohexyl-imidazo[4,5-c]pyridin-2-yl, imidazo-[2,1-b]-thiazol-6-yl, 3-methyl-imidazo-[2,1-b]-thiazol-6-yl, 2-phenyl-imidazo[2,1-b]thiazol-6-yl, 3-phenyl-imidazo-[2,1-b]-thiazol-6-yl, 2,3-dimethyl-imidazo[2,1-b]-thiazol-6-yl, 2,3-tri-methylene-imidazo-[2,1-b]-thiazol-6-yl, 2,3-tetramethylene-imidazo[2,1-b]thiazol-6-yl, imidazo-[1,2-c]-pyrimidin-2-yl, imidazo[1,2-a]pyrazin-2-yl, imidazo[1,2-b]pyridazin-2-yl, imidazo[4,5-c]-pyridin-2-yl, purin-8-yl, imidazo[4,5-b]pyrazin-2-yl, imidazo[4,5-c]pyridazin-2-yl, imidazo-[4,5-d]-pyridazin-2-yl, imidazolidin-2,4-dion-3-yl, 5-methyl-imidazolidin-2,4-dion-3-yl, 5-ethyl-imidazolidin-2,4-dion-3-yl, 5-n-propyl-imidazolidin-2,4-dion-3-yl, 5-benzyl-imidazolidin-2,4-dion-3-yl, 5-(2-phenylethyl)-imidazolidin-2,4-dion-3-yl, 5-(3-phenylpropyl)-imidazolidin-2,4-dion-3-yl, 5,5-tetramethylene-imidazolidin-2,4-dion-3-yl, 5,5-pentamethylene-imidazolidin-2,4-dion-3-yl, 5,5-hexamethylene-imidazolidin-2,4-dion-3-yl, 1-methyl-imidazolidin-2,4-dion-3-yl, 1-benzyl-imidazolin-2,4-dion-3-yl, 4,5-dihydro-2H-pyridazin-3-on-6-yl, 2-methyl-4,5-dihydro-2H-pyridazin-3-on-6-yl, 2-ethyl-4,5-dihydro-2H-pyridazin-3-on-6-yl, 2-n-propyl-4,5-dihydro-2H-pyridazin-3-on-6-yl, 2-isopropyl-4,5-di-hydro-2H-pyridazin-3-on-6-yl, 2-benzyl-4,5-dihydro-2H-pyridazin-3-on-6-yl, 2-(2-phenylethyl)-4,5-dihydro-2H-pyridazin-3-on-6-yl, 2-(3-phenylpropyl)-4,5-dihydro-2H-pyridazin-3-on-6-yl, 4-methyl-4,5-dihydro-2H-pyridazin-3-on-6-yl, 5-methyl-4,5-dihydro-2H-pyridazin-3-on-6-yl, 4,4-dimethyl-4,5-dihydro-2H-pyridazin-3-on-6-yl, 5,5-dimethyl-4,5-dihydro-2H-pyridazin-3-on-6-yl, 4,5-dimethyl-4,5-dihydro-2H-pyridazin-3-on-6-yl, 2,4-dimethyl-4,5-di-hydro-2H-pyridazin-3-on-6-yl, 2,5-dimethyl-4,5-dihydro-2H-pyridazin-3-on-6-yl, 2,4,5-trimethyl-4,5-dihydro-2H-pyridazin-3-on-6-yl, 2,4,4-trimethyl-4,5-dihydro-2H-pyridazin-3-on-6-yl, 2,5,5-trimethyl-4,5-dihydro-2H-pyridazin-3-on-6-yl, 2H-pyridazin-3-on-6-yl, 2-methyl-pyridazin-3-on-6-yl, 2-ethyl-pyridazin-3-on-6-yl, 2-n-propyl-pyridazin-3-on-6-yl, 3,4,5,6-tetrahydro-2-pyrimidon-1-yl, 3-methyl-3,4,5,6-tetrahydro-2-pyrimidon-1-yl, 3-ethyl-3,4,5,6-tetrahydro-2-pyrimidon-1-yl, 3-n-propyl-3,4,5,6-tetrahydro-2-pyrimidon-1-yl, 3-isopropyl-3,4,5,6-tetrahydro-2-pyrimidon-1-yl, 3-n-butyl-3,4,5,6-tetrahydro-2-pyrimidon-1-yl, 3-isobutyl-3,4,5,6-tetrahydro-2-pyrimidon-1-yl, 3-n-pentyl-3,4,5,6-tetrahydro-2-pyrimidon-1-yl, 3-n-hexyl-3,4,5,6-tetrahydro-2-pyrimidon-1-yl, 3-cyclopentyl-3,4,5,6-tetrahydro-2-pyrimidon-1-yl, 3-cyclohexyl-3,4,5,6-tetrahydro-2-pyrimidon-1-yl, 3-cycloheptyl-3,4,5,6-tetrahydro-2-pyrimidon-1-yl, 3-benzyl-3,4,5,6-tetrahydro-2-pyrimidon-1-yl, 3,4,5,6-tetrahydro-2(1H)-pyrimidon-1-yl, 3-methyl-3,4,5,6-tetrahydro-2(1H)-pyrimidon-1-yl, 3-ethyl-3,4,5,6-tetrahydro-2(1H)-pyrimidon-1-yl, 3-n-propyl-3,4,5,6-tetrahydro-2(1H)-pyrimidon-1-yl, 3-isopropyl-3,4,5,6-tetrahydro-2(1H)-pyrimidon-1-yl, 3-benzyl-3,4,5,6-tetrahydro-2(1H)-pyrimidon-1-yl, 3-(2-phenylethyl)-3,4,5,6-tetrahydro-2(1H)-pyrimidon-1-yl or 3-(3-phenylpropyl)-3,4,5,6-tetrahydro-2(1H)-pyrimidon-1-yl group, oxazol-4-yl, 2-methyl-oxazol-4-yl, 2-ethyl-oxazol-4-yl, 2-n-propyl-oxazol-4-yl, 2-isopropyl-oxazol-4-yl, 2-n-butyl-oxazol-4-yl, 2-isobutyl-oxazol-4-yl, 2-n-pentyl-oxazol-4-yl, 2-isoamyl-oxazol-4-yl, 2-n-hexyl-oxazol-4-yl, 2-phenyl-oxazol-4-yl, thiazol-4-yl, 2-methyl-thiazol-4-yl, 2-ethyl-thiazol-4-yl, 2-n-propyl-thiazol-4-yl, 2-isopropyl-thiazol-4-yl, 2-n-butyl-thiazol-4-yl, 2-isobutyl-thiazol-4-yl, 2-n-pentyl-thiazol-4-yl, 2-isoamyl-thiazol-4-yl, 2-n-hexyl-thiazol-4-yl, 2-phenyl-thiazol-4-yl, 1-methyl-imidazol-4-yl, 1-ethyl-imidazol-4-yl, 1-n-propyl-imidazol-4-yl, 1-isopropyl-imidazol-4-yl, 1-n-butyl-imidazol-4-yl, 1-isobutyl-imidazol-4-yl, 1-n-pentyl-imidazol-4-yl-, 1-isoamyl-imidazol-4-yl, 1-n-hexyl-imidazol-4-yl, 1-n -hexyl-2-methyl-imidazol-4-yl, 1-(1-methyl-n-pentyl)-imidazol-4-yl, 1-(1-ethyl-n-butyl)-imidazol-4-yl, 1-(1-methyl-n-hexyl)-imidazol-4-yl, i-(i-ethyl-n-pentyl)-imidazol-4-yl, 1-(1-n-propyl-n-butyl)-imidazol-4-yl, 1-n-heptyl-imidazol-4-yl, 1-ethyl-2-methyl-imidazol-4-yl, 1-n-propyl-2-methyl-imidazol-4-yl, 1-isopropyl-2-methyl-imidazol-4-yl, 1-n-butyl-2-methyl-imidazol-4-yl, 1-isobutyl-2-methyl-imidazol-4-yl, 1-n-pentyl-2-methyl-imidazol-4-yl, 1-isoamyl-2-methyl-imidazol-4-yl, 1-n-hexyl-2-methyl-imidazol-4-yl, 1-n-heptyl-2-methyl-imidazol-4-yl, 1-cyclopropylmethyl-imidazol-4-yl, 1-cyclobutylmethyl-imidazol-4-yl, 1-cyclopentylmethyl-imidazol-4-yl, 1-cyclohexylmethyl-imidazol-4-yl, 1-cycloheptylmethyl-imidazol-4-yl, 1-(2-cyclo-propylethyl)-imidazol-4-yl, 1-(2-cyclobutylethyl)-imidazol-4-yl, 1-(2-cyclopentylethyl)-imidazol-4-yl, 1-(2-cyclohexylethyl)-imidazol-4-yl, 1-(2-cycloheptyl-ethyl)-imidazol-4-yl, 1-(3-cyclopropylpropyl)-imidazol-4- yl-, 1-(3-cyclobutylpropyl)-imidazol-4-yl, 1-(3-cyclopentylpropyl)-imidazol-4-yl, 1-(3-cyclohexyl-propyl)-imidazol-4-yl, 1-(3-cycloheptyl-propyl)-imidazol-4-yl, 1-(2,2,2-trifluoroethyl)-imidazol-4-yl, 1-(3,3,3-trifluoropropyl)-imidazol-4-yl, 1-benzyl-imidazol-4-yl, 1-(2-phenylethyl)-imidazol-4-yl, 1-(3-phenylpropyl)-imidazol-4-yl, 1-(4-fluorobenzyl)-imidazol-4-yl, 1-(4-chlorobenzyl)-imidazol-4-yl, 1-(3-chlorobenzyl)-imidazol-4-yl, 1-(4-trifluoromethyl-benzyl)-imidazol-4-yl, 1-(3-methylbenzyl)-imidazol-4-yl, 1-(4-methyl-benzyl)-imidazol-4-yl, 1-(3-methoxy-benzyl)-imidazol-4-yl, 1-(4-methoxy-benzyl)-imidazol-4-yl, 1-(3,4-dimethoxy-benzyl)-imidazol-4-yl, 1-(3,5-di-methoxy-benzyl)-imidazol-4-yl, 1-cyclopropylmethyl-2-methyl-imidazol-4-yl, 1-cyclobutylmethyl-2-methyl-imidazol-4-yl, 1-cyclopentylmethyl-2-methyl-imidazol-4-yl, 1-cyclohexylmethyl-2-methyl-imidazol-4-yl, 1-cyclo-heptylmethyl-2-methyl-imidazol-4-yl, 1-(2-cyclopropylethyl)-2-methyl-imidazol-4-yl, 1-(2-cyclobutylethyl)-2-methyl-imidazol-4-yl, 1-(2-cyclopentylethyl)-2-methyl-imidazol-4-yl, 1-(2-cyclohexylethyl)-2-methyl-imidazol-4-yl, 1-(2-cycloheptylethyl)-2-methyl-imidazol-4-yl, 1-(3-cyclopropylpropyl)-2-methyl-imidazol-4-yl, 1-(3-cyclobutylpropyl)-2-methyl-imidazol-4-yl, 1-(3-cyclopentylpropyl)-2-methyl-imidazol-4-yl, 1-(3-cyclohexylpropyl)-2-methyl-imidazol-4-yl, 1-(3-cycloheptylpropyl)-2-methyl-imidazol-4-yl, 1-(2,2,2-trifluoroethyl)-2-methyl-imidazol-4-yl-, 1-(3,3,3-trifluoropropyl)-2-methyl-imidazol-4-yl-, 1-benzyl-2-methyl-imidazol-4-yl, 1-(2-phenylethyl)-2-methyl-imidazol-4-yl, 1-(3-phenylpropyl)-2-methyl-imidazol-4-yl, 1-(4-fluorobenzyl)-2-methyl-imidazol-4-yl, 1-(4-chlorobenzyl)-2-methyl-imidazol-4-yl, 1-(3-chloro-benzyl)-2-methyl-imidazol-4-yl, 1-(4-trifluoromethyl-benzyl)-2-methyl-imidazol-4-yl, 1-(3-methylbenzyl)-2-methyl-imidazol-4-yl, 1-(4-methyl-benzyl)-2-methyl-imidazol-4-yl, 1-(3-methoxybenzyl)-2-methyl-imidazol-4-yl, 1-(4-methoxy-benzyl)-2-methyl-imidazol-4-yl, 1-(3,4-di-methoxy-benzyl)-2-methyl-imidazol-4-yl, 1-(3,5-dimethoxy-benzyl)-2-methyl-imidazol-4-yl, 1-carboxymethyl-imidazol-4-yl, 1-(2-carboxyethyl)-imidazol-4-yl, 1-(3-carboxypropyl)-imidazol-4-yl, 1-(4-carboxybutyl)-imidazol-4-yl, 1-(5-carboxypentyl)-imidazol-4-yl, 1-(6-carboxyhexyl)-imidazol-4-yl, 1-(7-carboxyheptyl)-imidazol-4-yl, 1-methoxycarbonylmethylimidazol-4-yl, 1-(2-methoxycarbonylethyl)-imidazol-4-yl, 1-(3-methoxycarbonylpropyl)-imidazol-4-yl, 1-(4-methoxycarbonylbutyl)-imidazol-4-yl, 1-(5-methoxycarbonylpentyl)-imidazol-4-yl, 1-(6-methoxycarbonylhexyl)-imidazol-4-yl, 1-(7-methoxy-carbonylheptyl)-imidazol-4-yl, 1-ethoxycarbonylmethyl-imidazol-4-yl, 1-(2-ethoxycarbonylethyl)-imidazol-4-yl, 1-(3-ethoxycarbonylpropyl)-imidazol-4-yl, 1-(4-ethoxycarbonylbutyl)-imidazol-4-yl, 1-(5-ethoxycarbonylpentyl)-imidazol-4-yl, 1-(6-ethoxycarbonylhexyl)-imidazol-4-yl, 1-(7-ethoxycarbonylheptyl)-imidazol-4-yl, 1-n-propoxycarbonylmethyl-imidazol-4-yl, 1-(2-n-propoxycarbonylethyl)-imidazol-4-yl, 1-(3-n-propoxycarbonylprapyl)-imidazol-4-yl, 1-(4-n-propoxycarbonylbutyl)-imidazol-4-yl, 1-(5-n-propoxycarbonyl-pentyl)-imidazol-4-yl, 1-(6-n-propoxycarbonylhexyl)-imidazol-4-yl, 1-(7-n-propoxycarbonylheptyl)-imidazol-4-yl, 1-isopropoxycarbonylmethyl-imidazol-4-yl, 1-(2-isopropoxycarbonylethyl)-imidazol-4-yl, 1-(3-isopropoxycarbonylpropyl)-imidazol-4-yl, 1-(4-isopropoxycarbonylbutyl)-imidazol-4-yl, 1-(5-isopropoxycarbonylpentyl)-imidazol-4-yl, 1-(6-isopropoxycarbonylhexyl)-imidazol-4-yl, 1-(7-isopropoxycarbonylheptyl)-imidazol-4-yl, 1-aminocarbonylmethyl-imidazol-4-yl, 1-(2-aminocarbonyl-ethyl)-imidazol-4-yl, 1-(3-aminocarbonylpropyl)-imidazol-4-yl, 1-(4-aminocarbonylbutyl)-imidazol-4-yl, 1-(5-aminocarbonylpentyl)-imidazol-4-yl, 1-(6-aminocarbonylhexyl)-imidazol-4-yl, 1-(7-aminocarbonyl-heptyl)-imidazol-4-yl, 1-methylaminocarbonylmethyl-imidazol-4-yl, 1-(2-methylaminocarbonylethyl)-imidazol-4-yl, 1-(3-methylaminocarbonylpropyl)-imidazol-4-yl, 1-(4-methylaminocarbonylbutyl)-imidazol-4-yl, 1-(5-methylaminocarbonylpentyl)-imidazol-4-yl, 1-(6-methylaminocarbonylhexyl)-imidazol-4-yl, 1-(7-methylaminocarbonylheptyl)-imidazol-4-yl, 1-ethylaminocarbonylmethyl-imidazol-4-yl, 1-(2-ethylaminocarbonylethyl)-imidazol-4-yl, 1-(3-ethylaminocarbonyl-propyl)-imidazol-4-yl, 1-(4-ethylaminocarbonylbutyl)-imidazol-4-yl, 1-(5-ethylaminocarbonylpentyl)-imidazol-4-yl, 1-(6-ethylaminocarbonylhexyl)-imidazol-4-yl, 1-(7-ethylaminocarbonylheptyl)-imidazol-4-yl, 1-n-propylaminocarbonylmethyl-imidazol-4-yl, 1-(2-n-propylaminocarbonylethyl)-imidazol-4-yl, 1-(3-n-propylaminocarbonylpropyl)-imidazol-4-yl, 1-(4-n-propylaminocarbonylbutyl)-imidazol-4-yl, 1-(5-n-propylaminocarbonylpentyl)-imidazol-4-yl, 1-(6-n-propylaminocarbonylhexyl)-imidazol-4-yl, 1-(7-n-propylaminocarbonylheptyl)-imidazol-4-yl, 1-isopropylaminocarbonylmethyl-imidazol-4-yl, 1-(2-isopropylaminocarbonylethyl)-imidazol-4-yl, 1-(3-isopropylaminocarbonylpropyl)-imidazol-4-yl, 1-(4-isopropylaminocarbonylbutyl)-imidazol-4-yl, 1-(5-isopropylaminocarbonylpentyl)-imidazol-4-yl, 1-(6-isopropylaminocarbonylhexyl)-imidazol-4-yl, 1-(7-isopropylaminocarbonylheptyl)-imidazol-4-yl, 1-dimethylaminocarbonylmethyl-imidazol-4-yl, 1-(2-dimethylaminocarbonylethyl)-imidazol-4-yl, 1-(3-dimethylaminocarbonylpropyl)-imidazol-4-yl, 1-(4-dimethylaminocarbonylbutyl)-imidazol-4-yl, 1-(5-dimethylaminocarbonylpentyl)-imidazol-4-yl, 1-(6-dimethylaminocarbonylhexyl)-imidazol-4-yl, 1-(7-dimethylaminocarbonylheptyl)-imidazol-4-yl, 1-diethylaminocarbonylmethyl-imidazol-4-yl, 1-(2-diethylaminocarbonylethyl)-imidazol-4-yl, 1-(3-diethylaminocarbonylpropyl)-imidazol-4-yl, 1-(4-diethylaminocarbonylbutyl)-imidazol-4-yl, 1-(5-diethylaminocarbonylpentyl)-imidazol-4-yl, 1-(6-diethylaminocarbonylhexyl)-imidazol-4-yl, 1-(7-diethylaminocarbonylheptyl)-imidazol-4-yl, 1-di-n-propylaminocarbonylmethyl-imidazol-4-yl, 1-(2-di-n-propylaminocarbonylethyl)-imidazol-4-yl, 1-(3-din-propylaminocarbonylpropyl)-imidazol-4-yl, 1-(4-di-n-propylaminocarbonylbutyl)-imidazol-4-yl, 1-(5-di-n-propylaminocarbonylpentyl)-imidazol-4-yl, 1-(6-di-n-propylaminocarbonylhexyl)-imidazol-4-yl, 1-(7-di-n-propylaminocarbonylheptyl)-imidazol-4-yl, 1-diisopropylaminocarbonylmethyl-imidazol-4-yl, 1-(2-diisopropylaminocarbonylethyl)-imidazol-4-yl, 1-(3-diisopropylaminocarbonylpropyl)-imidazol-4-yl, 1-(4-diisopropylaminocarbonylbutyl)-imidazol-4-yl, 1-(5-diisopropylaminocarbonylpentyl)-imidazol-4-yl, 1-(6-diisopropylaminocarbonylhexyl)-imidazol-4-yl, 1-(7-diisopropylaminocarbonylheptyl)-imidazol-4-yl, 1-morpholinocarbonylmethyl-imidazol-4-yl, 1-(2-morpholinocarbonylethyl)-imidazol-4-yl, 1-(3-morpholinocarbonylpropyl)-imidazol-4-yl, 1-(4-morpholinocarbonylbutyl)-imidazol-4-yl, 1-(5-morpholinocarbonylpentyl)-imidazol-4-yl, 1-(6-morpholinocarbonylhexyl)-imidazol-4-yl, 1-(7-morpholinocarbonylheptyl)-imidazol-4-yl, 1-thiomorpholinocarbonylmethyl-imidazol-4-yl, 1-(2-thiomorpholinocarbonylethyl)-imidazol-4-yl, 1-(3-thiomorpholinocarbonylpropyl)-imidazol-4-yl, 1-(4-thiomorpholinocarbonylbutyl)-imidazol-4-yl, 1-(5-thiomorpholinocarbonylpentyl)-imidazol-4-yl, 1-(6-thiomorpholinocarbonylhexyl)-imidazol-4-yl, 1-(7-thiomorpholinocarbonylheptyl)-imidazol-4-yl, 1-oxidothiomorpholinocarbonylmethyl-imidazol-4-yl, 1-(2-oxidothiomorpholinocarbonylethyl)imidazol-4-yl, 1-(3-oxidothlomorpholinocarbonylpropyl)-imidazol-4-yl, 1-(4-oxidothiomorpholinocarbonylbutyl)-imidazol-4-yl, 1-(5-oxidothiomorpholinocarbonylpentyl)-imidazol-4-yl, 1-(6-oxidothiomorpholinocarbonylhexyl)-imidazol-4-yl, 1-(7-oxidothiomorpholinocarbonylheptyl)-imidazol-4-yl, 1-carboxymethyl-2-methyl-imidazol-4-yl, 1-(2-carboxyethyl)-2-methyl-imidazol-4-yl, 1-(3-carboxypropyl)-2-methyl-imidazol-4-yl, 1-(4-carboxybutyl)-2-methyl-imidazol-4-yl, 1-(5-carboxypentyl)-2-methyl-imidazol-4-yl, 1-(6-carboxyhexyl)-2-methyl-imidazol-4-yl, 1-(7-carboxyheptyl)-2-methyl-imidazol-4-yl, 1-methoxycarbonylmethyl-2-methyl-imidazol-4-yl, 1-(2-methoxycarbonylethyl)-2-methylimidazol-4-yl, 1-(3-methoxycarbonylpropyl)-2-methyl-imidazol-4-yl, 1-(4-methoxycarbonylbutyl)-2-methyl-imidazol-4-yl, 1-(5-methoxycarbonylpentyl)-2-methylimidazol-4-yl, 1-(6-methoxycarbonylhexyl)-2-methyl-imidazol-4-yl, 1-(7-methoxycarbonylheptyl)-2-methyl-imidazol-4-yl, 1-ethoxycarbonylmethyl-2-methyl-imidazol-4-yl, 1-(2-ethoxycarbonylethyl)-2-methyl-imidazol-4-yl, 1-(3-ethoxycarbonylpropyl)-2-methylimidazol-4-yl, 1-(4-ethoxycarbonylbutyl)-2-methyl-imidazol-4-yl, 1-(5-ethoxycarbonylpentyl)-2-methyl-imidazol-4-yl, 1-(6-ethoxycarbonylhexyl)-2-methyl-imidazol-4-yl, 1-(7-ethoxycarbonylheptyl)-2-methyl-imidazol-4-yl, 1-n-propoxycarbonylmethyl-2-methylimidazot-4-yl, 1-(2-n-propoxycarbonylethyl)-2-methyl-imidazol-4-yl, 1-(3-n-propoxycarbonylpropyl)-2-methyl-imidazol-4-yl, 1-(4-n-propoxycarbonylbutyl)-2-methyl-imidazol-4-yl, 1-(5-n-propoxycarbonylpentyl)-2-methyl-imidazol-4-yl, 1-(6-n-propoxycarbonylhexyl)-2-methyl-imidazol-4-yl, 1-(7-n-propoxycarbonylheptyl)-2-methyl-imidazol-4-yl, 1-isopropoxycarbonylmethyl- 2-methyl-imidazol-4-yl, 1-(2-isopropoxycarbonylethyl)-2-methylimidazol-4-yl, 1-(3-isopropoxycarbonylpropyl)-2-methyl-imidazol-4-yl, 1-(4-isopropoxycarbonylbutyl)-2-methyl-imidazol-4-yl, 1-(5-isopropoxycarbonylpentyl)-2-methyl-imidazol-4-yl, 1-(6-isopropoxycarbonylhexyl)-2-methyl-imidazol-4-yl, 1-(7-isopropoxycarbonylheptyl)-2-methyl-imidazol-4-yl, 1-aminocarbonylinethyl-2-methyl-imidazol-4-yl, 1-(2-aminocarbonylethyl)-2-methyl-imidazol-4-yl, 1-(3-aminocarbonylpropyl)-2-methyl-imidazol-4-yl, 1-(4-aminocarbonylbutyl)-2-methyl-imidazol-4-yl, 1-(5-aminocarbonylpentyl)-2-methyl-imidazol-4-yl, 1-(6-aminocarbonylhexyl)-2-methyl-imidazol-4-yl, 1-(7-aminocarbonylheptyl)-2-methyl-imidazol-4-yl, 1-methylaminocarbonylmethyl-2-methyl-imidazol-4-yl, 1-(2-methylaminocarbonylethyl)-2-methyl-imidazol-4-yl, 1-(3-methylaminocarbonylpropyl)-2-methyl-imidazol-4-yl, 1-(4-methylaminocarbonylbutyl)-2-methyl-imidazol-4-yl, 1-(5-methylaminocarbonylpentyl)-2-methyl-imidazol-4-yl, 1-(6-methylaminocarbonylhexyl)-2-methyl-imidazol-4-yl, 1-(7-methyl-amino-carbonyl-heptyl)-2-methyl-imidazol-4-yl, 1-ethylamino-carbonyl-methyl-2-methyl-imidazol-4-yl, 1-(2-ethylaminocarbonylethyl)-2-methylimidazol-4-yl, 1-(3-ethylaminocarbonylpropyl)-2-methyl-imidazol-4-yl, 1-(4-ethylaminocarbonylbutyl)-2-methyl-imidazol-4-yl, 1-(5-ethylaminocarbonylpentyl)-2-methyl-imidazol-4-yl, 1-(6-ethylaminocarbonylhexyl)-2-methyl-imidazol-4-yl, 1-(7-ethylaminocarbonylheptyl)-2-methyl-imidazol-4-yl, 1-n-propylaminocarbonylmethyl-2-methyl-imidazol-4-yl, 1-(2-*n*-propyl-amino-carbonyl-ethyl)-2-methyl-imidazol-4-yl, 1-(3-n-propyl-amino-carbonylpropyl)-2-methyl-imidazol-4-yl, 1-(4-*n*-propylaminocarbonylbutyl)-2-methyl-imidazol-4-yl, 1-(5-n-propylaminocarbonylpentyl)-2-methyl-imidazol-4-yl, 1-(6-n-propylaminocarbonylhexyl)-2-methyl-imidazol-4-yl, 1-(7-n-propylaminocarbonylheptyl)-2-methyl-imidazol-4-yl, 1-isopropylaminocarbonylmethyl-2-methyl-imidazol-4-yl, 1-(2-isopropylaminocarbonylethyl)-2-methyl-imidazol-4-yl, 1-(3-isopropylaminocarbonyl-propyl)-2-methyl-imidazol-4-yl, 1-(4-isopropylamino-carbonylbutyl)-2-methyl-imidazol-4-yl, 1-(5-isopropylaminocarbonylpentyl)-2-methyl-imidazol-4-yl, 1-(6-isopropylaminocarbonylhexyl)-2-methyl-imidazol-4-yl, 1-(7-isopropylaminocarbonylheptyl)-2-methyl-imidazol-4-yl, 1-dimethylaminocarbonylmethyl-2-methyl-imidazol-4-yl, 1-(2-dimethylaminocarbonylethyl)-2-methyl-imidazol-4-yl, 1-(3-dimethylaminocarbonylpropyl)-2-methyl-imidazol-4-yl, 1-(4-dimethylaminocarbonylbutyl)-2-methyl-imidazol-4-yl, 1-(5-dimethylaminocarbonylpentyl)-2-methyl-imidazol-4-yl, 1-(b-dimethylaminocarbonyl-hexyl)-2-methyl-iinidazol-4-yl, 1-(7-dimethylaminocarbonylheptyl)-2-methyl-imidazol-4-yl, 1-diethylaminocarbonylmethyl-2-methyl-imidazol-4-yl, 1-(2-diethylaminocarbonylethyl)-2-methyl-imidazol-4-yl, 1-(3-diethylaminocarbonylpropyl)-2-methyl-imidazol-4-yl, 1-(4-diethylaminocarbonylbutyl)-2-methyl-imidazol-4-yl, 1-(5-diethylaminocarbonylpentyl)-2-methyl-imidazol-4-yl, 1-(6-diethylaminocarbonylhexyl)-2-methyl-imidazol-4-yl, 1-(7-diethylaminocarbonylheptyl)-2-methyl-imidazol-4-yl, 1-di-n-propylaminocarbonylmethyl-2-methyl-imidazol-4-yl, 1-(2-di-n-propylaminocarbonylethyl)-2-methyl-imidazol-4-yl, 1-(3-di-n-propylaminocarbonylpropyl)-2-methyl-imidazol-4-yl, 1-(4-di-n-propylaminocarbonylbutyl)-2-methyl-imidazol-4-yl, 1-(5-di-n-propylaminocatbonylpentyl)-2-methyl-imidazol-4-yl, 1-(6-di-n-propylamino-carbonylhexyl)-2-methyl-imidazol-4-yl, 1-(7-di-n-propylaminocarbonylheptyl)-2-methyl-imidazol-4-yl, 1-diisopropylaminocarbonylmethyl-2-methyl-imidazol-4-yl, 1-(2-diisopropylaminocarbonylethyl)-2-methylimidazol-4-yl, 1-(3-diisopropylaminocarbonylpropyl)-2-methyl-imidazol-4-yl, 1-(4-diisopropylaminocarbonylbutyl)-2-methyl-imidazol-4-yl, 1-(5-diisopropylaminocarbonylpentyl)-2-methyl-imidazol-4-yl, 1-(6-diisopropylamino-carbonylhexyl)-2-methyl-imidazol-4-yl, 1-(7-diisopropyl-aminocarbonylheptyl)-2-methyl-imidazol-4-yl, 1-morpholinocarbonylmethyl-2-methyl-imidazol-4-yl, 1-(2-morpholinocarbonylethyl)-2-methyl-imidazol-4-yl, 1-(3-morpholinocarbonylpropyl)-2-methyl-imidazol-4-yl, 1-(4-morpholinocarbonylbutyl)-2-methyl-imidazot-4-yl, 1-(5-morpholinocarbonylpentyl)-2-methyl-imidazol-4-yl, 1-(6-morpholinocarbonylhexyl)-2-methyl-imidazol-4-yl, 1-(7-morpholinocarbonylheptyl)-2-methyl-imidazol-4-yl, 1-thiomorpholinocarbonylmethyl-2-methyl-imidazol-4-yl, 1-(2-thiomorpholinocarbonylethyl)-2-methyl-imidazol-4-yl, 1-(3-thiomorpholinocarbonylpropyl)-2-methyl-imidazol-4-yl, 1-(4-thiomorpholinocarbonyl-butyl)-2-methyl-imidazol-4-yl, 1-(5-thiomorpholinocarbonylpentyl)-2-methyl-imidazol-4-yl, 1-(6-thiomorpholinocarbonylhexyl)-2-methyl-imidazol-4-yl, 1-(7-thiomorpholino-carbonyl-heptyl)-2-methyl-imidazol-4-yl, 1-oxidothio-morpholinocarbonylmethyl-2-methyl-imidazol-4-yl, 1-(2-oxidothiomorpholinocarbonylethyl)-2-methyl-imidazol-4-yl, 1-(3-oxidothio-morpholinocarbonylpropyl)-2-methyl-imidazol-4-yl, 1-(4-oxidothiomorpholino-carbonyl-butyl)-2-methylimidazol-4-yl, 1-(5-oxidothiomorpholinocarbonyl-pentyl)-2-methyl-imidazol-4-yl, 1-(6-oxidothio-morpholinocarbonylhexyl)-2-methyl-imidazol-4-yl, 1-(7-oxidothiomorpholinocarbonylheptyl)-2-methyl-imidazol-4-yl, 1-(2-hydroxyethyl)-imidazol-4-yl, 1-(3-hydroxypropyl)-imidazol-4-yl, 1-(4-hydroxybutyl)-imidazol-4-yl, 1-(2-methoxyethyl)-imidazol-4-yl, 1-(3-methoxypropyl)-imidazol-4-yl, 1-(4-methoxybutyl)-imidazol-4-yl, 1-(2-ethoxyethyl)-imidazol-4-yl, 1-(3-ethoxypropyl)-imidazol-4-yl, 1-(4-ethoxybutyl)-imidazol-4-yl, 1-(2-n-propoxyethyl)-imidazopropoxypropyl)-imidazol-4-yl, 1-(4-n-propoxybutyl)-imidazol-4-yl, 1-(2-isopropoxyethyl)-imidazol-4-yl, 1-(3-isopropoxypropyl)-imidazol-4-yl, 1-(4-isopropoxybutyl)-imidazol-4-yl, 1-(2-imidazol-1-yl-ethyl)-imidazol-4-yl, 1-(3-imidazol-1-yl-propyl)-imidazol-4-yl, 1-(4-imidazol-1-yl-butyl)-imidazol-4-yl, 1-(2,2-diphenyl-ethyl)-imidazol-4-yl, 1-(3,3-diphenyl-propyl)-imidazol-4-yl, 1-(4,4-diphenyl-butyl)-imidazol-4-yl, 1-(2-hydroxyethyl)-2-methyl-imidazol-4-yl, 1-(3-hydroxy-propyl)-2-methyl-imidazol-4-yl, 1-(4-hydroxybutyl)-2-methyl-imidazol-4-yl, 1-(2-methoxyethyl)-2-methyl-imidazol-4-yl, 1-(3-methoxypropyl)-2-methyl-imidazol-4-yl, 1-(4-methoxybutyl)-2-methyl-imidazol-4-yl, 1-(2-ethoxyethyl)-2-methyl-imidazol-4-yl, 1-(3-ethoxypropyl)-2-methyl-imidazol-4-yl, 1-(4-ethoxybutyl)-2-methyl-imidazol-4-yl, 1-(2-n-propoxyethyl)-2-methyl-imidazol-4-yl, 1-(3-n-propoxypropyl)-2-methyl-imidazol-4-yl, 1-(4-n-propoxybutyl)-2-methyl-imidazol-4-yl, 1-(2-isopropoxyethyl)-2-methyl-imidazol-4-yl, 1-(3-isopropoxypropyl)-2-methyl-imidazol-4-yl, 1-(4-isopropoxybutyl)-2-methyl-imidazol-4-yl, 1-(2-imidazol-1-yl-ethyl)-2-methyl-imidazol-4-yl, 1-(3-imidazol-1-yl-propyl)-2-methyl-imidazol-4-yl, 1-(4-imidazol-1-yl-butyl)-2-methylimidazol-4-yl, 1-(2,2-diphenyl-ethyl)-2-methyl-imidazol-4-yl, 1-(3,3-diphenyl-propyl)-2-methyl-imidazol-4-yl, 1-(4,4-diphenyl-butyl)-2-methyl-imidazol-4-yl, 1-[2-(2-methoxyethoxy)-ethyl]-imidazol-4-yl, 1-[3-(2-methoxyethoxy)-propyl]-imidazol-4-yl, 1-[4-(2-methoxyethoxy)-butyl]-imidazol-4-yl, 1-[2-(2-ethoxyethoxy)-ethyl]-imidazol-4-yl, 1-[3-(2-ethoxyethoxy)-propyl]-imidazol-4-yl, 1-[4-(2-ethoxyethoxy)-butyl]-imidazol-4-yl, 1-[2-(2-n-propoxyethoxy)-ethyl]-imidazol-4-yl, 1-[3-(2-n-propoxyethoxy)-propyl]-imidazol-4-yl, 1-[4-(2-n-propoxyethoxy)-butyl]-inudazol-4-yl, 1-[2-(2-isopropoxyethoxy)-ethyl]-imidazol-4-yl, 1-[3-(2-isopropoxyethoxy)-propyl]-imidazol-4-yl, 1-[4-(2-isopropoxyethoxy)-butyl]-imidazol-4-yl, 1-(2-dimethylaminoethyl)-imidazol-4-yl, 1-(2-diethylamino-ethyl)-imidazol-4-yl, 1-(2-di-n-propylamino-ethyl)-imidazol-4-yl, 1-(2-diisopropylaminoethyl)-imidazol-4-yl, 1-(3-dimethylaminopropyl)-imidazol-4-yl, 1-(3-diethylaminopropyl)-imidazol-4-yl, 1-(3-din-propylamino-propyl)-imidazol-4-yl, 1-(3-di-isopropylamino-propyl)-imidazol-4-yl, 1-(4-dimethylamino-butyl)-imidazol-4-yl, 1-(4-diethylamino-butyl)-imidazol-4-yl, 1-(4-di-n-propylamino-butyl)-imidazol-4-yl, 1-(4-diisopropylamino-butyl)-imidazol-4-yl, 1-(2-morpholino-ethyl)-imidazol-4-yl, 1-(3-morpholino-propyl)-imidazol-4-yl, 1-(4-morpholinobutyl)-imidazol-4-yl, 1-(2-pyrrolidino-ethyl)-imidazol-4-yl, 1-(3-pyrrolidino-propyl)-imidazol-4-yl, 1-(4-pyrrolidino-butyl)-imidazol-4-yl, 1-(2-piperidino-ethyl)-imidazol-4-yl, 1-(3-piperidino-propyl)-imidazol-4-yl, 1-(4-piperidino-butyl)-imidazol-4-yl, 1-(2-hexamethyleneiminoethyl)-imidazol-4-yl, 1-(3-hexamethyleneimino-propyl)-imidazol-4-yl, 1-(4-hexamethyleneimino-butyl)-imidazol-4-yl, 1-(2-thiomorpholino-ethyl)-imidazol-4-yl, 1-(3-thiomorpholino-propyl)-imidazol-4-yl, 1-(4-thiomorpholino-butyl)-imidazol-4-yl, 1-[2-(1-oxido-thiomorpholino)-ethyl]-imidazol-4-yl, 1-[3-(1-oxido-thiomorpholino)-propyl]-imidazol-4-yl or 1-[4-(1-oxido-thiomorpholino)-butyl]-imidazol-4-yl group, purine, pyrimidine, pyridine, pyrrole, indole, imidazole, pyrazole, quinazoline, pyridazine, pyrazine, cinnoline, phthalazine, quinoxaline, xanthine, hypoxanthine, adenine, guanine, cytosine, uracil, thymine, pteridine, 5-azacytosine, 5-fluorocytosine, 5-azauracil, 5-fluorouracil, 6-chloropurine, triazolopyridine, imidazolepyridine, imidazolotriazine, pyrrolopyrimidine, or pyrazolopyrimidine, 1-triphenylmethyl-tetrazolyl or 2-triphenylmethyl-tetrazolyl group, 2-isopropyl-pyridazin-3-on-6-yl, 2-benzyl-pyridazin-3-on-6-yl, 2-(2-phenylethyl)-pyridazin-3-on-6-yl, 2-(3-phenylpropyl)-pyridazin-3-on-6-yl, 4-methyl-pyridazin-3-on-6-yl, 5-methylpyridazin-3-on-6-yl, 4,5-dimethyl-pyridazin-3-on-6-yl, 2,4-dimethyl-p

The heteroaromatic group can be optionally substituted as described above for aryl. The heterocyclic group can be optionally substituted with one or more moieties selected from the group consisting of alkyl, halo, haloalkyl, hydroxyl, carboxyl, acyl, acyloxy, amino, amido, carboxyl derivatives, alkylamino, dialkylamino, arylamino, alkoxy, aryloxy, nitro, cyano, sulfonic acid, thiol, imine, sulfonyl, sulfanyl, sulfinyl, sulfamonyl, ester, carboxylic acid, amide, phosphonyl, phosphinyl, phosphoryl, phosphine, thioester, thioether, acid halide, anhydride, oxime, hydrozine, carbamate, phosphonic acid, phosphonate, or any other viable functional group that does not inhibit the pharmacological activity of this compound, either unprotected, or protected as necessary, as known to those skilled in the art, for example, as taught in Greene, *et al*., Protective Groups in Organic Synthesis, John Wiley and Sons, Second Edition, 1991, hereby incorporated by reference. The heteroaromatic can be partially or totally hydrogenated as desired. As a nonlimiting example, dihydropyridine can be used in place of pyridine. Functional oxygen and nitrogen groups on the heteroaryl group can be protected as necessary or desired. Suitable protecting groups are well known to those skilled in the art, and include trimethylsilyl, dimethylhexylsilyl, *t*-butyldimethylsilyl, and *t*-butyldiphenylsilyl, trityl or substituted trityl, alkyl groups, acyl groups such as acetyl and propionyl, methanesulfonyl, and p-toluenesulfonyl.

The term "protected" as used herein and unless otherwise defined refers to a group that is added to an oxygen, nitrogen, or phosphorus atom to prevent its further reaction or for other purposes. A wide variety of oxygen and nitrogen protecting groups are known to those skilled in the art of organic synthesis, for example, as taught in Greene, *et al*., Protective Groups in Organic Synthesis, John Wiley and Sons, Second Edition, 1991.

As used herein, the term "substantially free of" or "substantially in the absence of" refers to a nucleoside composition that includes at least 95% to 98%, or more preferably, 99% to 100%, of the designated enantiomer of that nucleoside. In a preferred embodiment, the compound is administered substantially free of its corresponding β-D isomer.

Any of the compounds described herein for combination or alternation therapy can be administered as any derivative that upon administration to the recipient, is capable of providing directly or indirectly, the parent compound, or that exhibits activity itself. Nonlimiting examples are the pharmaceutically acceptable salts (alternatively referred to as "physiologically acceptable salts"), and compounds which have been alkylated or acylated at the appropriate positions, typically, hydroxyl or amino positions. The modifications can affect the biological activity of the compound, in some cases increasing the activity over the parent compound. This can easily be assessed by preparing the derivative and testing its antiviral activity according to known methods.

As used herein, the term "pharmaceutically acceptable salts" refers to salts that retain the desired biological activity of the herein-identified compounds and exhibit minimal undesired toxicological effects. Non-limiting examples of such salts are (a) acid addition salts formed with inorganic acids (for example, hydrochloric acid, hydrobromic acid, sulfuric acid, phosphoric acid, nitric acid, and the like), and salts formed with organic acids such as amino acid, acetic acid, oxalic acid, tartaric acid, succinic acid, malic acid, ascorbic acid, benzoic acid, tannic acid, palmoic acid, alginic acid, polyglutamic acid, naphthalenesulfonic acid, napbthalenedisulfonic acid, and polygalacturonic acid; (b) base addition salts formed with metal cations such as zinc, calcium, bismuth, barium, magnesium, aluminum, copper, cobalt, nickel, cadmium, sodium, potassium, and the like, or with a cation formed from ammonia, *N*,*N*-dibenzylethylenediamine, D-glucosamine, tetraethylammonium, or ethylenediamine; or (c) combinations of (a) and (b); e.g., a zinc tannate salt or the like.

The compound can be converted into a pharmaceutically acceptable ester by reaction with an appropriate esterifying agent, for example, an acid halide or anhydride. The compound or its pharmaceutically acceptable derivative can be converted into a pharmaceutically acceptable salt thereof in a conventional manner, for example, by treatment with an appropriate base. The ester or salt of the compound can be converted into the parent compound, for example, by hydrolysis.

In the practice of this invention, the administration of the composition may be effected by any of the well known methods including, but not limited to, oral, intravenous, intraperitoneal, intramuscular or subcutaneous or topical administration.

### Pharmaceutical Compositions

Humans suffering from any of the disorders described herein can be treated by administering to the patient an effective amount of the active compound or a pharmaceutically acceptable derivative or salt thereof in the presence of a pharmaceutically acceptable carrier or diluent. The active materials can be administered by any appropriate route, for example, orally, parenterally, intravenously, intradermally, subcutaneously, or topically, in liquid or solid form.

A preferred dose of the compound for all of the abovementioned conditions will be in the range from about 1 to 50 mg/kg, preferably 1 to 20 mg/kg, of body weight per day, more generally 0.1 to about 100 mg per kilogram body weight of the recipient per day. The effective dosage range of the pharmaceutically acceptable derivatives can be calculated based on the weight of the parent nucleoside to be delivered. If the derivative exhibits activity in itself, the effective dosage can be estimated as above using the weight of the derivative, or by other means known to those skilled in the art.

The compound is conveniently administered in unit any suitable dosage form, including but not limited to one containing 7 to 3000 mg, preferably 70 to 1400 mg of active ingredient per unit dosage form. A oral dosage of 50-1000 mg is usually convenient.

Ideally the active ingredient should be administered to achieve peak plasma concentrations of the active compound of from about 0.2 to 70 pM, preferably about 1.0 to 10 µM. This may be achieved, for example, by the intravenous injection of a 0.1 to 5% solution of the active ingredient, optionally in saline, or administered as a bolus of the active ingredient.

The concentration of active compound in the drug composition will depend on absorption, inactivation, and excretion rates of the drug as well as other factors known to those of skill in the art. It is to be noted that dosage values will also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed composition. The active ingredient may be administered at once, or may be divided into a number of smaller doses to be administered at varying intervals of time.

A preferred mode of administration of the active compound is oral. Oral compositions will generally include an inert diluent or an edible carrier. They may be enclosed in gelatin capsules or compressed into tablets. For the purpose of oral therapeutic administration, the active compound can be incorporated with excipients and used in the form of tablets, troches, or capsules. Pharmaceutically compatible binding agents, and/or adjuvant materials can be included as part of the composition.

The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring. When the dosage unit form is a capsule, it can contain, in addition to material of the above type, a liquid carrier such as a fatty oil. In addition, dosage unit forms can contain various other materials which modify the physical form of the dosage unit, for example, coatings of sugar, shellac, or other enteric agents.

The compound can be administered as a component of an elixir, suspension, syrup, wafer, chewing gum or the like. A syrup may contain, in addition to the active compounds, sucrose as a sweetening agent and certain preservatives, dyes and colorings and flavors.

The compound or a pharmaceutically acceptable derivative or salts thereof can also be mixed with other active materials that do not impair the desired action, or with materials that supplement the desired action, such as antibiotics, antifungals, anti-inflammatories, or other antivirals, including other nucleoside compounds. Solutions or suspensions used for parenteral, intradermal, subcutaneous, or topical application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parental preparation can be enclosed in ampoules, disposable syringes or multiple dose vials made of glass or plastic.

If administered intravenously, preferred carriers are physiological saline or phosphate buffered saline (PBS).

In a preferred embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation.

Liposomal suspensions (including liposomes targeted to infected cells with monoclonal antibodies to viral antigens) are also preferred as pharmaceutically acceptable carriers. These may be prepared according to methods known to those skilled in the art, for example, as described in U.S. Patent No. 4,522,811 (which is incorporated herein by reference in its entirety). For example, liposome formulations may be prepared by dissolving appropriate lipid(s) (such as stearoyl phosphatidyl ethanolamine, stearoyl phosphatidyl choline, arachadoyl phosphatidyl choline, and cholesterol) in an inorganic solvent that is then evaporated, leaving behind a thin film of dried lipid on the surface of the container. An aqueous solution of the active compound or its monophosphate, diphosphate, and/or triphosphate derivatives is then introduced into the container. The container is then swirled by hand to free lipid material from the sides of the container and to disperse lipid aggregates, thereby forming the liposomal suspension.

### Anti-HIV Activity

In one embodiment, the disclosed compounds or their pharmaceutically acceptable derivatives or salts or pharmaceutically acceptable formulations containing these compounds are useful in the prevention and treatment of HIV infections and other related conditions such as AIDS-related complex (ARC), persistent generalized lymphadenopathy (PGL), AIDS-related neurological conditions, anti-HIV antibody positive and HIV-positive conditions, Kaposi's sarcoma, thrombocytopenia purpurea and opportunistic infections. In addition, these compounds or formulations can be-used prophylactically to prevent or retard the progression of clinical illness in individuals who are anti-HIV antibody or HIV-antigen positive or who have been exposed to HIV.

The ability of nucleosides to inhibit HIV can be measured by various experimental techniques. One technique, described in detail below, measures the inhibition of viral replication in phytohemagglutinin (PHA) stimulated human peripheral blood mononuclear (PBM) cells infected with HIV-1 (strain LAV). The amount of virus produced is determined by measuring the virus-coded reverse transcriptase enzyme. The amount of enzyme produced is proportional to the amount of virus produced.

### Antiviral and cytotoxicity assays

Anti-HIV-1 activity of the compounds is determined in human peripheral blood mononuclear (PBM) cells as described previously (Schinazi, R. F.; McMillan, A.; Cannon, D.; Mathis, R.; Lloyd, R. M. Jr.; Peck, A.; Sommadossi, J.-P.; St. Clair, M.; Wilson, J.; Furman, P. A.; Painter, G.; Choi, W.-B.; Liotta, D. C. *Antimicrob. Agents Chemother.* **1992,** *36*, 2423; Schinazi, R. F.; Sommadossi, J.-P.; Saalmann, V.; Cannon, D.; Xie, M.-Y.; Hart, G.; Smith, G.; Hahn, E. *Antimicrob. Agents Chemother.* **1990**, *34,* 1061). Stock solutions (20-40 mM) of the compounds were prepared in sterile DMSO and then diluted to the desired concentration in complete medium. 3'-azido-3'-deoxythymidine (AZT) stock solutions are made in water. Cells are infected with the prototype HIV-1_{LAI} at a multiplicity of infection of 0.01. Virus obtained from the cell supernatant are quantitated on day 6 after infection by a reverse transcriptase assay using poly(rA)ₙoligo(dT)₁₂₋₁₈ as template-primer. The DMSO present in the diluted solution (< 0.1%) should have no effect on the virus yield. The toxicity of the compounds can be assessed in human PBM, CEM, and Vero cells. The antiviral EC₅₀ and cytotoxicity IC₅₀ is obtained from the concentration-response curve using the median effective method described by Chou and Talalay (*Adv. Enzyme Regul*. **1984,** *22,* 27).

Three-day-old phytohemagglutinin-stimulated PBM cells (10⁶ cells/ml) from hepatitis B and HIV-1 seronegative healthy donors are infected with HIV-1 (strain LAV) at a concentration of about 100 times the 50% tissue culture infectious dose (TICD 50) per ml and cultured in the presence and absence of various concentrations of antiviral compounds.

Approximately one hour after infection, the medium, with the compound to be tested (2 times the final concentration in medium) or without compound, is added to the flasks (5 ml; final volume 10 ml). AZT is used as a positive control.

The cells are exposed to the virus (about 2 x 10⁵ dpm/ml, as determined by reverse transcriptase assay) and then placed in a CO₂ incubator. HIV-1 (strain LAV) is obtained from the Center for Disease Control, Atlanta, Georgia. The methods used for culturing the PBM cells, harvesting the virus and determining the reverse transcriptase activity are those described by McDougal et al. (*J. Immun*. *Meth*. **1985,** *76*, 171-183) and Spira et al. (*J. Clin*. *Meth*. **1987**, *25*, 97-99), except that fungizone was not included in the medium (see Schinazi, et al., *Antimicrob. Agents Chemother*. **1988**, *32*, 1784-1787; Id., **1990**, *34*, 1061-1067).

On day six, the cells and supernatant are transferred to a 15 ml tube and centrifuged at about 900 g for 10 minutes. Five ml of supernatant are removed and the virus concentrated by centrifugation at 40,000 rpm for thirty minutes (Beckman 70.1 Ti rotor). The solubilized virus pellet is processed for determination of the levels of reverse transcriptase. Results are expressed in dpm/ml of sampled supernatant. Virus from smaller volumes of supernatant (1 ml) can also be concentrated by centrifugation prior to solubilization and determination of reverse transcriptase levels.

The median effective (EC₅₀) concentration is determined by the median effect-method *(Antimicrob. Agents Chemother.* **1986***, 30, 491-498).* Briefly, the percent inhibition of virus, as determined from measurements of reverse transcriptase, is plotted versus the micromolar concentration of compound. The EC₅₀ is the concentration of compound at which there is a 50% inhibition of viral growth.

Mitogen stimulated uninfected human PBM cells (3.8 x 10⁵ cells/ml) can be cultured in the presence and absence of drug under similar conditions as those used for the antiviral assay described above. The cells are counted after 6 days using a hemacytometer and the trypan blue exclusion method, as described by Schinazi et al., *Antimicrobial Agents and Chemotherapy,* **1982**, *22*(3), 499. The IC₅₀ is the concentration of compound which inhibits 50% of normal cell growth.

### Anti-Hepatitis B Activity

The ability of the active compounds to inhibit the growth of hepatitis virus in 2.2.15 cell cultures (HepG2 cells transformed with hepatitis virion) can be evaluated as described in detail below.

A summary and description of the assay for antiviral effects in this culture system and the analysis of HBV DNA has been described (Korba and Milman, *Antiviral Res.* **1991,** *15*, 217). The antiviral evaluations are optimally performed on two separate passages of cells. All wells, in all plates, are seeded at the same density and at the same time.

Due to the inherent variations in the levels of both intracellular and extracellular HBV DNA, only depressions greater than 3.5-fold (for HBV virion DNA) or 3.0-fold (for HBV DNA replication intermediates) from the average levels for these HBV DNA forms in untreated cells are considered to be statistically significant (P<0.05). The levels of integrated HBV DNA in each cellular DNA preparation (which remain constant on a per cell basis in these experiments) are used to calculate the levels of intracellular HBV DNA forms, thereby ensuring that equal amounts of cellular DNA are compared between separate samples.

Typical values for extracellular HBV virion DNA in untreated cells ranged from 50 to 150 pg/ml culture medium (average of approximately 76 pg/ml). Intracellular HBV DNA replication intermediates in untreated cells ranged from 50 to 100 µg/pg cell DNA (average approximately 74 pg/µg cell DNA). In general, depressions in the levels of intracellular HBV DNA due to treatment with antiviral compounds are less pronounced, and occur more slowly, than depressions in the levels of HBV virion DNA (Korba and Milman, *Antiviral Res.,* **1991**, *15*, 217).

The manner in which the hybridization analyses can be performed for these experiments resulted in an equivalence of approximately 1.0 pg of intracellular HBV DNA to 2-3 genomic copies per cell and 1.0 pg/ml of extracellular HBV DNA to 3 x 10⁵ viral particles/ml.

Toxicity analyses were performed to assess whether any observed antiviral effects are due to a general effect on cell viability. The method used herein are the measurement of the uptake of neutral red dye, a standard and widely used assay for cell viability in a variety of virus-host systems, including HSV and HIV. Toxicity analyses are performed in 96-well flat bottomed tissue culture plates. Cells for the toxicity analyses are cultured and treated with test compounds with the same schedule as described for the antiviral evaluations below. Each compound are tested at 4 concentrations, each in triplicate cultures (wells "A", "B", and "C"). Uptake of neutral red dye are used to determine the relative level of toxicity. The absorbance of internalized dye at 510 nm (Aₛᵢₙ) are used for the quantitative analysis. Values are presented as a percentage of the average Aₛᵢₙ values in 9 separate cultures of untreated cells maintained on the same 96-well plate as the test compounds.

### Anti-Hepatitis C Activity

Compounds can exhibit anti-hepatitis C activity by inhibiting HCV polymerase, by inhibiting other enzymes needed in the replication cycle, or by other known methods. A number of assays have been published to assess these activities.

WO 97/12033, filed on September 27, 1996, by Emory University, listing C. Hagedorn and A. Reinoldus as inventors, and which claims priority to U.S.S.N. 60/004,383, filed on September 1995, describes an HCV polymerase assay that can be used to evaluate the activity of the compounds described herein. This application and invention is exclusively licensed to Triangle Pharmaceuticals, Inc., Durham, North Carolina. Another HCV polymerase assays has been reported by Bartholomeusz, et al., Hepatitis C virus (HCV) RNA polymerase assay using cloned HCV non-structural proteins; Antiviral Therapy **1996,** *1*(Supp 4),18-24.

### Treatment of Abnormal Cellular Proliferation

In an altemative embodiment, the compounds are used to treat abnormal cellular proliferation. The compound can be evaluated for activity by testing in a routine screen, such as that performed by the National Cancer Institute, or by using any other known screen, for example as described in WO 96/07413.

The extent of anticancer activity can be easily assessed by assaying the compound according to the procedure below in a CEM cell or other tumor cell line assay. CEM cells are human lymphoma cells (a T-lymphoblastoid cell line that can be obtained from ATCC, Rockville, MD). The toxicity of a compound to CEM cells provides useful information regarding the activity of the compound against tumors. The toxicity is measured as IC₅₀ micromolar). The IC₅₀ refers to that concentration of test compound that inhibits the growth of 50% of the tumor cells in the culture. The lower the IC₅₀, the more active the compound is as an antitumor agent. In general, 2'-fluoro-nucleoside exhibits antitumor activity and can be used in the treatment of abnormal proliferation of cells if it exhibits a toxicity in CEM or other immortalized tumor cell line at less than 50 micromolar, more preferably, less than approximately 10 micromolar, and most preferably, at less than 1 micromolar. Drug solutions, including cycloheximide as a positive control, are plated in triplicate in 50 µl growth medium at 2 times the final concentration and allowed to equilibrate at 37°C in a 5% CO₂ incubator. Log phase cells are added in 50 µL growth medium to a final concentration of 2.5 x 10³ (CEM and SK-MEL-28), 5 x 10³ (MMAN, MDA-MB-435s, SKMES-1, DU-145, LNCap), or 1 x 10⁴ (PC-3, MCF-7) cells/well and incubated for 3 (DU-145, PC-3, MMAN), 4 (MCF-7, SK-MEL-28, CEM), or 5 (SK-MES-1, MDA-MB-435s, LNCaP) days at 37°C under a 5% CO₂ air atmosphere. Control wells include media alone (blank) and cells plus media without drug. After growth period, 15 µL of Cell Titer 96 kit assay dye solution (Promega, Madison, WI) are added to each well and the plates are incubated 8 hr at 37°C in a 5% CO₂ incubator. Promega Cell Titer 96 kit assay stop solution is added to each well and incubated 4-8 hr in the incubator. Absorptance is read at 570 nm, blanking on the medium-only wells using a Biotek Biokinetics plate reader (Biotek, Winooski, VT). Average percent inhibition of growth compared to the untreated control is calculated. IC₅₀, IC₉₀, slope and r value are calculated by the method of Chou and Talalay. Chou T-C, Talalay P. Quantitative analysis of dose-effect relationships: The combined effects of multiple drugs or enzyme inhibitors. Adv Enzyme Regul **1984**, *22*, 27-55.

The active compound can be administered specifically to treat abnormal cell proliferation, and in particular, cell hyperproliferation. Examples of abnormal cell proliferation include, but are not limited to: benign tumors, including, but not limited to papilloma, adenoma, firoma, chondroma, osteoma, lipoma, hemangioma, lymphangioma, leiomyoma, rhabdomyoma, meningioma, neuroma, ganglioneuroma, nevus, pheochromocytoma, neurilemona, fibroadenoma, teratoma, hydatidiform mole, granuosatheca, Brenner tumor, arrhenoblastoma, hilar cell tumor, sex cord mesenchyme, interstitial cell tumor, and thyoma as well as proliferation of smooth muscle cells in the course of development of plaques in vascular tissue; malignant tumors (cancer), including but not limited to carcinoma, including renal cell carcinoma, prostatic adenocarcinoma, bladder carcinoma, and adenocarcinoma, fibrosarcoma, chondrosarcoma, osteosarcoma, liposarcoma, hemangiosarcoma, lymphangiosarcoma, leiomyosarcoma, rhabdomyosarcoma, myelocytic leukemia, erythroleukemia, multiple myeloma, glioma, meningeal sarcoma, thyoma, cystosarcoma phyllodes, nephroblastoma, teratoma choriocarcinoma, cutaneous T-cell lymphoma (CTCL), cutaneous tumors primary to the skin (for example, basal cell carcinoma, squamous cell carcinoma, melanoma, and Bowen's disease), breast and other tumors infiltrating the skin, Kaposi's sarcoma, and premalignant and malignant diseases of mucosal tissues, including oral, bladder, and rectal diseases; preneoplastic lesions, mycosis fungoides, psoriasis, dermatomyositis, rheumatoid arthritis, viruses (for example, warts, herpes simplex, and condyloma acuminata), molluscum contagiosum, premalignant and malignant diseases of the female genital tract (cervix, vagina, and vulva). The compounds can also be used to induce abortion.

In this embodiment, the active compound, or its pharmaceutically acceptable salt, is administered in an effective treatment amount to decrease the hyperproliferation of the target cells. The active compound can be modified to include a targeting moiety that concentrates the compound at the active site. Targeting moieties can include an antibody or antibody fragment that binds to a protein on the surface of the target cell, including but not limited to epidermal growth factor receptor (EGFR), c-Esb-2 family of receptors and vascular endothelial growth factor (VEGF).

### Preparation of the Compound

The compounds of this invention can be prepared, for example, according to the following methods.

### I. From L-Ribose

This invention includes the synthesis of 3',5'-di-O-protected β-L-ribonucleosides, which can be represented by the following general structure (I), followed by deoxygenation of the 2'-hydroxyl group (first approach), or the preparation of an 2'-S-bridged cyclonucleosides (II) from L-ribose, followed by desulfurization (second approach). 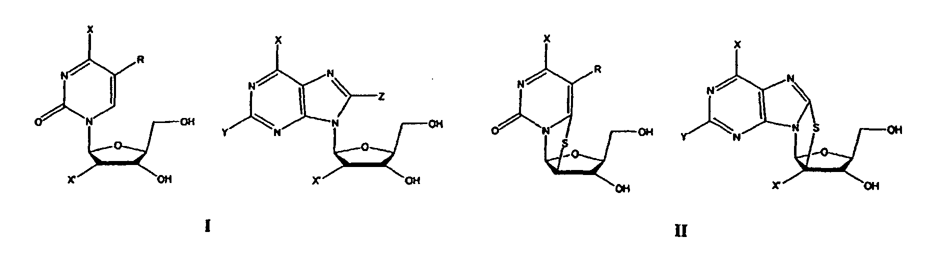 wherein
Z is H, F, Cl, Br, I, CN or NH₂;
X and Y are independently H, OH, OR, SH, SR, NH₂, NHR' or NR'R";
X' = Cl, Br, I, 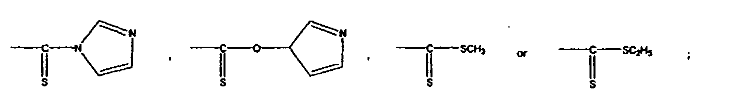 R is an alkyl, aralkyl, H, F, Cl, Br, I, NO₂, NH₂, NHR¹, NR¹R², OH, OR¹, SH, SR¹, CN, CONH₂, CSNH₂, CO₂H, CO₂R¹, CH₂CO₂H, CH₂CO₂R¹, CH=CHR¹, CH₂CH=CHR¹ or C≡CR¹.
R¹ and R² are independently lower alkyl of C₁ - C₆, e.g., methyl, ethyl, propyl, butyl, and alkyl possessing 6 carbons or less; cyclic, branched, or straight chains; unsubstituted or substituted wherein the alkyl bears one, two, or more substituents, including but not limited to, amino, carboxyl, hydroxyl or phenyl.

### I-1. First Approach via 2'-O-thiocarbonyl intermediate

### I-1-a.

1-*O*-Acetyl-2,3,5-tri-*O*-acyl-L-ribofuranose **(1, Scheme 1)** is readily available from L-ribose. For example, acetylation of L-ribose, by the same procedure that converts D-ribose into tetra-O-acetyl-D-ribofuranose (Zinner, H. *Chem. Ber.* **1950,** *83,* 517.), gives tetra-*O*-acetyl-L-ribofuranose (1, R = R¹ = Ac) which can be converted into 1-bromo or 1-chloro sugar **(2,** R¹ = Ac) by treatment with HBr or HCl in a suitable solvent such as diethyl ether or methylene chloride at ambient temperature. Reaction of 2 with a purine base and NaH in an inert solvent, such as acetonitrile or nitromethane, at a suitable temperature, for example, of from -20 °C to 120 °C, preferably from 25 °C to 82 °C, results in the formation of the corresponding purine nucleoside (3, B = purine) (Kazimierczuk, Z.; Cottom, H. B.; Revankar, G. R.; Robins, R. K. *J. Am. Chem. Soc.* **1984,** *106, 6379.)* Direct treatment of 1 with silylated pyrimidine base in an inert solvent such as acetonitrile, methylene chloride, dichloroethane or nitromethane in the presence of a Lewis acid, such as SnCl₂, TiCl₄, TMSOTf or a like at a suitable temperature, for example, of from -20 °C to 100 °C, preferably 25 °C to 80 °C, for a period of from 15 minutes to one week, preferably from 30 minutes to 6 hours, will give the corresponding protected nucleoside **(3,** B = pyrimidine) (Niedballa, U.; Vorbruggen, H. *J. Org. Chem.* **1974,** *39*, 3654; Vorbruggen, H.; Krolikiewicz, K.; Bennua, B. *Chem. Ber.* **1981,** *114,* 1256; Vorbruggen, H.; Hofle, G. *Chem. Ber.* **1981,** *114*, 1256.). Deprotection of **3** with ammonia or alkali metal alkoxide in alcohol, preferably ammonia in methanol or sodium alkoxide in methanol, at a temperature of from -20 °C to 100 °C, preferably from 25 °C to 80 °C, for a period of from 5 minutes to 3 days, preferably from 30 minutes to 4 hours, gives the free nucleoside **(4).** Selective acylation of **4** in the presence of dibutyltin oxide affords the 2'-*O*-protected nucleoside **(5,** R² = acyl) which is converted into 3',5'-di-O-substituted compound **(6).** The substituent at 3' and 5'-positions in **6** is stable to base but removable by other means, such as acid hydrolysis, hydrogenolysis, photolysis or fluoride action, and includes, but not limited to, tetrahydropyranyl, benzyl, *o*-nitrobenzyl, *t*-butyldimethylsilyl or *t*-butyldiphenylsilyl group. Removal of the 2'-O-acyl group of 6 with base affords **7** which is converted into the corresponding 2'-*O*-thiocarbonyl derivative **8** by treatment with 1,1'-thiocarobnyldimidazole in DMF (Pankiewicz, K. W.; Matsuda, A.; Watanabe, K. A. *J. Org. Chem*. **1982**, 47, 485.) or with phenyl chlorothiono-formate in pyridine (Robins, M. J.; Wilson, J. S.; Hansske, F. *J. Am. Chem. Soc*. **1983,** *105,* 4059.). Reduction of **8** with tributyltin hydride (Barton, D. H. R.; McCombie, S. W. *J. Chem. Soc., Perkin Trans. I*. **1975,** 1574) gives the protected 2'-deoxynucleoside **9** which, upon deprotection, furnishes the desired 2'-deoxy-L-purine nucleoside **10.** Compound **7** can also be obtained directly from **4** in the form of 3',5'-O-(1,1,3,3-tetraisopropyldisiloxan-1,3-diyl) derivative by treatment with 1,3-dichloro-1,1,3,3-tetraisopropyldisiloxane in pyridine at a temperature of from -20 °C to 115 °C, preferably from 0 °C to 44 °C, for from 3 hours to 1 week, preferably 12 hours to 3 days. Conversion of **7** into **9** can be achieved as described above. The synthesis of **10** can be achieved by dissolving **9** in an inert solvent, such as diethyl ether, tetrahydrofuran, dioxane or a like, preferably tetrahydrofuran, and treated it with 2 to 5 equivalents, preferably from 2.5 to 3 equivalents of tetra-*n*-butylammonium fluoride or triethylammonium hydrogen fluoride at a temperature from -20°C to 66 °C, preferably from 4 °C to 25 °C, for a period of 15 minutes to 24 hours, preferably from 30 minutes to 2 hours.

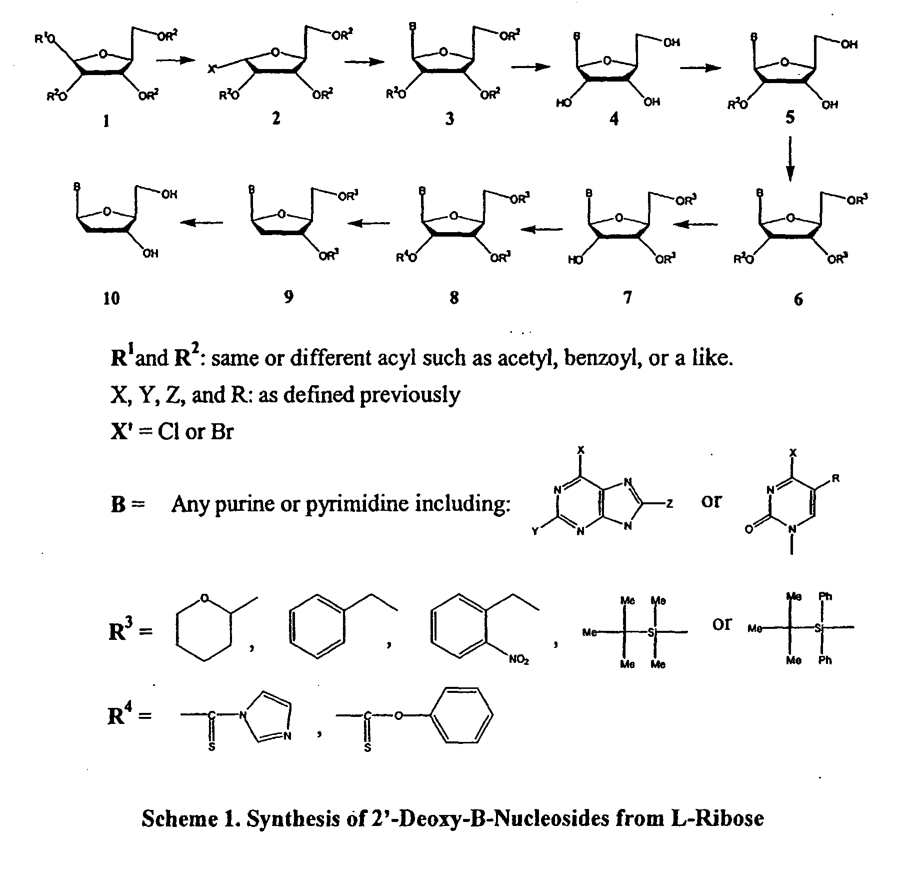

### I-1-b.

1-*O*-Acetyl-2,3,5-tri-*O*-benzoyl-β-L-ribofaranose (1, R¹ = acetyl, R² = benzoyl, **Scheme 2)** can be obtained in a one-pot reaction from L-ribose by following the procedure to prepare the D-congener of **1** (Recondo, E. F.; Rinderknecht, H. *Helv. Chim. Acta* **1959,** *42,* 1171). Treatment of **1** with HBr/CH₂Cl₂ affords the bromo-sugar **2** which, on a mild hydrolysis gives 1,3,5-tri-O-benzoyl-α-L-ribofuranose **(11,** R² = benzoyl). This conversion is well-established in the D-ribose series (Brodfuehrer, P. R.; Sapino, C.; Howell, H. G. *J. Org. Chem*. **1985,** 50, 2597). Acetylation of **13** with 1 to 20 equivalents, preferably 5 to 10 equivalents, of acetic anhydride or acetyl chloride in pyridine as solvent or in an inert solvent such as methylene chloride, chloroform, ethyl acetate, tetrahydrofuran or a like, in the presence of a base such as pyridine, 4-N,N-dimethylaminopyridine, DBU, DBN at a temperature of from -20 °C to 80 °C, preferably from 0°C to 35°C, affords the 2-*O*-acetyl derivative **12.** Condensation of **12** with silylated pyrimidine base in an inert solvent such as acetonitrile, methylene chloride, dichloroethane, nitromethane in the presence of a Lewis acid, such as SnCl₂, TiCl₄, TMSOTf or a like at a suitable temperature, for example, of from -20°C to 100°C, preferably 25°C to 80°C, for a period of from 15 minutes to one week, preferably from 30 minutes to 6 hours, will give the corresponding protected nucleoside **(14,** B = pyrimidine).

Alternatively, **12** is converted into the chloro or bromo sugar **13** (X = Cl or Br) by treatment with HCl/Et₂O or HBr/CH₂Cl₂ and then condensed with a sodio derivative of purine in an inert solvent, such as acetonitrile or nitromethane, at a temperature of from -20°C to 120°C, preferably from 25°C to 82°C, to give **14** (B = purine). Selective de-*O*-acetylation of **16** in methanol containing a few drops of hydrochloric acid (Stanek, J., *Tetrahedron Lett*. **1966,** 0000) or in a mixture of methanol and triethylamine yields the 2'-OH derivative **7** which is treated with 1,1'-thiocarobnyldiimidazole in DMF or with phenyl chlorothiono-formate in pyridine to give the 2'-thiocarbonyl derivative **8.** Reduction of **8** with tributyltin hydride gives 3',5'-di-*O*-benzoyl-2'-deoxynucleoside **9** which, upon deprotection with ammonia or alkali metal alkoxide in alcohol, preferably ammonia in methanol or sodium alkoxide in methanol, at a temperature of from -20°C to 100°C, preferably from 25°C to 80°C, for a period of from 5 minutes to 3 days, preferably from 30 minutes to 4 hours, furnishes the desired 2'-deoxy-L-nucleoside **10.**

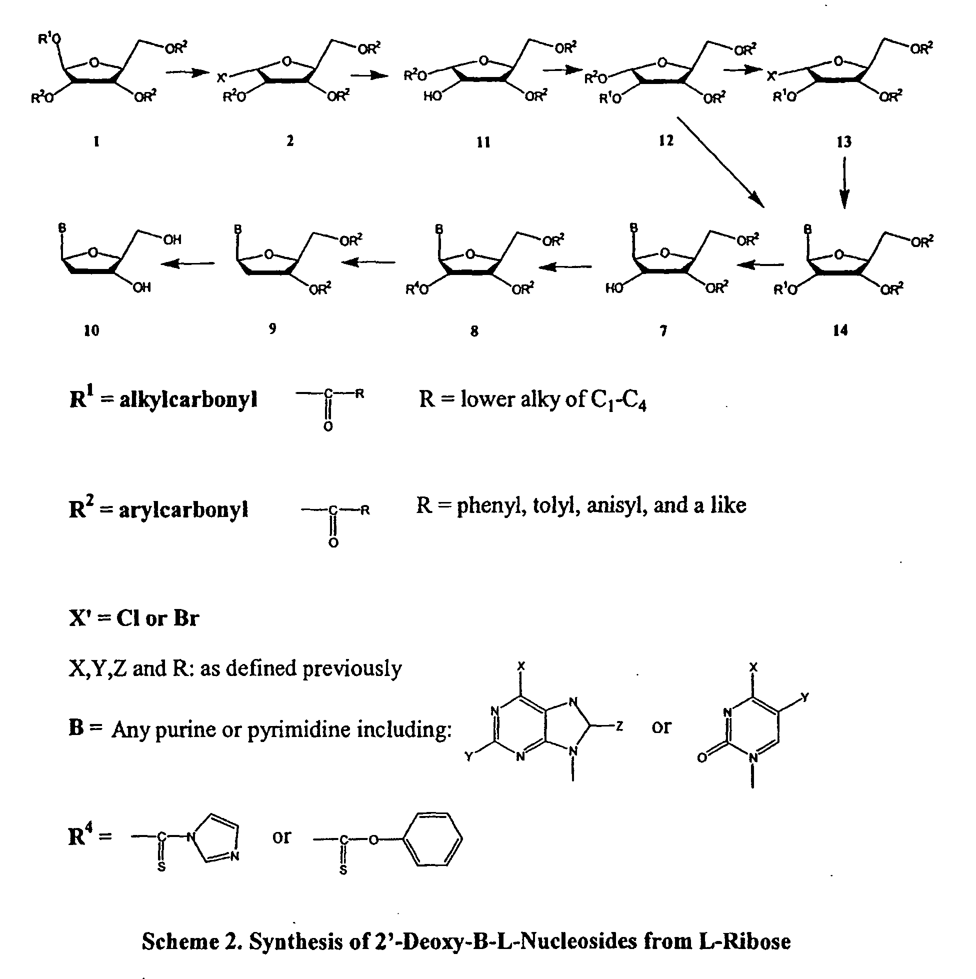

### I-2. Second Approach via S-bridged intermediate

### I-2-a.

The first of the second approach from L-ribose starts with a 1,3,5-tri-O-protected ribofuranose derivative with a leaving group at the C-2 position, such as compound **15 (Scheme 3).** Treatment of **15** with 8-thiopurine or 6-thiopyrimidine in a solvent, such as 2,3-dimethyl-2,3-butanediol, ethyl acetate, acetone, butanone, *N,N*-dimethylformamide (DMF), pyridine, dimethylsulfoxide or hexamethylphosphoric triamide, preferably DMF in the presence of base, such as alkali metal hydrogen carbonate, alkali metal carbonate, alkali metal hydroxide or alkali metal alkoxide, preferably potassium carbonate, at temperature of from 0 °C to 215 °C, preferably from 0 °C to 114 °C, affords the corresponding 2-S-substituted 2-deoxy-L-arabinose derivative **16.** Treatment of **16** with a mixture of acetic acid, acetic anhydride and sulfuric acid (acetolysis) at a temperature of from -20 °C to 40 °C, preferably from 0 °C to 25 °C, gives the cyclonucleoside **17** which yields 3',5'-di-O-benzoyl-2'-deoxy-β-L-nucleoside (**9**) by treatment with Raney nickel in ethanol, methanol, propanol or isopropanol at the reflux temperature. There is a report of somewhat similar reaction (Mizuno, Y.; Kaneko, M.; Oikawa, Y.; Ikeda, Y.; Itoh, T. *J. Am. Chem. Soc.* **1972,** 94, 4737). Alkylation of 8-mercaptoadenine with 5-deoxy-5-iodo-1,2-O-iso-propylidene-α-D-xylofuranose gives 8-S-(5-deoxy-1,2-O-iso-propylidene-β-D-xylofuranos-5-yl)adenine which, upon treatment with acetic acid/acetic anhydride/sulfuric acid affords 8,5'-anhydro-9-(5-deoxy-5-thio-β-D-xylofuranosyl)-adenine. Saponification of **9** with methanolic or ethanolic ammonia, sodium or potassium methoxide or ethoxide furnishes the desired 2'-deoxy-L-nucleoside **10.** Compound **15** in which R¹ and R² are benzoyl and O-sulfonyl-imidazolide, respectively, is known (Du, J.; Choi, Y.-S.; Lee, K.; Chun, B. K.; Hong, J. H.; Chu, C. K *Nucleosides Nucteotides* **1999,** 18, 187).

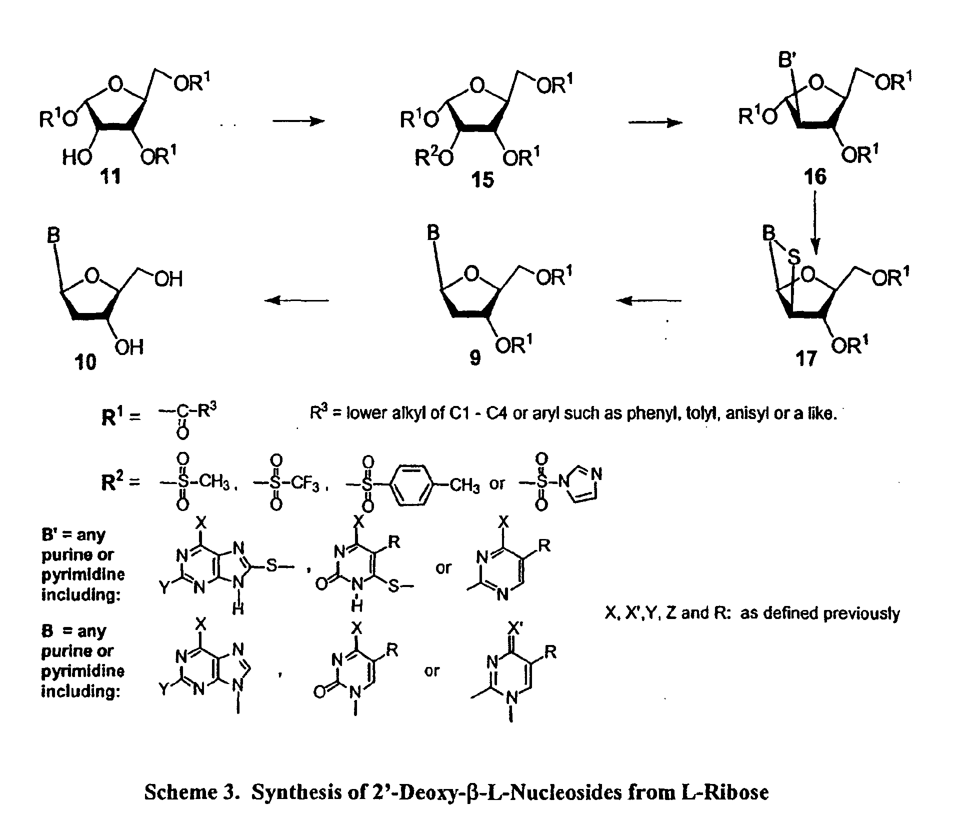

### I-2-b

Alternatively, compound **15** is treated with alkali metal thioacylate, such as sodium thioacetate or potassium thiobenzoate to give acylthio derivative **18** (R=Ac, Bz, etc) or a metal sulfide, such as KSH or NaSH, to give the 2-deoxy-2-thio-arabino derivative **19** (R=H) **(Scheme 4).** Treatment of **19** with 6-halogenopyrimidine or 8-halogenopurine affords **16.** Conversion of **16** into the targeted 2'-deoxy-β-L-nucleosides **10** is achieved as described in the previous section.

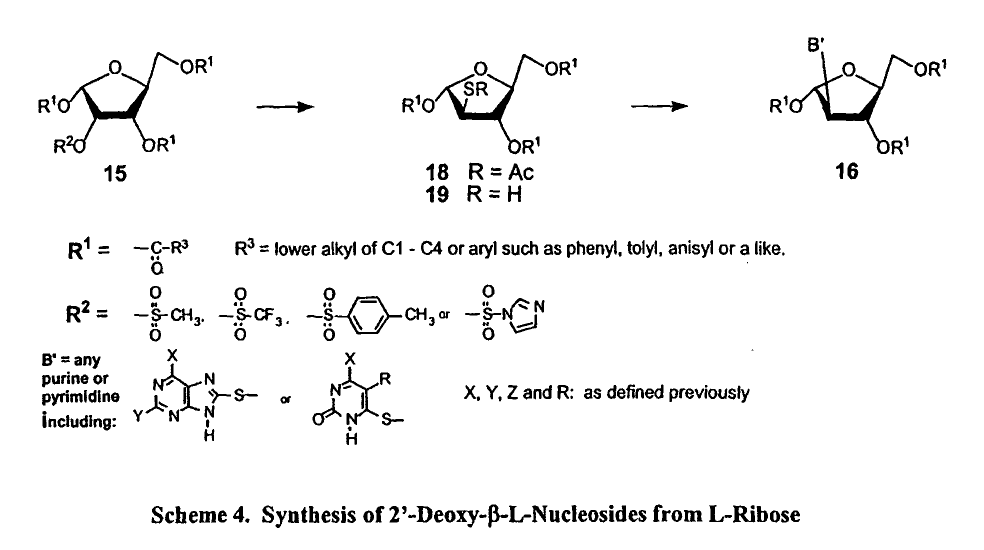

### II. From L-Xylose

In this invention, advantage is taken of the xylofuranoid structure in which the 2- and 3-hydroxy groups are in the *trans* and *cis* disposition with respect to the 4-hydroxymethyl function. Thus, exclusive introduction of a purine or pyrimidine base into the β configuration is quite easy. Selective protection of the 3'- and 5'-hydroxy groups can readily be achieved by simple acetal or ketal formation giving a nucleoside of the general structure **III** below. Deoxygenation of the 2'-hydroxy group of **III** by various methods will afford a compound of general structure **IV,** from which the 3'-hydroxy group can be epimerized with ease furnishing the targeted 2'-deoxy-β-L-nucleosides. 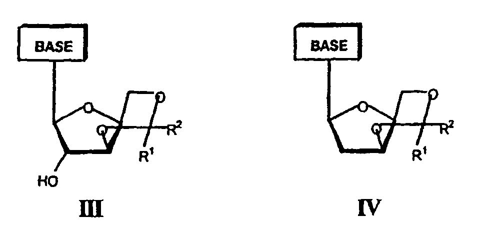 **BASE = any pyrimidine or purineincluding:** 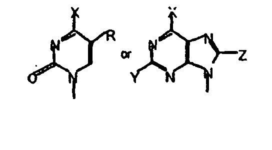 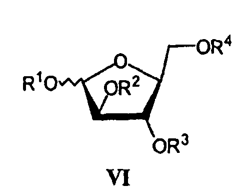 R¹ = CH₃, C₂H₅ or CH₂Ph
R² = mesyl, tosyl, triflyl, and the like.
R³ and R⁴ = same or different and are benzyl, nitrobenzyl, *p*-methylbenzyl or *p*-methoxybenzyl, *t*-butyldimethylsilyl, *t*-butyldiphenylsilyl
R = alkyl or aralkyl, H, F, Cl, Br, I, NO₂, NH₂, NHR', NR'R", OH, OR, SH, SR, CN, CONH₂,
CO₂H, CO₂R', CH₂CO₂H, CH₂CO₂R', CH=CHR.
wherein R' and R" = same or different and lower alkyl of C₁-C₆
R¹ and R² = same or different, and H, CH₃, CH₂CH₃, phenyl, tolyl, anisyl.
X and Y = same or different, and H, OH, OR, SH, SR, NH2, NHR', NR'R".
wherein R' and R" are defined as above.
Z = H, F, Cl, Br, I, CN, NH₂.

### II-1. Synthesis of compound with a general Structure III.

Treatment of L-xylose with an aldehyde, such as formaldehyde, acetaldehyde, benzaldehyde, or a ketone, such as acetone, butanone, cyclohexanone, or the corresponding, acetal or ketal, such as dimethoxymethane, acetaldehyde dimethylacetal, benzaldehyde dimethylacetal, 2,2-dimethoxypropane, 2,2-dimethoxybutane, 1,1-dimethoxycyclohexane, preferably acetone, in the presence of catalytic amount of mineral acid or Lewis acid, such as H₂SO₄, HCl, H₃PO₄, CuSO₄, ZnCl₂, preferably H₂SO₄, will afford the corresponding L-xylose-1,2;3,5-diketal or acetal, such as 1,2;3,5-di-O-isopropylidene-α-L-xylosfuranose (**19**, R¹ = R² = CH₃, **Scheme 5**). Compound **19** can be converted into a 1,2,3,5-tetra-O-acyl-L-xylofuranose of general structure (V) by several different routes. Simplest, however, is acetolysed to give L-xylofuranose tetraacetate (**20**). Since the 3,5-O-isopropylidene group is much more unstable to acid, the reaction occurs by way of the 3,5-di-O-acetyl intermediate fixing the fitranose ring. Vorbruggen reaction with silylated pyrimidine base or one pot halogenation of **20** followed by Na-purine condensation affords exclusively the protected α-L-nucleoside (**21**) which is readily 2'-de-O-acylated in base, such as methanolic ammonia, alkali metal alkoxide in alcohol, preferably sodium methoxide in methanol, to give **22**. Acetalation or ketalation as described earlier in this section will give the 3',5'-di-O-protected product **III**. Preferable method is isopropylidenation of **22** with 2,2-dimethoxypropane in acetone in the presence of a catalytic amount of p-toluenesulfonic acid yields the 3',5'-O-isopropylidene derivative (23; **III**, R¹ = R² = CH₃) in high yield.

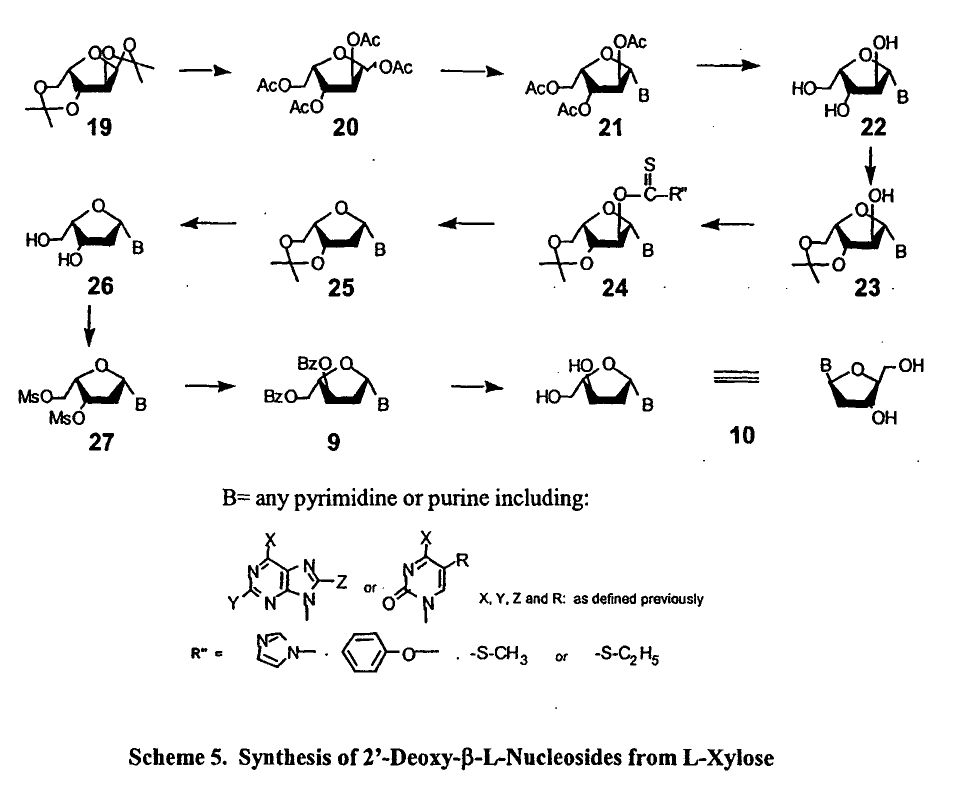

### II-2-1. via a thiocarbonyl intermediate

The free OH group can be reduced by a number of ways. For example, treatment of **23** with 1,1'- thiocarobnyldiimidazole in DMF or with phenyl chlorothionoformate in pyridine affords the 2'-thiocarbonyl derivative **24.** Alternatively, **23** can be converted into the methyl or ethyl xanthate **(24,** R = CH₃ or C₂H₅). Reduction of **24** with tributyltin hydride gives 3',5'-O-isopropylidene β-L-*threo*pentofuranosyl nucleoside **(25).** After de-O-isopropylidenation to **26** followed by mesylation results in 3',5'-di-O-mesylated nucleoside **27.** Nucleophilic replacement of the mesyl groups with sodium benzoate in DMF gives the 2'-deoxy-β-L-*erythro*pentofuranosyl nucleoside **9.** Saponification of **9** gives the desired 2'-deoxy-L-nucleoside **10.**

### II-2-2. Sulfonate intermediate

### 1. Pyrimidine nucleosides

Sulfonylation of pyrimidine nucleoside **28** with mesyl chloride, tosyl chloride, triflyl chloride or a like in pyridine gives **29** which is readily converted into the anhydro-nucleoside **30 (Scheme 6).** Nucleophilic attack on the anhydro-linkage in **30** with thioacetate or thiobenzoate affords **31** (R² = Ac or Bz). Raney nickel desulfurization affords the 2'-deoxy-β-L-nucleoside **(25).** Deprotection of **25** with 80% acetic acid to **26,** followed by sulfonylation with mesyl chloride, tosyl chloride, triflyl chloride, triflyl anhydride or a like, preferably mesyl chloride, in pyridine gives disulfonate **(27).** Treatment of **27** with alkali metal carboxylate, preferably sodium benzoate, in DMF affords the 3',5'-di-O-acyl derivative with the desired β-L-*erythro* configuration **(9).** Saponification of **9** will give the 2'-deoxy-β-L-ribonucleoside **10.**

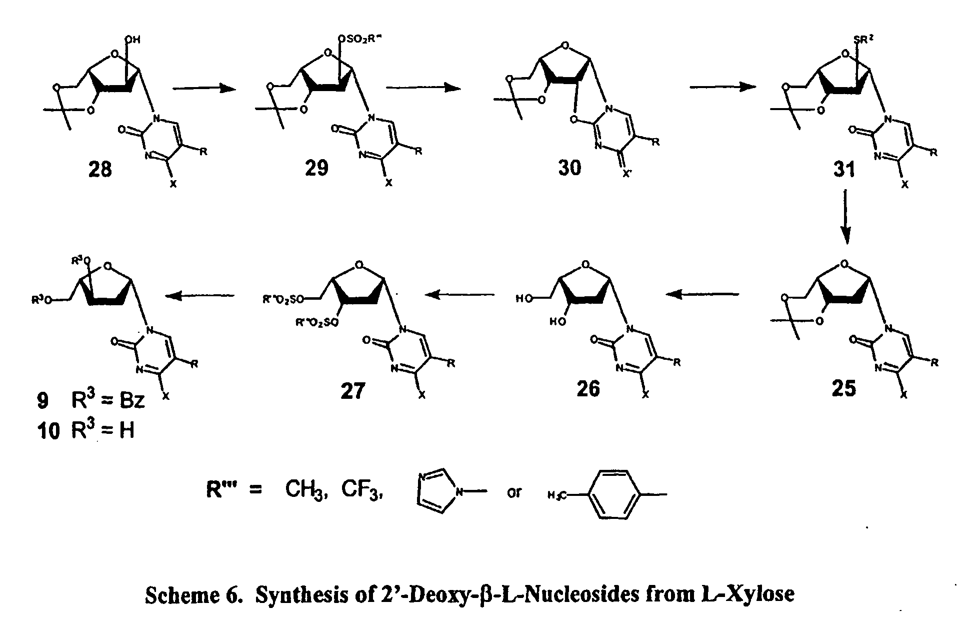

Alternatively, treatment of **29** with 1 equivalent of alkali metal hydroxide or alkoxide in alcohol, preferably sodium methoxide in methanol, affords high yield of **30** which is readily converted into the 2'-chloro or 2'-bromo derivative **(32, Scheme 7**) by treatment with tetraalkylammonium chloride or bromide, or a like. Hydrogenolysis of **32** furnishes the synthesis of **25** which is converted into the desired 2'-deoxy nucleoside **10** as described above.

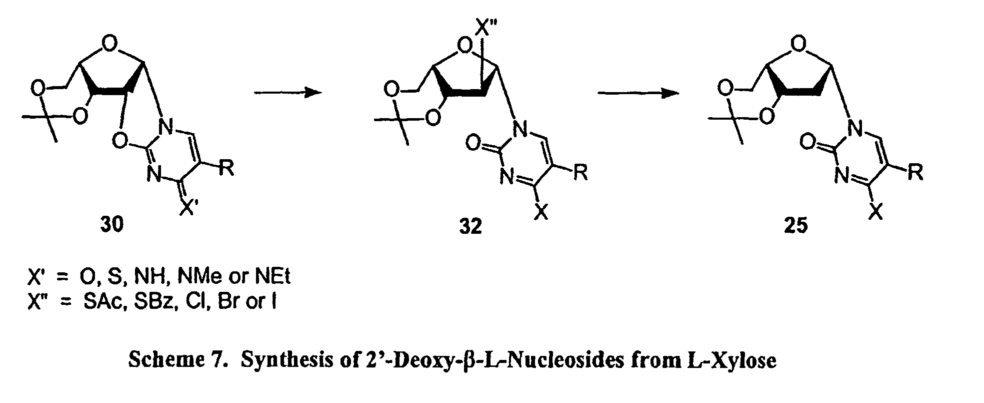

### 2. Purine nucleosides

In the case of purine nucleoside Walden inversion does not occur during the treatment of a sulfonate **33 (Scheme 8)** with a nucleophilic reagent, such as sodium thioacetate, sodium thiobenzoate, lithium chloride, lithium bromide, tetraalkylammonium chloride or tetraalkylammonium bromide, giving the L-*lyxo* nucleoside **(33,** X is in the "down" or "α" configuration). Conversion of **34** into the desired 2'-deoxy-β-L-*erythro*pentofuranosyl nucleoside **10** is achieved as described above.

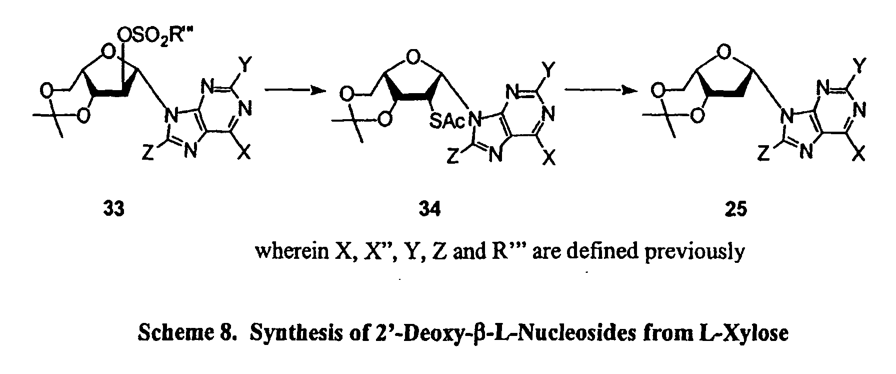

### II-2-3.2'-Carbonyl intermediates:

### 1. Purine Nucleosides

A purine nucleoside **23** is subjected to mild oxidation, such as Swern oxidation or Moffatt oxidation using DMSO and oxalyl chloride or DMSO and DCC, or with pyridinium dichromate in methylene chloride (Froehlich, M. L.; Swarting, D. J.; Lind, R. E.; Mott, A. W.; Bergstrom, D. E.; Maag, H.; Coll, R. L. *Nucleoside Nucleotide* **1989**, *8*, 1529) to give the 2'-koto derivative **35** (**Scheme 9**). The Huang Minlon modification of Wolff-Kischner reaction with hydrazine hydrate and KOH in diethyleneglycol affords the desired 2'-deoxy-β-L-nucleoside.

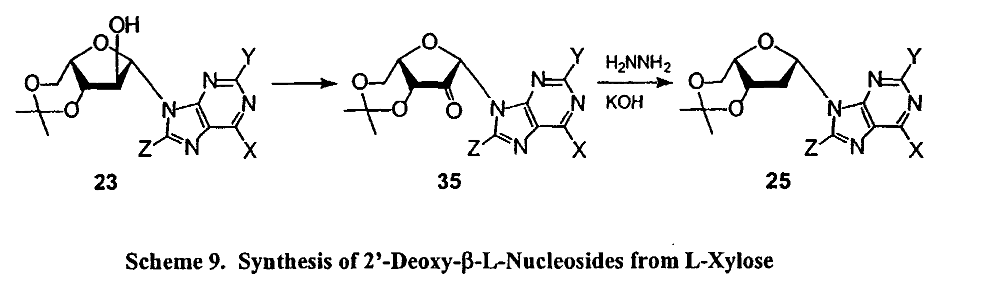

### 2. Pyrimidine Nucleosides

The above method cannot be applied to pyrimidine nucleosides, since hydrazine would destroy the pyrimidine ring. However, treatment of a 2-carbonyl nucleoside **(35, Scheme 10,** B = pyrimidine) with tosylhydrazine would give the hydrazone **36.** The tosylhydrazone **35** can be subjected to Kabalka's deoxygenation (Kabalka, G. W.; Baker, J. D. *J. Org. Chem*. **1975**, *40*, 1834) using catecholborane and sodium acetate in chloroform or methylene chloride, or reduced under Caglioti's condition with NaBH₄ (Caglioti, L. *Org. Synth.* **1972,** *52,* 122) or NaBH₃CN (Hutchins, R. O.; Milewski, C. A.; Maryanoff, B. E. *J. Am. Chem. Soc.* **1973,** *95,* 3662). This method can also be applied to purine nucleosides corresponding to **35.**

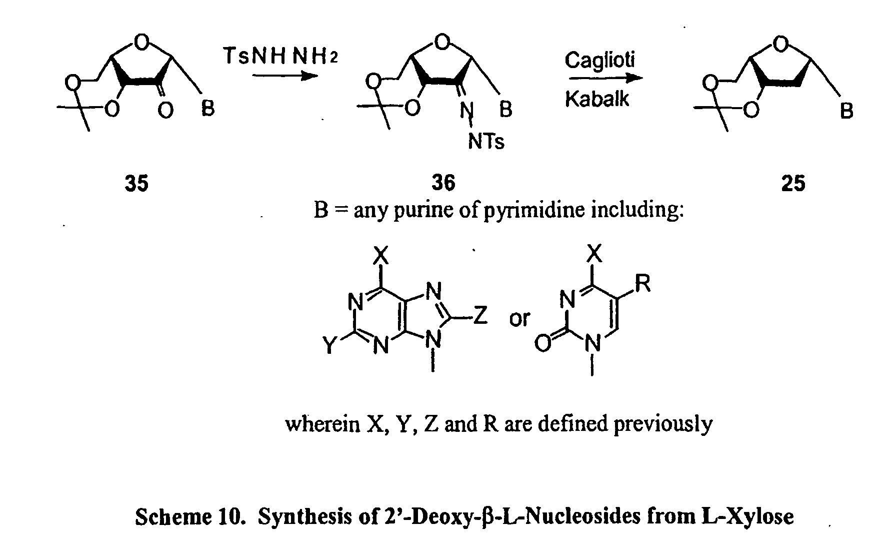

### III. From L-Arabinose

In this invention, attention is focused on epimerization at C-2 to a ribose derivative intermediate with a readily reducible functional group which can control the anomeric configuration when the intermediate is condensed with a base thus exclusively forming the desired β-nucleosides. Two such functional groups are used: one is acylthio group and the other thioacyl. The key intermediate has the general alkyl L-arabinofuranoside structure **VI** below, in which 3 and 5 positions are protected with *non-participating* group, such as benzyl, *p*-methylbenzyl, *p*-methoxybenzyl, *t*-butyldimethylsilyl or *t*-butyldiphenylsilyl, and the C-2 hydroxy group is substituted by a sulfonyloxy group, such as mesyloxy, tosyloxy, triflyloxy and a like. The aglycon is methyl, ethyl or benzyl. 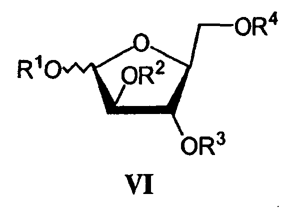 R¹ = CH₃, C₂H₅ or CH₂Ph
R² = mesyl, tosyl, triflyl, and the like.
R³ and R⁴ = same or different and are benzyl, nitrobenzyl, *p*-methylbenzyl or *p*-methoxybenzyl, *t*-butyldimethylsilyl, *t*-butyldiphenylsilyl

### III-1. Acylthio Intermediate

An advantage of this approach is the synthesis 1-O-acetyl-2-acetylthio-3,5-di-O-benzyl-2-deoxy-L-ribofuranose (**43**, R = CH₃, R² = Benzyl, R = R''' = Ac, **Scheme 11**) which serves as a very versatile intermediate. The starting material, 1,2-O-isopropylidene-α-L-arabino-furanose (**39**, R' = R" = CH₃) is known, but the synthesis is rather laborious and requires mercury amalgam during the synthesis. The following easier method has been developed. L-Arabinose is silylated with one equivalent of silylating agent, such as *t*-butyldimethylsilyl halide or *t*-butyldiphenylsilyl halide or a like to obtain 5-silyated-L-arabinose (**37**), which is treated with acetone in the presence of mineral acid with or without dehydrating agent such as anhydrous copper sulfate, or with a mixture of acetone and 2,2-dimethoxypropane in the presence of mineral acid, such as hydrochloric acid, sulfuric acid or phosphoric acid and the like, or Lewis acid such as zinc chloride, to afford 5-O-protected-1,2-O-isopropylidene-β-L-arabinofuranose (**38**). Treatment of **38** with fluoride ion removes the silyl protecting group producing 1,2-O-isopropylidene-β-L-arabinofuranose **(39).** Compound **39** is benzylated with benzyl chloride and sodium hydride in an inert solvent such as tetrahydrofuran to give 3,5-di-O-benzyl-1,2-O-isopropylidene-α-L-arabinofuranose (**40**, R² = Benzyl). Methanolysis of **40** affords methyl 3,5-di-O-*benzyl*-L-arabinofuranoside **41** (R = CH₃). Sulfonylation of **41** with a suitable sulfonylating agent gives 2-O-sulfonate (**42**, wherein R³ is mesyl, tosyl, or triflyl, preferably triflyl) which, upon treatment with alkali metal thioacylate, such as potassium thioacetate and sodium thiobenzoate, preferably potassium thioacetate in a solvent such as *N,N*-dimethylformamide, N-methylpyrrazolidinone, hexamethylphosphoramide, and the like, preferably N-methylpyrrolidinone, gives the methyl 2-deoxy-2-thioacetyl-L-riboside **43** (X=SAc; R'''=Ac). Acetolysis of **43** affords the L-ribofuranose **44** (X=SAc). Condensation of **44** with a silylated pyrimidine nucleobase in the presence of Friedel-Crafts catalyst yields exclusively the corresponding desired β-L-nucleoside (**45**, X=SAc). Alternatively, **44** can be converted into the 1-chloro-sugar which is then treated with a purine Na salt to give the corresponding purine β-L-nucleoside **45** exclusively. The presence of 2'-S-acyl group is essential for the stereo-specific synthesis. Desulfurization of **45** with Raney Ni treatment gives **9** (R² = Benzyl). Hydrogenolysis of the benzyl groups of **9** furnishes the desired 2'-deoxy-β-L-nucleoside (**10**). The protecting groups at the 3 and 5 positions in **42** must be non-participating. Reist *et at*. (Reist, E. J.; Hart, P. A.; Goodman, L.; Baker, B. R. *J. Am. Chem.* *Soc.* 1959, 81, 5176-5180) synthesized methyl 3,5-di-O-*benzoyl*-L-arabinofuranoside which, however, should give the 2-S-acetyl-*arabino* via neighboring group participation or 3-S-acetyl-*xylo* derivative *via* 2,3-ribo-epoxide. Although 3-S-acetyl-xylofuranoside may be useful in the synthesis of 2'-deoxynucleosides the procedure is not straightforward for preparative synthesis (Anderson, C. D.; Goodman, L.; Baker, B. R. *J. Am. Chem. Soc.* **1959,** *81*, 3967-3973).

Alternatively, 39 is p-methylbenzoylated with p-methylbenzoyl halide in base such as pyridine to give **40** (R² = p-CH₃Bz), which is methanolyzed to methyl 3,5-di-O-*p*-methylbenzoyl-L-arabinofuranoside 41 (R² = p-CH₃Bz, R = CH₃). Conversion of **41** into thiocarbonyl derivatives **(42,** R³=phenoxythiocarbonyl, imidazothiocarbonyl, *N*-phenylthiocarbamoyl or alkylxanthyl), followed by radical deoxygenation with tri-*n*-butyltin hydride in the presence of AIBN in refluxing toluene affords methyl 2-deoxy-3,5-di-O-*p*-methylbenzoyl-L-ribofuranoside **(44,** X = H, R² = p-MeBz). Treatment of the latter with hydrogen chloride in acetic acid gives the crystalline 2-deoxy-3,5-di-O-p-methylbenzoyl-α-L-ribofuranosyl chloride in very high yield. This chloro-sugar also can be synthesized from L-arabinose, which is converted methyl or benzyl arabinopyranoside (**38a**, β-anomer is the major product) by treatment with methyl or benzyl alcohol and hydrogen chloride. 3,4-O-Isopropylidene derivative **(39a)** is obtained by treatment of the arabinoside in acetone with 2,2-dimethoxypropane in the presence of a catalytic amount of acid, such as p-toluenesulfonic, methanesulfonic, ethanesulfonic, or sulfuric acid and the like. Conversion of **39a** into a thiocarbonyl derivative **40a,** followed by radical deoxygenation to 2-deoxy-3,4-O-isopropylidene-L-ribopyranoside **41a** can be achieved by treatment of **40a** with tri-n-butyltin hydride in the presence of AIBN in refluxing toluene. Acid hydrolysis of **41a** affords free 2-deoxy-L-ribose **42a,** which, upon short treatment of methanolic hydrogen chloride gives methyl L-ribofuranoside **(43,** X = H, R₂ = H). *p*-Methoxybenzoylation of **43** (X = H, R² = H) to **44** (X = H, R² = p-MeBz) and subsequent methanolic hydrogen chloride gives the same chloro sugar.

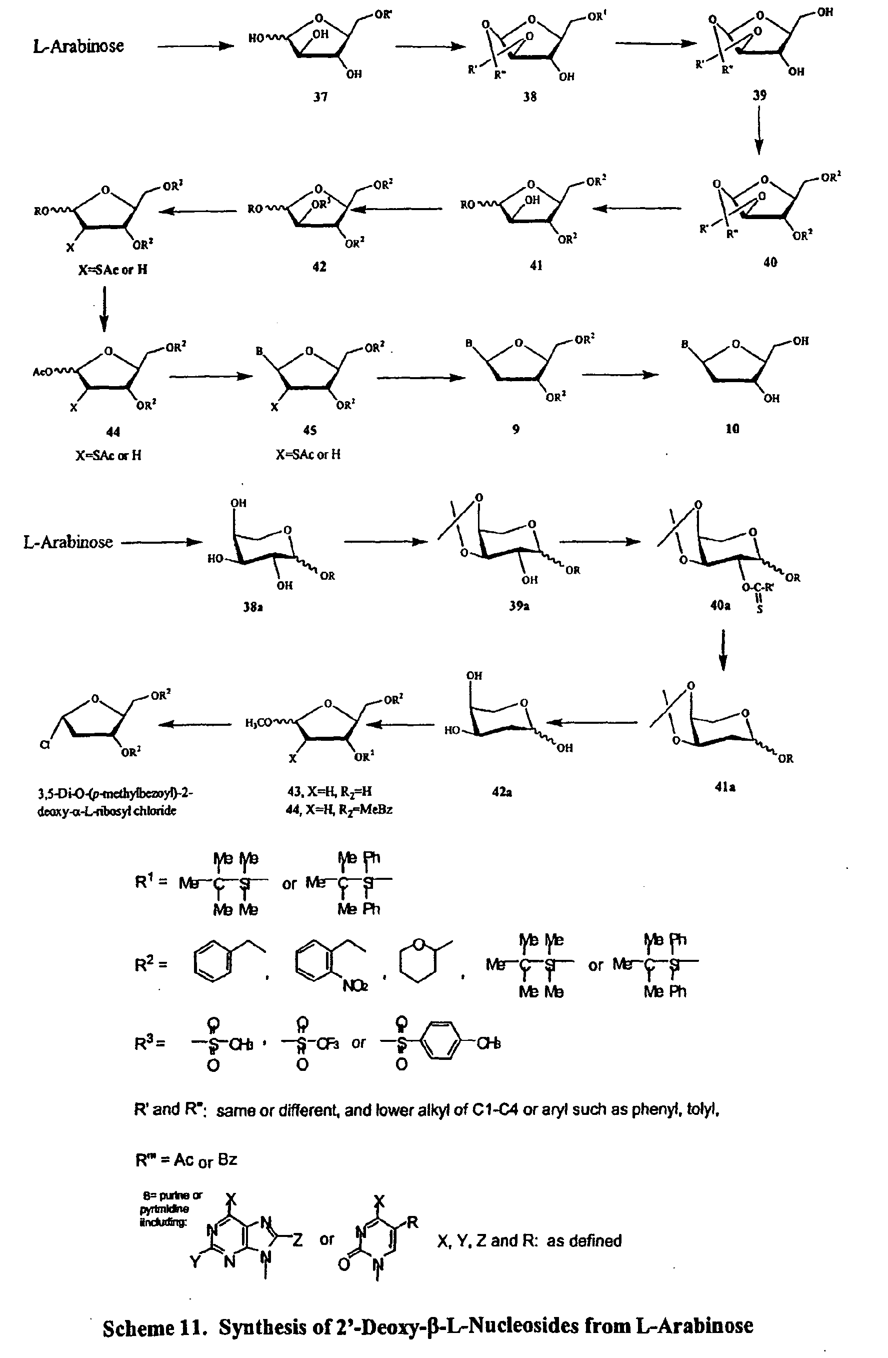

### IV. From D-Arabinose

The advantage of this procedure is that it is rather easy to prepare an α-D nucleoside in a stereospecific manner. Epimerization at C-4' will convert the α-D arabino-nucleoside into the β-L-xylo-nucleoside. Further epimerization at the C-3' position affords the targeted 2'-deoxy-β-L-ribo-nucleoside. Such chemistry has never been reported. The key intermediate has a general 1,2-di-O-acyl-D-arabino structure **VII** below. Condensation of purine or pyrimidine base with a molecule of structure **VII** results in exclusive formation of α-D-nucleoside of general structure VIII, from which the 2'-O-acyl can be selectively removed to give 2'-free hydroxy intermediate **IX.** The synthetic methods of β-L-nucleoside from **IX** can be classified into three routes dependent upon the way this 2'-OH is deoxygenated.

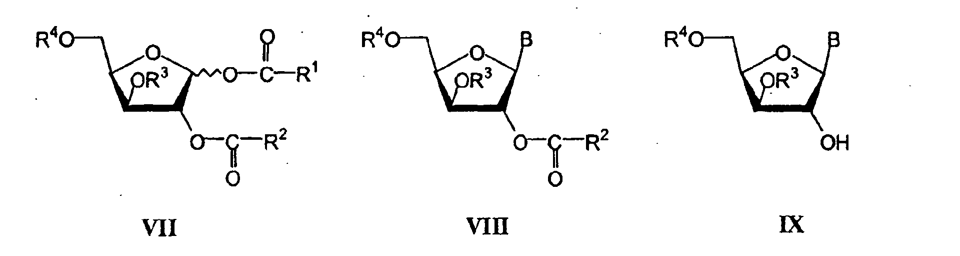

R¹ and R² are the same or different and are lower alkyl of C₁-C₃ or unsubstituted or substituted phenyl;

R³ and R⁴ are the same or different and are benzyl, p-methylbenzyl, p-methoxybenzyl, o-nitrobenzyl, t-butyldimethylsilyl or t-butyldiphenylsilyl.

### IV-1. 2'-O-Thiocarbonyl Intermediate

The most readily available starting material is 1,2-O-isopropylidene-β-D-arabinofuranose (46, R' = R" = CH₃, **Scheme 12).** Benzylation of **46** with benzyl chloride or benzyl bromide in pyridine at a temperature of from 0 °C to 115 °C, preferably from 20 °C to 60 °C, for a period of from 1 hour to 72 hours, or treatment of 46 with alkali metal hydride, such as NaH, KH or LiH, preferably NaH, in a solvent such as low alkanol of C₁-C₄ followed by benzyl halide at a temperature of from -20°C to 100°C, preferably from 0 °C to 35 °C for a period of from 30 minutes to 24 hours, preferably from 1 to 3 hours gives the 3,5-di-O-benzyl derivative **47** (R₃ = R₄ = CH₂Ph). Acetolysis of **47** with acetic acid, acetic anhydride and sulfuric acid at a temperature from -20 °C to 40 °C for 30 minutes to 4 hours, preferably 1-2 hours, gives 1,2-di-O-acetyl-D-arabinose **48** (R³ = R⁴ = CH₂Ph, R¹ = R² = Ac). Alternatively, hydrolysis of 47 with aqueous alcoholic mineral acid, such as hydrochloric acid, sulfuric acid, followed by acylation of the product will give **48** with other 1,2-di-O-acylated, such as benzoyl, *p*-nitrobenzoyl, toluoyl, anisyloyl, propanoyl, derivatives. Condensation of 48 with a silylated pyrimidine in the presence of a Lewis acid in an inert solvent, such as methylene chloride, ethylenedichloride or acetonitrile, affords the α-D-nucleoside **49** (B = pyrimidine, R³ = R⁴ = CH₂Ph, R¹ = R² = Ac) exclusively. The corresponding α-D-purine nucleoside **49** (B = purine, R³ = R⁴ = CH₂Ph, R¹ = R² = Ac) can also be prepared by conversion of **46** into the corresponding halo-sugar by treatment with HCl/diethyl ether or HBr/CH₂Cl₂, followed by condensation with sodio-purine in a solvent, such as acetonitrile, *N,N*-dimethylformamide, 1,2-dimethoxyethane, diglyme, or a like, preferably acetonitrile, at a temperature of from 0°C to 76°C, preferably from 15°C to 35°C, for a period of from 30 minutes to 72 hours, preferably from 1 to 4 hours. Saponification of **52** yields the 2'-free hydroxy derivative **50.** Conversion of 50 to the 2'-O-thiocarbonyl derivative **54** is achieved by treatment with thiocarbonyldiimidazole in *N*,*N*-dimethylformamide or phenoxythiocarbonyl chloride in pyridine. Barton reduction of **51** with tri-n-butyltin hydride in toluene in the presence of 2,2'-azobis(methylpropionitrile) affords the 2'-deoxy-*α*-D-*threo*(xylo)-nucleoside **52.** After de-O-benzylation of **55** by hydrogenolysis over palladium catalyst, the product **53** is subjected to Moffatt or Swern oxidation using dimethylsulfoxide and limited amounts of dicyclohexylcarbodiimide or oxalyl chloride affords the aldehyde 54. This aldehyde can be converted into the enolacetate **55** or similar enamine. Hydrogenation of **55** occurs from the least sterically hindered side (from the top) to furnish the 2'-deoxy-*threo*pentofuranosyl-β-L-nucleoside **56**. After de-*O*-acetylation, the product **26** is sulfonylated to the di-*O*-sulfonylate **57**. Treatment of **57** with alkali metal acylate such as sodium acetate or sodium benzoate, preferably sodium benzoate, in a solvent such as *N,N*-dimethylformamide, dimethylsulfoxide, hexamethylphosphoric triamide, preferably *N,N*-dimethylformamide, at a temperature of from 10 °C to 200 °C, preferably from 35 °C to 115 °C, for a period of from 30 minutes to 3 days, preferably from 1 to 3 hours, gives the desired 2'-deoxy-βL-*erythro*-ribo-nucleoside which, upon saponification, is converted into 2'-deoxy-βL-nucleoside **10**.

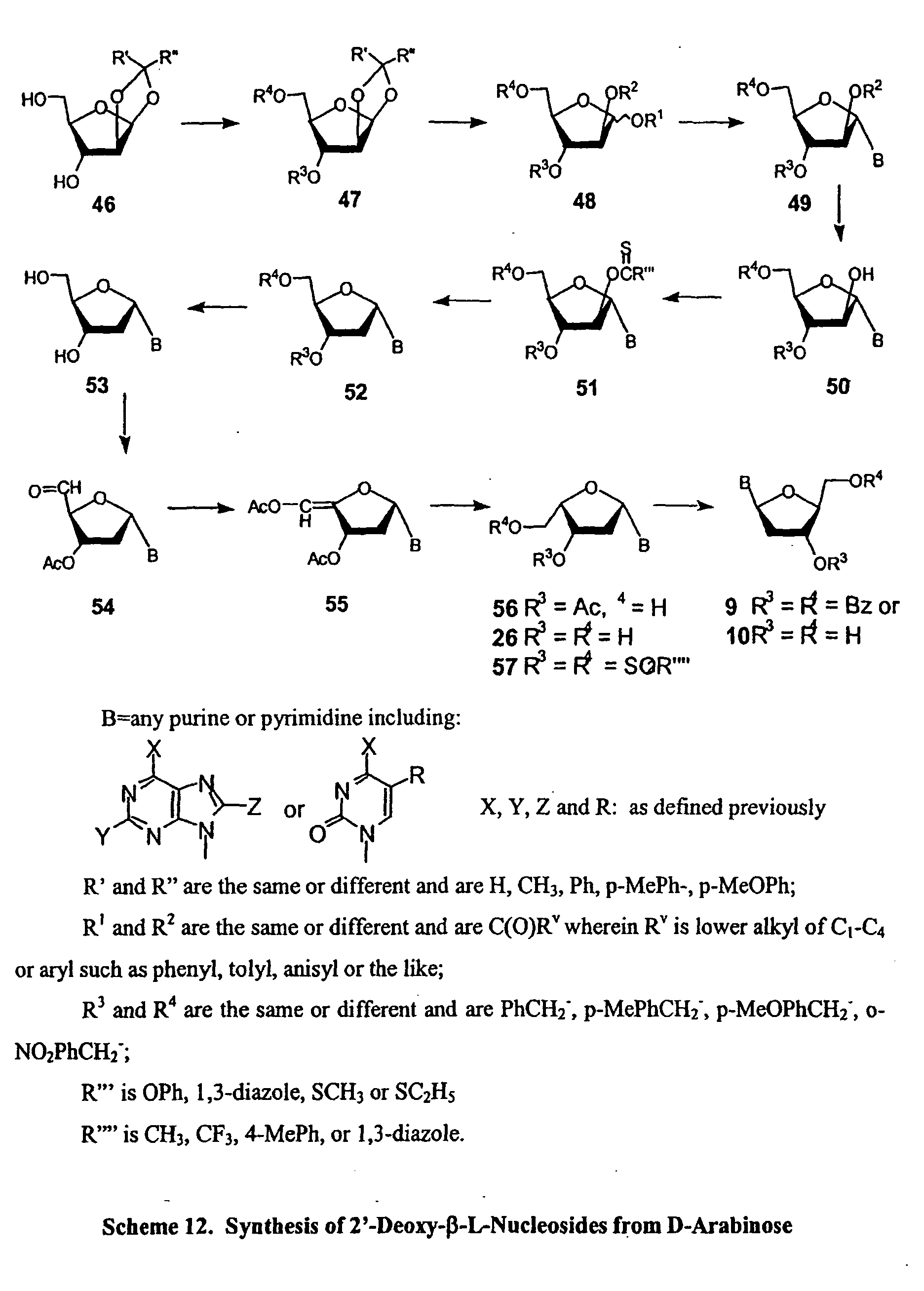

### IV-2. 2'-Deoxy-2'-Acylthio Intermediate

Compound **50** described above is sulfonylated to give **58 (Scheme 13)** wherein R''' is methyl, ethyl, *p*-toluyl, trifluoromethyl or imidazolyl. Treatment of **58** with alkali metal thioacylate, such as potassium thioacetate or sodium thiobenzoate, preferably potassium thioacetate, gives 2'-deoxy-2'-acylthio derivative **59**. In the case of pyrimidine nucleoside 59 (B = pyrimidine), the product is the *arabino*-nucleoside, namely, the 2'-substituent retains the same "down" configuration as the reaction proceeds *via* the 2,2'-anhydro-α-D-*ribo*-nucleoside intermediate. In purine nucleoside (**59**, B = purine), direct nucleophilic displacement of the 2'-sulfonyloxy group should occur forming the ribo-nucleoside. Raney nickel desulfurization affords the 2'-deoxy-α-D-*threo*-xylo-nucleoside **52.** Conversion of **52** to the targeted 2'-deoxy-β-L-nucleoside **10** is already described.

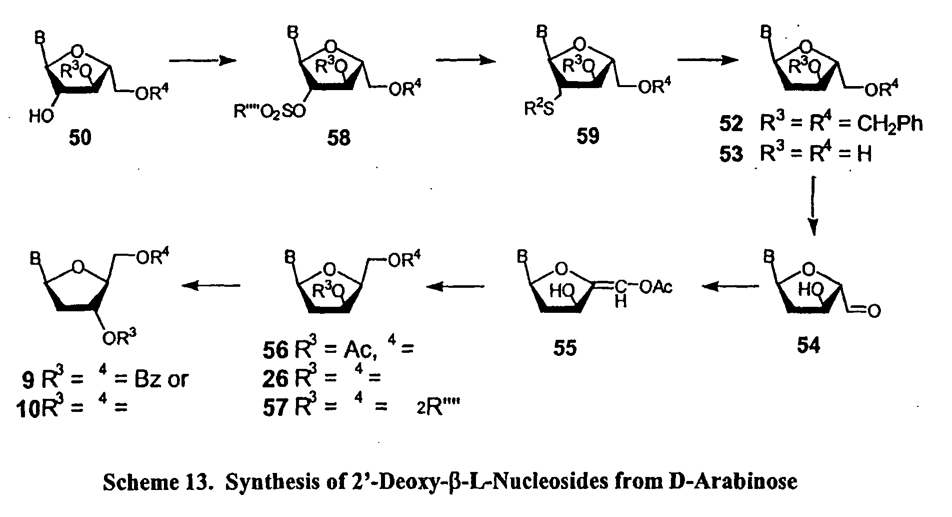

### IV-3. 2'-Deoxy-2'-halogeno Intermediate

Treatment of **58** with trialkyl ammonium hydrogen chloride or bromide, or tetrabutyl ammonium bromide, or alkali metal iodide such as potassium iodide or sodium iodide in acetone, 2-butanone, acetonitrile, *N,N*-dimethylformamide, 1,2-dimethoxyethane affords the 2'-halogeno arabino derivative **60 (Scheme 14).** Catalytic hydrogenolysis of **60** over a palladium catalyst will give the 2'-deoxy-α-L-*erythro*(ribo)-nucleoside **52**. Conversion of **52** to the targeted 2'-deoxy-β-L-nucleoside 10 is already described.

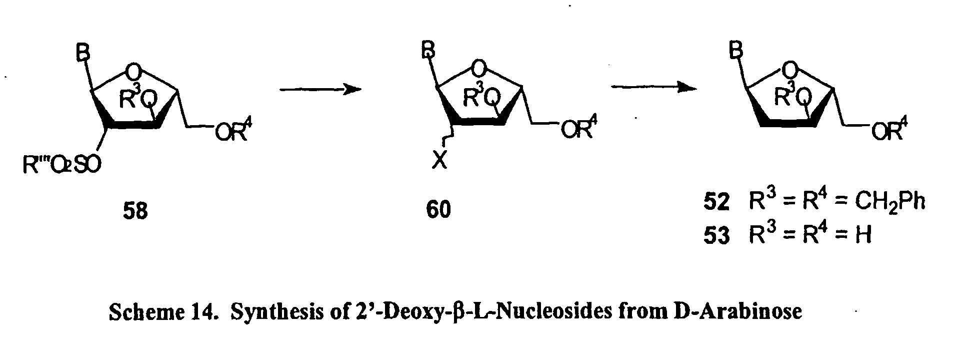

It should also be noted that a 2'-deoxy-α-D-nucleoside is always formed as a by product during the chemical synthesis of 2'-deoxy-β-D-nucleoside by condensation with 2'-deoxy sugar with a base. This α-D-nucleoside by-product can be converted into the corresponding β-L-nucleoside by the procedures described above.

### V. Synthesis of β-L-Nucleosides from β-D-Nucleosides

This invention discloses methods of synthesis of a group of 2'-deoxy-β-L-nucleosides, which are found to be very active against HBV starting from naturally-occurring 2'-deoxy-β-D-nucleosides.

In 1984, Yamaguchi and Saneyoshi (Yamaguchi, T.; Saneyoshi, M. *Chem*. *Pharm*. *Bull*. 1984, *32*, 1441.) reported a method of anomerizing 2'-deoxy-β-D-nucleosides into 2'-deoxy-α-D-nucleosides. This method has not been further utilized since. In 1965, Pfitzner and Moffatt prepared β-nucleoside-5'-aldehydes by oxidation of naturally-occurring β-nucleosides with dimethylsulfoxide (DMSO) and dicyclohexylcarbodiimide (DCC) (Pfitzner, K. E.; Moffatt, J. G. *J. Am. Chem. Soc*. **1965,** *87*, 5661). Cook and Secrist then, convert a Moffatt's aldehyde into the corresponding enols (Cook, S. L.; Secrist, J. A. *J. Am*. *Chem*. *Soc*. **1979,** *101*, 1554). By combination of these reactions, followed by stereoselective reduction of the enol, it is possible to for the first time to convert the naturally-occurring 2'-deoxy-β-D-nucleosides into their corresponding, biologically-active 2'-deoxy-β-L-nucleosides. Thus, 2'-deoxynucleosides having the natural β-D-glycosyl configuration with a general structure X is transformed into their mirror images XI by inverting every chiral center in the molecule. 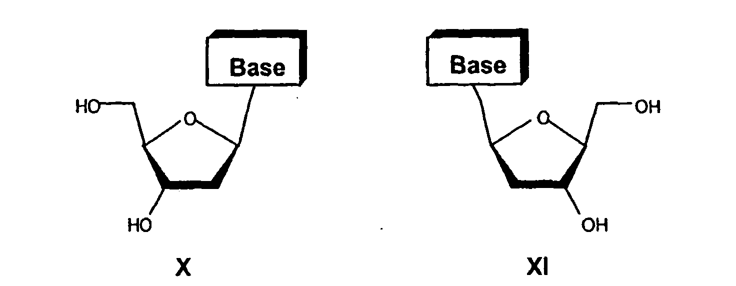 **Base= any pyrimdine or purine including:** 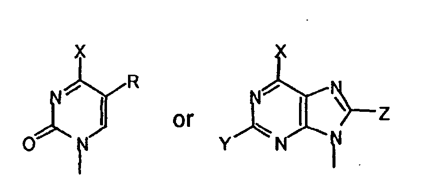 R, X, Y and Z = as defined previously

### V-1. From 2'-deoxy-β-L-nucleoside

Peracylated pyrimidine β-D-nucleoside (**61, Scheme 15**) is prepared. Compound **61** is then treated with trimethylsilyltriflate and bis(trimethylsilyl)acetamide in a solvent such as acetonitrile, ethyl acetate, *N,N*-dimethylformamide, hexamethylphosphoric triamide, 1,2-dimethoxyethane, diglyme, chloroform, methylene chloride, preferably acetonitrile, at a temperature of from 0 °C to 125 °C, preferably from 25 °C to 100 °C, for a period of from 30 minutes to 24 hours, preferably from 2 hours to 6 hours, to obtain 3',5'-di-O-acyl-2'-deoxy-α-D-pyrimidine nucleoside **52**. Mild saponification of **52** with base such as NH₃/MeOH or NaOMe/MeOH gives the free nucleoside **53**. Reaction of **53** with one equivalent of dicyclohexylcarbodiimide in dimethylsulfoxide in the presence of phosphoric acid or dichloroacetic acid or oxalyl chloride in dimethylsulfoxide gives the 5'-aldehyde **54**. Acetylation of **54** with acetic anhydride in the presence of potassium carbonate affords the acetyl enolate **55.** Alternatively, silylation of **54** with trialkylsilyl halide in dry pyridine in the presence or absence of a super base, such as *p*-*N,N*-dimethylaminopyridine, or heating with hexamethylsilazane in the presence of a catalytic amount of ammonium sulfate, will give silylated enolate **(55, 62 - 66).** Treatment of the enolate **55** (or **62 - 66)** with hydrogen over palladium-charcoal catalyst furnishes hydrogenation of the 4',5'-double bond from the least hindered β-face converting the α-D-nucleoside into the 2'-deoxy-β-L-nucleoside with the *threo* configuration **56.** Saponification of **56,** followed by di-O-sulfonylation with a reagent such as mesyl chloride, tosyl chloride, triflyl chloride in pyridine gives 3',5'-di-O-sulfonate **57** which, upon treatment with alkali metal acylate, such as potassium or sodium benzoate or acetate in *N,N*-dimethylformamide, provides 3',5'-di-O-acyl-2'-deoxy-β-L-nucleoside **9.** Saponification of **9** gives the desired β-L-nucleoside **10.**

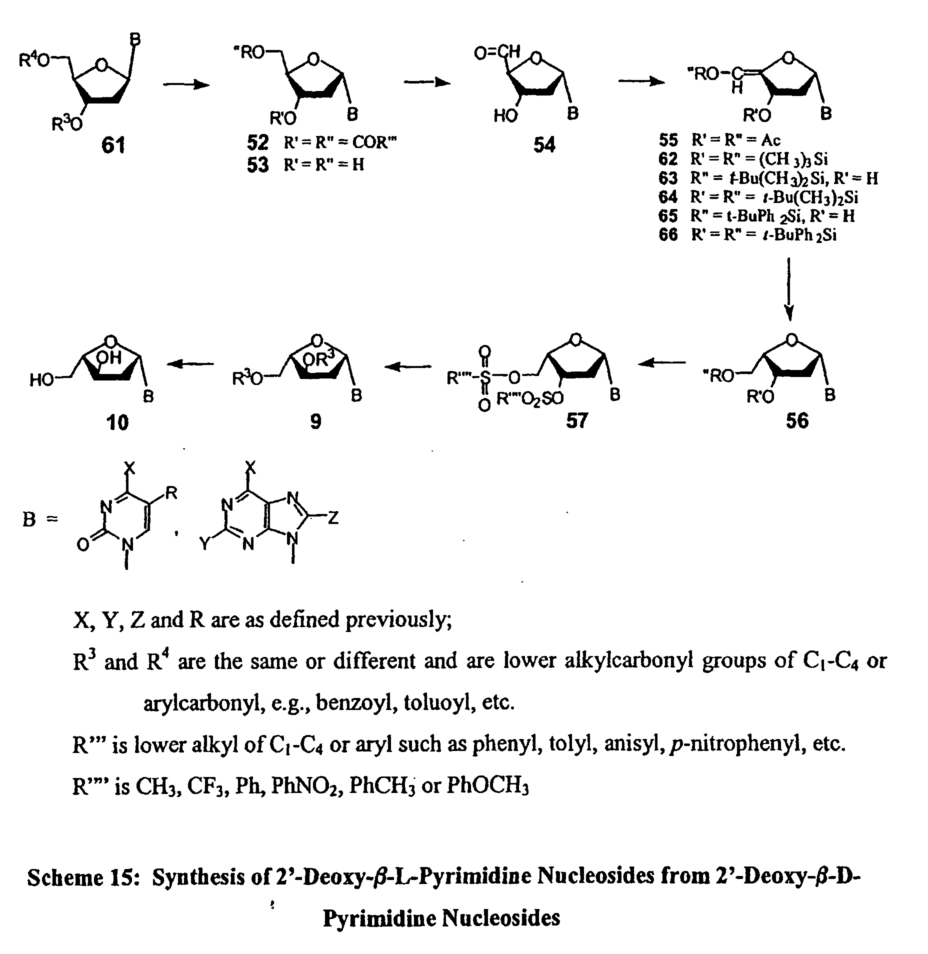

2'-Deoxy-β-L-nucleoside, such as 2'-deoxy-L-adenosine **(10,** X = NH₂, Y = H) and 2'-deoxy-L-guanosine **(10,** X = OH, Y = NH₂, Z = H) can also be obtained from 2'-deoxy-pyrimidine-β-L-nucleoside by transglycosylation. Thus, treatment of **61 (Scheme 16,** X=NHAc, R=H) with *N*⁶-benzoyl-*N*⁶,*N*⁹-bis(trimethylsilyl)adenine or *N*²,*N*²,*N*⁹-tris(trimethylsilyl)guanine with bis(trimethylsilyl)acetamide and trimethylsilyl triflate in acetonitrile affords protected 2'-deoxy-L-adenosine (**10**, X=NHBz, Y=H, Z=H) and 2'-deoxy-L-guanosine (**10**, X=OH, Y=NH₂, Z=H).

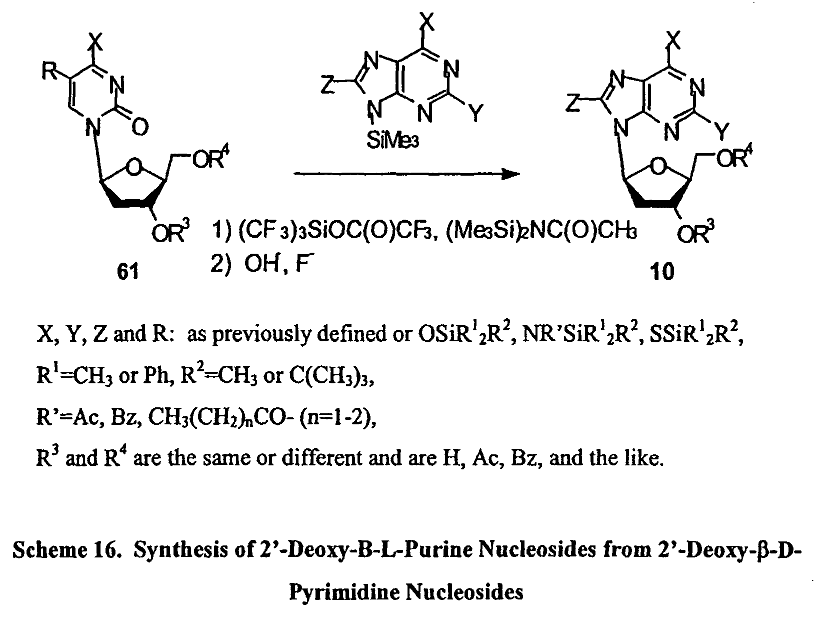

This invention is further illustrated in the Experimental Details section which follows. The Experimental Details section and Examples contained therein are set forth to aid in an understanding of the invention. This section is not intended to, and should not be interpreted to, limit in any way the invention set forth in the claims which follow thereafter.

### Examples

### EXAMPLE 1

### 1-O-Acetyl-2,3,5-tri-O-benzoyl-β-L-ribofuranose (1, R¹ = Ac, R² = Bz)

This compound is prepared from L-ribose according to Recondo and Rinderknecht (*loc. cit*.) who prepared 1-O-acetyl-2,3,5-tri-O-benzoyl-β-D-ribofuranose (1, R¹ = Ac, R² = Bz) from D-ribose. A mixture of L-ribose (150 g, 1.0 mol) in methanol (2.5 L) containing 1% hydrogen chloride is stirred for 2 hours, and then neutralized with pyridine (250 mL). The mixture is concentrated *in vacuo,* and the residue dissolved in pyridine (1 L). To the solution is added benzoyl chloride (385 mL, 3.3 mol) dropwise while chilling to 0 °C. After being kept overnight at room temperature, the mixture is concentrated *in vacuo* at 35 - 40 °C, and the residue is dissolved in ethyl acetate (1.5 L). The organic solution is washed successively with cold water (2 x 0.5 L), IN H₂SO₄ (3 x 0.5 mL), water (0.5 L), saturated sodium bicarbonate (2 x 0.5 mL), dried over magnesium sulfate, concentrated *in vacuo* to a syrup which is dissolved in a mixture of glacial acetic acid (200 mL) and acetic anhydride (0.5 L). To the solution is added concentrated sulfuric acid dropwise at 0 °C. The product solidified is filtered, washed successively with cold water (2 x 0.5 L), saturated sodium bicarbonate (2 x 0.5 L), cold water (2 x 0.5 L), and recrystallized from methanol to give compound *I* (225 g, 45%), mp 124-125 °C. The ¹H-NMR spectrum of this sample is identical to that of the D-isomer.

### EXAMPLE 2

### 2,3,5-Tri-O-benzoyl-D-ribofuranosyl bromide (2, X' = Br, R² = Bz)

Hydrogen bromide is bubbled into an ice-cold solution of 1 (25.2 g, 0.05 mol) in methylene chloride (150 mL) for 15 minutes. After being kept at 0 °C for 1 hour and at room temperature for 15 minutes, the solution is concentrated *in vacuo*. Traces of hydrogen bromide are removed by successive azeotropic distillation with toluene (25 mL x 5). The syrupy residue (**2**) is used immediately for condensation with appropriate purine or pyrimidine. The ¹H-NMR spectrum of this syrup includes a singlet at δ 6.5 (H-1 for β-anomer) and a doublet at 6.9 (H-1 for α-anomer, J_{1,2} = 4.4 Hz). The α/β ratio is approximately 3:2.

### EXAMPLE 3

### 1-(2,3,5-tri-O-benzoyl-β-L-ribofuranosyl)-N⁴-anisoylcytosine (3, B=N⁴-anisoyl-cytosine, R² = Bz) - condensation without catalyst

A mixture of N⁴-anisoylcytosine (12.5 g, 0.05 mol), ammonium sulfate (~10 mg) in hexamethyldisilazane (50 mL) is stirred and heated to reflux. When the reaction mixture becomes clear, the excess hexamethyldisilazane is removed *in vacuo.* To the residue is added a solution of compound 2 (X' = Br, R² = Bz) prepared as above from 25.2 g of **1**, and dissolved in 70 mL of dry acetonitrile). The mixture is stirred for 24 hours at room temperature, then condensed *in vacuo*. The residue is taken up in methylene chloride (300 mL), and the solution washed with saturated sodium bicarbonate (300 mL) and water (300 mL), dried over sodium sulfate. After evaporation of the solvent, compound 3 (B = N⁴-anisoylcytosine, R² = Bz) is crystallized from ethanol; 24.8 g (72 %), mp 229-230 °C. The ¹H-NMR spectrum is identical to that of the D-isomer. (Matsuda, A.; Watanabe, K. A.; Fox, J. J. Synthesis 1981, 748.)

### EXAMPLE 4

### 9-(2,3,5-tri-O-acetyl-β-L-ribofuranosyl)-2,6-dichloropurine (3, B=2,6-dichloropurine, R² = Ac) - sodium procedure)

To a solution of 2,6-dichloropurine (1.9 g, 0.01 mol) in dry *N*,*N*-dimethylformamide (25 mL) is added sodium hydride (60 % in mineral oil, 0.4 g, 0.01 mol). After the evolution of hydrogen has ceased, a solution of **2** [(X'=Br, R² = Ac) prepared as above from 3.2 g of 1 (R¹ = R² = Ac, 0.01 mol)] in *N*,*N*-dimethylformamide (10 mL) is added dropwise. The mixture, after being stirred overnight at room temperature, is added a few drops of acetic acid then diluted with cold water (100 mL), and extracted with methylene chloride (100 mL x 3). The combined organic layers are washed with water (100 mL x 2), dried over sodium sulfate, and evaporated *in vacuo*. The residue is crystallized from ethanol, 4.7 g (75 %), mp 158-159°C. The ¹H-NMR spectrum is identical to that of the D-isomer prepared by the fusion method. (Ishido, Y.; Kikuchi, Y.; Sato, T. *Nippon Kagaku Zasshi* **1965**, *86*, 240.)

### EXAMPLE 5

### 1-(2,3,5-tri-O-benzoyl-β-L-ribofuranosyl)thymine (3, B = thymine, R² = Bz) - direct condensation of 1 in the presence of catalyst)

A mixture of thymine (12.6 g, 0.1 mol) and ammonium sulfate (~10 mg) in hexamethyldisilazane (120 mL) is stirred and heated to reflux. When the mixture becomes clear, excess hexamethyldisilazane is removed *in vacuo*. The residue is dissolved in 1,2-dichloroethane (250 mL) and added to a solution of 1 (R¹ = Ac, R² = Bz) (50 g, 0.1 mol) in 1,2-dichloroethane (150 mL). To the stirred solution is added tin tetrachloride (25 mL), and the mixture is stirred overnight at room temperature, then poured into saturated sodium bicarbonate solution (500 mL). When the frothing ceased, the suspension is filtered through a Celite pad which is then thoroughly washed with methylene chloride (500 mL). The combined organic layers are washed with water (500 mL x 2), dried over sodium sulfate, concentrated *in vacuo*, and the residue crystallized from ethyl acetate to give 3 (B = thymine, R² = Bz), 48.5 g (85 %), mp 167 - 168 °C. The ¹H-NMR spectrum of this sample is identical to that of the D-counterpart. (Watanabe, K. A.; Fox, J. J. *J. Heterocycl. Chem.* **1969,** 6,109.)

### EXAMPLE 6

### 1-(β-L-Ribofuranosyl)thymine (4, B = thymine)

Compound **3** (B = thymine, R² = Bz) prepared above (28 g, 0.05 mol) in 560 mL of ethanolic ammonia (saturated at 0 °C) is kept standing overnight at room temperature. The solution is concentrated *in vacuo*, and the residue is triturated with diethyl ether (200 mL x 2) to remove ethyl benzoate and benzamide. The insoluble residue is crystallized from ethanol to give 13.0 g (95 %) of 4 (B = thymine), mp 183 - 185 °C. The ¹H-NMR spectrum of this sample is identical to that of the D-counterpart. (Fox, J. J.; Yung, N.; Davoll, J.; Brown, G. B. *J. Am. Chem. Soc.* **1956**, 78, 2117.)

### EXAMPLE 7

### 9-(2-O-Triflyl-β-L-ribofuranosyl)hypoxanthine (5, B = hypoxanthine, R² = CF₃SO₂-)

A mixture of 4 (B = hypoxanthine) (2.68 g, 0.01 mol) and dibutyltin oxide (2.5 g, 0.01 mol) in methanol (500 mL) is heated under reflux until a clear solution is obtained, and then concentrated *in vacuo.* The residue is dissolved in *N,N*-dimethylformamide (150 mL), and treated with trifluoromethanesulfonyl chloride (1.85 g, 0.011 mol) at room temperature for 1 hour. The mixture is concentrated *in vacuo*, and the residue chromatographed on silica gel using chloroform-ethanol (9 : **1** v/v) as the eluent to obtain 5 (B = hypoxanthine, R² = CF₃SO₂), 1.5 g (37%) as a foam. ¹H-NMR δ 3.64-3.74 (2H, m, H-5',5"), 3.99-4.08 (1H, m, H-4'), 4.59 (1H, dd, H-3', J_{2',3'} = 4.4, J_{3',4'} = 5.5 Hz), 5.77 (1H, dd, H-2', J_{1',2'} = 4.1, J_{2',3'} = 4.4 Hz), 6.38 (1H, d, H-1', J_{1',2'} = 4.1 Hz), 8.12 (1H, d, H-2), 8.37 (1H, s, H-8).

### EXAMPLE 8

### 9-(3,5-O-[1,1,3,3-Tetraisopropyldisiloxan-1,3-yl]-β-L-ribofuranosyl)hypoxanthine (7, R³, R³ = -iPr₂Si-O-iPr₂Si-)

A mixture of 1-benzyl-9-(β-L-ribofuranosyl)hypoxanthine **4** (B = 1-benzylhypoxanthine) (7.16 g, 0.02 mol) and 1,3-dichloro-1,1,3,3-tetraisopropyldisiloxane (7.2 g, 0.023 mol) in pyridine (100 mL) is stirred at room temperature overnight. The pyridine is removed *in vacuo*, and the residue partitioned between chloroform (300 mL) and water (50 mL). The organic layer is washed with water (50 mL x 2), dried over sodium sulfate, and concentrated *in vacuo*. The residue is chromatographed on silica gel using chloroform-ethanol (40:1 v/v) as the eluent to obtain 7 [B = 1-benzylhypoxanthine, R²,R³ = -Si(*i*Pr)₂-O-(*i*Pr)₂Si-] as a foam, 12,0 g (81 %). ¹H-NMR δ 0.94-1.02 (28H, m, *i*Pr), 3.95-4.02 (3H, m, H-4',5',5"), 4.49-4.54 (2H, m, H-2',3'), 5.25 (2H, s, CH₂Ph), 5.85 (1H, s, H-1'), 7.32 (5H, s, CH₂Ph), 8.19, 8.52 (two 1H singlets, H-2 and H-8).

### EXAMPLE 9

### 9-(2-O-Triflyl-β-L-ribofuranosyl)adenine (5, B = adenine, R² = CF₃SO₂-)

To a mixture of 9-(3,5-O-[1,1,3,3-tetraisopropyldisiloxan-1,3-yl]-β-L-ribofuranosyl)-adenine 7 [B = adenine, R³,R³ = -Si(*i*Pr)₂-O-(*i*Pr)₂Si-] ((5.0 g, 0.01 mol), *p*-dimethylaminopyridine (1.2 g, 0.01 mol) and triethylamine (2.4 mL, 0.02 mol) in methylene chloride (100 mL) is added triflyl chloride (2.12 mL, 0.02 mol), and the mixture is stirred at room temperature for 1 hour. After concentration of the mixture *in vacuo*, the residue is dissolved in 1N triethylamine hydrogen fluoride in tetrahydrofuran (30 mL). The mixture is kept overnight at room temperature, and then concentrated *in vacuo*. The residue is chromatographed on silica gel using chloroform-ethanol (9:1 v/v) as the eluent to obtain 5 (B = adenine, R² = CF₃SO₂-), 3.0 g (75 %) as a foam. ¹H-NMR δ 3.66-3.88 (2H, m, H-5',5"), 4.02-4.13 (1H, m, H-4') 4.48-4.64 (1H, m, H-3', becomes dd at 4.61 upon addition of deuterated water, J_{2',3'} = 5.0, J_{3',4'} = 5.5 Hz), 5.89 (1H, dd, H-2', J_{1',2'} = 4.4, J_{2',3'} = 5.0 Hz), 6.40 (1H, d, H-1', J_{1',2'} = 4.4 Hz), 8.20, 8.42 (two 1H singlets, H-2, H-8).

### EXAMPLE 10

### 9-(3,5-Di-O-acetyl-2-O-triflyl-β-L-ribofuranosyl)adenine (6, B = adenine, R² = CF₃SO₂-, R³ = Ac)

A mixture of **5** (B = adenine, R² = CF₃SO₂-) (4.0 g, 0.01 mol) and acetic anhydride (8 mL) in pyridine (100 mL) is left standing for 6 hours, and then concentrated *in vacuo*. The residue is dried by azeotropic distillation with toluene (50 mL x 4) and ethanol (50 mL x 4) to obtain quantitative yield of 6 (B = adenine, R² = CF₃SO₂-, R³ = Ac) as a foam. ¹H-NMR δ 1.98 (3H, s, Ac), 2.16 (3H, s, Ac), 4.11-4.51 (3H, m, H-4',5',5"), 5.87 (1H, t, H-3', J_{2',3'} = J_{3',4'} = 5.9 Hz), 6.35 (1H, dd, H-2', J_{1',2'} = 4.1, J_{2',3'} = 5.9 Hz), 6.53 (1H, d, H-1', J_{1',2'} = 4.1 Hz), 8.17, 8.57 (two 1H singlets, H-2 and H-8).

### EXAMPLE 11

### 1-(2-O-[imidazol-1-yl]thicarbonyl-3,5-O-[1,1,3,3-tetraisopropyldisiloxan-1,3-yl]-β-L-ribofuranosyl)thymine (8, B = thymine, R³,R³ = -Si(iPr)₂-O-(iPr)₂Si-, R⁴ = [imidazol-1-yl]thiocarbonyl)

A mixture of 7 (B = thymine, R³,R³ = -Si(*i*Pr)₂-O-(*i*Pr)₂Si-) (10.0 g, 0.02 mol) and thiocarbonyldiimidazole (7.12 g, 0.04 mol) in *N,N*-dimethylformamide (40 mL) is stirred for 4 hours at room temperature, and then partitioned between ethyl acetate (600 mL) and water (200 mL). The organic layer is separated, washed with water (2 x 150 mL), dried over sodium sulfate, and concentrated *in vacuo*. The residue is purified by chromatography on a silica gel column using ethyl acetate as the eluent to give 8.3 g (70 %) of **(8,** B = thymine, R³,R³ = -Si(*i*Pr)₂-O-(*i*Pr)₂Si-, R⁴ = [imidazol-1-yl]thiocarbonyl). ¹H-NMR δ 1.02 (28H, m, *i*-Pr), 1.62 (3H, s, 5-Me), 3.9-4.6 (3H, m, H-4',5',5"), 4.7 (1H, m, H-3'), 5.82 (1H, s, H-1'), 6.15 (1H, d, H-2', J2',3' = 4.5 Hz), 7.11 (1H, s, imidazole) 7.76 (1H, s, H-6), 7.86, 8.54 (two 1H singlets, imidazole).

### EXAMPLE 12

### 1-(2-Deoxy-3,5-O-[1,1,3,3-tetraisopropyldisiloxan-1,3-yl]-β-L-ribofuranosyl)thymine (9, B = thymine, R³,R³ = -Si(iPr)₂-O-(iPr)₂Si-)

To a refluxing solution of **8** (B = thymine, R³,R³ = -Si(*i*Pr)₂-O-(*i*Pr)₂Si-, R⁴ = [imidazol-1-yl]thiocarbonyl) (6.1 g, 0.01 mol) in dry toluene (100 mL) is added dropwise a solution of 2,2'-azobis(methylpropionitrile) (1 g) and tri-n-butyltin hydride (12 g, 0.04 mol) in toluene (100 mL) over 2 hours. The solvent is removed *in vacuo*, the residue dissolved in acetonitrile (100 mL), and the solution extracted with petroleum ether (3 x 50 mL). The acetonitrile solution is concentrated in vacuo, and the residue chromatographed over a silica gel column which is washed first with chloroform (1 L) to remove all the tri-*n*-butyltin derivatives, then chloroform-ethyl acetate (7:3 v/v) to give (9, B = thymine, R³,R³ = -Si(*i*Pr)₂-O-(*i*Pr)₂Si-) as syrup, 4.8 g (96 %). ¹H-NMR δ 1.02 (28H, m, *i*Pr), 1.60 (3H, s, 5-Me), 1.9-2.4 (2H, m, H-2',2"), 3.30 (1H, m, H-5'), 3.75 (1H, m, H-5''), 3.99 (1H, m, H-4'), 4.53 (1H, m, H-3'), 6.50 (1H, dd, H-1', J1',2' = 5.4, J1',2" = 8.8 Hz), 7.76 (1H, s, H-6).

### EXAMPLE 13

### 1-(2-Deoxy-β-L-ribofuranosyl)thymine (10, B = thymine, L-thymidine)

Compound **9** (B = thymine, R³,R³ = -Si(*i*Pr)₂-O-(*i*Pr)₂Si-) (4.84 g, 0.01 mol) is dissolved in 1M solution of triethylammonium fluoride in tetrahydrofuran (40 mL). After 16 hours at room temperature, the mixture is diluted with saturated sodium bicarbonate solution (40 mL) and concentrated in vacuo. The residue is partitioned between water (50 mL) and diethyl ether (50 mL). The aqueous layer is separated, washed with diethyl ether (50 mL), and then concentrated in vacuo. The residue is triturated with pyridine, insoluble salts are removed by filtration, and filtrate is concentrated *in vacuo,* and the residue purified by chromatography on silica gel using methylene chloride-tetrahydrofuran (1:2 v/v) as the eluent. The UV absorbing fractions are collected, concentrated *in vacuo*, and the residue crystallized from ethyl acetate, mp 183-185 °C, 1.93 g (79 %). The ¹H-NMR spectrum of this sample is identical to that of the natural thymidine.

### EXAMPLE 14

### 1,3,5-Tri-O-benzoyl-α-L-ribofuranose (11, R² = Bz)

Compound 2 (see Examples 1 and 2) (prepared from 50.4 g, 0.1 mol of 1) is dissolved in acetonitrile (100 mL). To the stirred solution is added dropwise water (12 mL) at 0 °C over 30 minutes. The mixture is kept at 0 °C for 3 hours, and the precipitated product is collected by filtration, washed with saturated sodium bicarbonate solution (30 mL), water (60 mL), and recrystallized from ethanol-hexane to give 11 (26.1 g, 57 %), mp 142 - 143 °C. The ¹H-NMR spectrum of this sample is identical to that of the D-counterpart.

### EXAMPLE 15

### 2-O-Acetyl-1,3,5-tri-O-benzoyl-α-L-ribofuranose (12, R¹ = Ac, R² = Bz)

Compound **11** (9.22 g, 0.02 mol) is dissolved in pyridine (50 mL). To the stirred solution is added acetic anhydride (5 mL), and the mixture is kept at room temperature overnight. Ethanol (10 mL) is added, and the mixture concentrated *in vacuo*. Traces of pyridine and acetic acid are removed several azeotropic distillation with toluene and ethanol from the residue to give crude 12 (R¹ = Ac, R² = Bz), 10.1 g (100 %). ¹H-NMR δ 2.10 (3H, s, Ac), 4,6-4.8 (3H, m, H-4,5,5"), 5.78 (1H, d, H-2, J_{1,2} = 8.0), 6.51 (1H, d, H-1, J_{1,2} = 8.0), 7.3-8.2 (15H, m, Ph).

### EXAMPLE 16

### 2-O-Acetyl-3,5-di-O-benzoyl-α-L-ribofuranosyl bromide (13, R¹ = Ac, R² = Bz, X' = Br)

Hydrogen bromide is bubbled into an ice-cold solution of **12** (10.1 g, 0.02 mol) in methylene chloride (100 mL) for 15 minutes. After being kept at 0 °C for 1 hour and at room temperature for 15 minutes, the solution is poured in a thin stream into ice-water (200 mL). The organic layer is separated, washed rapidly with ice-cold sodium bicarbonate solution (75 mL) and then ice-water (100 mL), dried over sodium bicarbonate, and concentrated *in vacuo*. The syrupy residue (13) is used immediately for condensation with appropriate purine or pyrimidine

### EXAMPLE 17

### 9-(2-O-Acetyl-3,5-di-O-benzoyl-β-L-ribofuranosyl)-N⁶-benzoyladenine (14, B = N⁶-benzoyladenine, R¹ = Ac, R² = Bz)

To a solution of N⁶-benzoyladenine (4.8 g, 0.02 mol) in dry *N*,*N*-dimethylformamide (50 mL) is added sodium hydride (60 % in mineral oil, 0.8 g, 0.02 mol). After the evolution of hydrogen has ceased, a solution of 13 (X' = Br, R² = Bz, R¹ = Ac, prepared from 10.1 g of 12) in *N*,*N*-dimethylformamide (20 mL) is added dropwise. The mixture, after being stirred overnight at room temperature, is added a few drops of acetic acid then diluted with cold water (100 mL), and extracted with methylene chloride (100 mL x 3). The combined organic layers are washed with water (100 mL x 2), dried over sodium sulfate, and evaporated *in vacuo* to a foam: ¹H-NMR δ 2.18 (3H, s, Ac), 4.75 (2H, m, H-5',5"), 4.46 (1H, m, H-4'), 5.50 (1H, t, H-3', ), 5.72 (1H, t, H-2'), 6.67 (1H, d, H-1'), 7.3-8.2 (15H, m, Bz), 8.31, 8.82 (two 1H singlet, H-2, H-8). Crude **14** (10.5 g, 88 %) is used directly in the next step.

### EXAMPLE 18

### 9-(3,5-di-O-benzoyl-β-L-ribofuranosyl)adenine (7, B = adenine, R² = Bz)

Crude **14** (6.0 g, 0.01 mol) is treated with 180 mL of 1% hydrogen chloride in methanol overnight at room temperature, and then evaporated *in vacuo*. The residue is crystallized from ethanol to give **7** (B = adenine, R² = Bz), 4.2 g (88 %), mp 192-194 °C. ¹H-NMR of this sample is identical with that of the D-isomer. (Ishido, Y.; Nakazaki, N.; Sakairi, *N. J. C. S., Perkin Trans. 1* **1979,** 2088).

### EXAMPLE 19

### 1,3,5-Tri-O-benzoyl-2-O-triflyl-α-L-ribofuranose (15, R¹ = Bz, R² = -SO₂CF₃)

Compound **11** (9.22 g, 0.02 mol) is dissolved in pyridine (50 mL). To the stirred solution is added trifluoroacetic anhydride (5 mL) at 0 °C, and the mixture is kept refrigerated overnight. Ethanol (10 mL) is added, and the mixture concentrated *in vacuo* below 35 °C. Traces of pyridine and trifluoroacetic acid are removed several azeotropic distillation *in vacuo* below 35°C with toluene and ethanol from the residue to give crude **15** (R¹ = Bz, R² = -SO₂CF₃), 11.1 g (100 %). This compound is rather unstable for further purification, and used directly in the next step. ¹H-NMR δ 4,6-4.8 (3H, m, H-4,5,5'), 5.23 (1H, m, H-3), 6.35 (1H, d, H-2, J_{1,2}= 8.0), 6.51 (1H, d, H-1, J_{1,2}= 8.0), 7.3-8.2 (15H, m, Ph).

### EXAMPLE 20

### 2-Deoxy-1,3,5-tri-O-benzoyl-2-thio-2-S-(4-oxopyrimidin-2-yl)-α-L-arabinofuranose (16, R¹ = Bz, B' = 4-oxopyrimidin-2-yl)

To a solution of 2-thiouracil (2.56 g, 0.02 mol) in *N,N*-dimethylformamide (50 mL) is added sodium hydride (60% in mineral oil, 0.6 g, 0.015 mol) with stirring. After evolution of hydrogen is ceased, the solution is added to a stirred solution of 15 (5.5 g, 0.01 mol dissolved in 50 mL of *N,N*-dimethylformamide). The mixture is heated at 60-70 °C overnight, and then concentrated *in vacuo*. The residue is taken up in methylene chloride, washed successively with saturated bicarbonate solution (75 mL x 2) and water (75 mL x 2), dried over sodium sulfate, and then evaporated *in vacuo* to give crude **16** (R¹ = Bz, B' = 4-oxopyrimidin-2-yl) as a foam, 5.7 g (100 %). ¹H-NMR δ 4,6-4.8 (3H, m, H-4',5',5"), 5.0-5.2 (2H, m, H-2',3'), 6.51 (1H, s, H-1'), 7.3-8.2 (15H, m, Ph).

### EXAMPLE 21

### 2,2'-Anhydro-1-(2-deoxy-2-thio-3,5-di-O-benzoyl-β-L-arabinofuranosyl)-2-thiouracil (17, R¹ = Bz, B = 4-oxopyrimidin-2-yl)

To a solution of crude **16** (5.7 g, 0.01 mol, R¹ = Bz, B' = 4-oxopyrimidin-2-yl) in methylene chloride (100 mL) is added dropwise stannic chloride (1.2 mL, 0.01 mol) at 0 °C, and the mixture is stirred overnight at room temperature. Methanol (20 mL) is added to the mixture while stirring, and the precipitates are filtered through a Celite pad which is thoroughly washed with methylene chloride (100 mL). The combined filtrate and washings are washed with water (100 mL x 2), saturated sodium bicarbonate solution (100 mL) and water (100 mL), dried over sodium sulfate, and concentrated *in vacuo*. The residue is chromatographed on a silica gel column using chloroform-methanol (7:1 v/v) as the eluent to give pure **17** (R¹ = Bz, B = 4-oxopyrimidin-2-yl), 3.0 g (72 %). ¹H-NMR δ 3.8-4.5 (3H, m, H-4',5',5"), 4.67 (1H, dd, H-2', J_{1',2'} = 7.1, J_{2',3'} = 2.2 Hz), 5.20 (1H, dd, H-3', J_{2',3'} = 2.2, J_{3',4'} = 3.8 Hz), 5.93 (1H, d, H-5, J_{5,6} = 7.7 Hz), 6.48 (1H, d, H-1', J_{1',2'} = 7.1 Hz), 7.3-8.2 (11H, m, H-6 and Ph).

### EXAMPLE 22

### 8,2'-Anhydro-9-(2-deoxy-2-thio-3,5-di-O-benzoyl-β-L-arabinofuranosyl)adenine (17, R¹ = Bz, B = adenin-8-yl)

To a solution of crude **16** (6.1 g, 0.01 mol, R¹ = Bz, B' = adenin-8-yl) in a mixture of acetic anhydride (20 mL) and acetic anhydride (30 mL) is added dropwise concentrated sulfuric acid (4.0 mL) at 0 °C. The mixture, after being stirred overnight at room temperature, is partitioned between ice-water (100 mL) and methylene chloride (100 mL). The organic layer is separated, washed successively with cold water (50 mL x 2), saturated sodium bicarbonate solution (50 mL x 2) and water (50 mL x 2), dried over sodium sulfate, and evaporated *in vacuo*. Traces of acetic acid are removed by azeotropic distillation with toluene. The residue (4.3 g, 88 %), crude 17 (R¹ = Bz, B = adenin-8-yl).

### EXAMPLE 23

### 8,2'-Anhydro-9-(2-deoxy-2-thio-β-L-arabinofuranosyl)adenine (17, R¹ = H, B = adenin-8-yl)

To a boiling solution of crude **17** (2.5 g, 0.005 mol, R¹ = Bz, B' = adenin-8-yl) in ethanol (50 mL) is added dropwise a freshly prepared 1M solution of sodium methoxide in methanol (1.2 mL), and the mixture heated under reflux for 1 hour, then concentrated *in vacuo*. The residue is triturated with diethyl ether (25 mL x 2), and the solid material dissolved in water (50 mL). After neutralization to pH 2 with IN hydrochloric acid, the aqueous solution is extracted with diethyl ether (50 mL x 2) and then freeze-dried. The residue is crystallized from a small amount of water to give 770 mg (52 %) of 17 (R¹ = H, B = adenin-8-yl), mp 191-194 °C. UV λₘₐₓ (ethanol) 276 nm.

### EXAMPLE 24

### 9-(2-Deoxy-β-L-erythropentofuranosyl)adenine (10, B = adenine or 2'-deoxy-L-adenosine)

Compound **17** (300 mg, 0.001 mol, R¹ = H, B = adenine) is refluxed in water (30 mL) with Raney nickel (2 g) for 6 hours. After the catalyst is filtered off, the solution is evaporated *in vacuo,* and the residue crystallized from a small amount of water to give 108 mg (40 %) of 2'-deoxy-L-adenosine (10, B = adenine), mp 184-187 °C. UV λₘₐₓ (H₂O) 260 nm.

### EXAMPLE 25

### 2-Acetylthio-1,3,5-tri-O-benzoyl-2-deoxy-L-arabinofuranose (18, R¹ = Bz)

To a solution of 1,3,5-tri-O-benzoyl-2-O-triflyl-L-ribofuranoside (15, 11.1 g, 0.02 mol) in *N*-methyl-2-pyrrolidinone (100 mL) is added potassium thioacetate (3.4 g), and the mixture is stirred for 6 hours at 75 °C, and then concentrated *in vacuo.* The residue is dissolved in methylene chloride (100 mL), filtered, and the filtrate is evaporated to dryness *in vacuo* to give crude 2-acetylthio-1,3,5-tri-O-benzoyl-2-deoxy-L-arabinofuranoside **(18,** R¹ = Bz) (11.2 g, 100%). ¹H-NMR shows that the major product is an α anomer. ¹H-NMR (major signals) δ 2.41 (3H, s, SAc), 3.52 (2H, m, H-5,5'), 4.12 (1H, m, H-4), 4.25 (1H, m, H-3), 4.35 (1H, m, H2), 4.92 (1H, s, H-1), 7.24-7.40 (15H, m, Ph).

### EXAMPLE 26

### 2-Deoxy-1,3,5-tri-O-benzoyl-2-thio-2-S-(4-methoxypyrimidin-2-yl)-α-L-arabino-furanose (16, R¹ = Bz, B' = 4-methoxypyrimidin-2-yl)

To a solution of **18** (R¹ = Bz, 11.2 g, 0.02 mol) in *N*,*N*-dimethylformamide (120 mL) is added 1N sodium hydroxide solution (20 mL) at 0 °C with stirring. After 2 hours at room temperature, a solution of 2-chloro-4-methoxypyrimidine (2.9 g, 0.02 mol) in 50 mL of *N,N*-dimethylformamide) is added to a stirred solution of **18.** The mixture is stirred at room temperature overnight, and then concentrated *in vacuo*. The residue is taken up in methylene chloride, washed successively with water (75 mL), 0.5N hydrochloric acid (75 mL), saturated bicarbonate solution (75 mL) and water (75 mL), dried over sodium sulfate, and then evaporated *in vacuo* to give crude **16** (R¹ = Bz, B' = 4-methoxypyrimidin-2-yl) as a foam, 11.0 g (100 %). ¹H-NMR δ 3.85 (3H, s, OCH3), δ 4,6-4.8 (3H, m, H-4',5',5"), 5.0-5.2 (2H, m, H-2',3'), 6.51 (1H, s, H-1'), 7.3-8.2 (15H, m, Ph).

### EXAMPLE 27

### 9-(β-L-Xylofuranosyl)adenine (22, B = adenine)

To a solution of 18 g of hydrogen bromide in 70 mL of *p*-dioxane is added 9.6 g (0.03 mol) of 1,2,3,5-tetra-O-acetyl-L-xylofuranose **20**. (This compound is prepared by following the same procedure for 1,2,3,5-tetra-O-acetyl-D-xylofuranose, except L-xylose is used instead of D-xylose (Reist, E. J.; Goodman, L. *Biochemistry* **1964**, *3*, 15)). The temperature is kept below 20 °C during this addition. The mixture is diluted with toluene (70 mL) and the solvent removed *in vacuo*. Traces of hydrogen bromide is removed by azeotropic distillation with toluene (70 mL x 2), and the residue is dissolved in dry acetonitrile (75 mL). This solution is added to a suspension of N⁶-benzoylsodioadenine prepared by treatment of 7.1 g (0.03 mol) of N⁶-benzoyladeine with sodium hydride (60% in mineral oil, 1.2 g, 0.03 mol) in *N,N*-dimethylformamide (120 mL) while stirring. After stirring at room temperature overnight, the mixture is concentrated *in vacuo*, and the residue is treated with 1M solution of sodium methoxide in methanol (100 mL) overnight at room temperature. Acetic acid (5 mL) is added, and the mixture is concentrated *in vacuo*. The residue is dissolved water (100 mL) and the solution is passed through a bed of Amberlite IRC-50. The resin is washed with water, and combined aqueous solutions are concentrated *in vacuo* to give crude **22** (B = adenine) as a glass (6.2 g, 78 %).

### EXAMPLE 28

### 9-(3,5-O-Isopropylidene-β-L-xylofuranosyl)adenine (23, B = adenine)

A mixture of crude **22** (5.3 g, 0.02 mol), ethanesulfonic acid (7 g) and 2,2-dimethoxypropane (20 mL) in acetone (200 mL) is stirred overnight at room temperature, and the solution is decanted into 100 mL of saturated sodium bicarbonate solution. The mixture is stirred for 30 minutes at room temperature, and filtered, and concentrated to about 30 mL, and extracted five 60 mL portions of chloroform. The combined chloroform extracts are dried over sodium sulfate, and then concentrated, and the residue crystallized from ethanol to give **23** (B = adenine), 4.0 g (65 %), mp 203-207 °C. The reported mp for the D-isomer is 204-207. (Baker, B. R.; Hewson, K. *J. Org. Chem*. **1957**, *22*, 966).

### EXAMPLE 29

### 9-(3,5-O-Isopropylidene-2-O-phenoxythiocarbonyl-β-L-xylofuranosyl)adenine (24, B= adenine, R" = OPh)

To a mixture of **23** (B = adenine) (3.1 g, 0.01 mol) and p-dimethylaminopyridine (1.2 g, 0.01 mol) in pyridine (60 mL) is added phenyl chlorothionofonnate (2 g, 1.16 mol), and the mixture is stirred at room temperature for 4 hours. The solvent is removed *in vacuo*, and the residue is taken up in methylene chloride (60 mL), washed with water (50 mL x 2), dried over sodium sulfate, and concentrated *in vacuo* to give crude 24 (B = adenine), 4.4 g (100 %). ¹H-NMR shows that this material contains isopropylidene and phenyl groups. Without further purification, crude 24 is directly processed in the next step.

### EXAMPLE 30

### 9-(2-Deoxy-3,5-O-isopropylidene-β-L-threopentofuranosyl)adenine (25, B = adenine)

To a refluxing solution of **24** (B = adenine) (4.4 g, 0.01 mol) in dry toluene (100 mL) is added dropwise a solution of 2,2'-azobis(methylpropionitrile) (1 g) and tri-n-butyltin hydride (12 g, 0.04 mol) in toluene (100 mL) over 2 hours. The solvent is removed *in vacuo*, the residue dissolved in acetonitrile (100 mL), and the solution extracted with petroleum ether (3 x 50 mL). The acetonitrile solution is concentrated *in vacuo*, and the residue chromatographed over a silica gel column which is washed first with chloroform (1 L) to remove all the tri-*n*-butyltin derivatives, then chloroform-ethyl acetate (7:3 v/v) to give (25, B = adenine) as a foam, 2.6 g (89 %). 1H-NMR δ 1.35 (3H, s, *i*-Pr), 1.50 (3H, s, *i*-Pr), 2.0-2.6 (2H, m, H-2',2"), 3.32 (1H, m, H-5'), 3.76 (1H, m, H-5"), 3.89 (1H, m, H-4'), 4.49 (1H, m, H-3'), 6.05 (1H, dd, H-1', J_{1',2'} = 1.4, J_{1',2"} = 7.8 Hz), 8.72 and 8.51 (two 1H, s, H-2 and 8).

### EXAMPLE 31

### 1-(3,5-O-Isopropylidene-β-L-xylofuranosyl)thymine (28, X = OH)

A mixture of **22** (B = thymine) (5.2 g, 0.02 mol), *p*-toluenesulfonic acid (1 g), 2,2-dimetho-xypropane (5 mL) and acetone (100 mL) is stirred for 8 hours at room temperature. Solid sodium bicarbonate (2 g) is added, and the mixture is stirred for 2 hours, filtered, and the filtrate is concentrated *in vacuo*. The residue is recrystallized from methanol to give 28 (5.4 g, 91%), mp 175-177 °C. The ¹H-NMR spectrum of this sample is identical to that of the D-counterpart prepared previously. (Fox, J. J.; Codington, J. F.; Yung, N. C.; Kaplan, L.; Lampen, J. O. *J. Am. Chem. Soc.* **1958**, *80,* 5155)*.*

### EXAMPLE 32

### 1-(3,5-O-Isopropylidene-2-O-mesyl-β-L-xylofuranosyl)thymine (29, R = R'' = CH₃, X=OH)

To a solution of **23** (B = thymine) (3.0 g, 0.01 mol) in pyridine (50 mL) is added mesyl chloride (1 mL, 0.013 mol). After being stirred overnight at room temperature, the mixture is poured into ice-water (300 mL). The solid precipitates are collected by filtration, and crystallized from ethanol to give 28 (R'' = CH₃), 3.0 g, (80 %), mp 163-165 °C. The melting point of the D-counterpart is reported to be 162-165 °C. (Fox, J. J.; Codington, J. F.; Yung, N. C.; Kaplan, L.; Lampen, J. O. *J. Am. Chem. Soc.* **1958**, *80*, 5155).

### EXAMPLE 33

### 2,2'-Anhydro-1-(3,5-O-isopropylidene-β-L-lyxofuranosyl)thymine (30, X'=O, R=CH₃)

To a suspension of **29** (R = R'' = CH₃, X = OH) (3.8 g, 0.01 mol) in ethanol (300 mL) is added IN sodium hydroxide (11 mL) while stirring, and the mixture is heated to reflux overnight. The solvent is removed *in vauo*, and the residue is crystallized from water to give **30** (X' = O, R = CH₃), 2.1 g (75 %), mp 258-261 °C. The mp of the D-isomer is reported to be 259-262 °C.*8

### EXAMPLE 34

### 1-(2-S-Acetylthio-2-deoxy-3,5-O-isopropylidene-β-L-xylofuranosyl)thymine (31, R = CH₃, R² = Ac, X = OH)

To a solution of **30** (R = CH₃, X' = O) (1.4 g, 5 mmol) in *N*-methyl-2-pyrrolidinone (50 mL) is added potassium thioacetate (1.1 g, 10 mmol), and the mixture is stirred overnight at 65-75 oC. The mixture is concentrated *in vacuo*, and the residue is partitioned between methylene chloride (50 mL) and water (50 mL). The organic layer is dried over sodium sulfate and evaporated to dryness *in vacuo* to give **31** (R = CH₃, R² = Ac, X = OH), (1.7 g, 95%). ¹H-NMR δ 1.41 (3H, s, *i*Pr), **1.49** (3H, s, *i*Pr), 1.88 (3H, s, 5-CH₃), 2.33 (3H, s, SAc), 4.07 (1H, m, H-4'), 4.14 (2H, m, H-5',5"), 4.45 (1H, m, H-3'), 5.35 (1H, s, H-2'), 6.08 (1H, s, H-1), 7.87 (1H, s, H-6).

### EXAMPLE 35

### 1-(3,5-O-Isopropylidene-2-O-triflyl-β-L-xylofuranosyl)adenine (33, R''' = CF₃, X = NH₂,Y=Z=H)

To a solution of **23** (B = adenine) (2.9 g, 0.01 mol) in pyridine (50 mL) is added triflyl chloride (1.5 g, 0.011 mol). After being stirred overnight at room temperature, the mixture is poured into ice-water (300 mL). The supernatant is decanted, the precipitates are taken up in methylene chloride (50 mL), dried over sodium sulfate, and concentrated *in vacuo* to give crude 33 (R'' = CF₃, X = NH₂, Y = Z = H) (4.0 g, 100 %). This compound is rather unstable and used directly in the next step.

### EXAMPLE 36

### 1-(2-Acetylthio-2-deoxy-3,5-O-Isopropylidene-β-L-lyxofuranosyl)adenine (34, X = NH₂, Y=Z=H)

To a solution of **33** (X = NH₂, Y = Z = H) (4.2 g, 0.01 mol) in *N*-methyl-2-pyrrolidinone (80 mL) is added potassium thioacetate (2.2 g, 20 mmol), and the mixture is stirred overnight at 65-75 °C. The mixture is concentrated *in vacuo*, and the residue is partitioned between methylene chloride (80 mL) and water (80 mL). The organic layer is dried over sodium sulfate and evaporated to dryness *in vacuo* to give **34** (X = NH₂, Y = Z = H), (3 g, 83%). ¹H-NMR 6 1.36 (3H, s, *i*-Pr), 1.42 (3H, s, *i*-Pr), 2.03 (3H, s, SAc), 4.16 (1H, m, H-4'), 4.02 (2H, m, H-5',5"), 4.43 (1H, m, H-3'), 5.24 (1H, s, H-2'), 6.12 (1H, s, H-1), 8.52 and 8.71 (two 1H, s, H-2 and 8).

### EXAMPLE 37

### 1-(3,5-O-Isopropylidene-β-L-threopentofuranos-2-ulosyl)adenine (35, X=NH₂, Y=Z=H)

To a mixture of **23** (X=NH₂, Y=Z=H) (2.9 g, 0.01 mol), finely pulverized 3Å molecular sieve (6 g) in methylene chloride (50 mL) is added a solution of pyridinium dichromate (6 g, 0.016 mol) in methylene chloride (40 mL). After stirring the mixture for 1 hour, isopropanol (12 mL) is added, and the stirring continued for 1 hour, and then filtered through a Celite pad. The filtrate is concentrated *in vacuo,* and the residue is triturated well with ethyl acetate (2 x 200 mL). The combined organic layers are dried over sodium sulfate, and then concentrated to dryness to give crude 35 (X = NH₂, Y = Z = H) (2.9 g, 100%). ¹H-NMR δ 1.34 (3H, s, *i*Pr), 1.41 (3H, s, *i*Pr), 4.00 (2H, m, H-5',5"), 4.16 (1H, m, H-4'), 4.43 (1H, m, H-3'), 6.52 (1H, s, H-1'), 8.52 and 8.71 (two 1H, s, H-2 and 8).

### EXAMPLE 38

### 1-(3,5-O-Isopropylidene-β-L-threopentofuranos-2-ulosyl)adenine 2-tosylhydrazone (36, B = adenine)

To a mixture of **35** (B = adenine) (2.9 g, 0.01 mol) and *p*-toluenesulfonylhydrazine (2.8 g, 0.02 mol) in ethanol (75 mL) is heated to reflux for 4 hours. After standing at room temperature overnight, the precipitated product (36, B = adenine) is collected by filtration (3 g, 83%). ¹H-NMR 6 1.36 (3H, s, *i*Pr), 1.42 (3H, s, *i*Pr), 2.35 (3H, s, *CH*₃Ph), 4.16 (1H, m, H-4'), 4.02 (2H, m, H-5',5"), 4.43 (1H, m, H-3'), 6.12 (1H, s, H-1'), 7.35-7.85 (4H, CH₃*Ph*), 8.51 and 8.72 (two 1H, s, H-2 and H-8).

### EXAMPLE 39

### 5-O-tert-Butyldiphenylsilyl-β-L-arabinose (37, R¹ = tBuPh₂Si)

A mixture of L-arabinose (360 g, 2.4 mol), *tert*-butylchlorodiphenylsilane (605 g, 2.2 mol) and imidazole (150 g, 2.2 mol) in *N*,*N*-dimethylformamide (3 L) is stirred over night at room temperature. Solid precipitates are removed by filtration, and the filtrate is condensed *in vacuo* below 65 °C. The residue is dissolved in methylene chloride (3 L) and washed with cold water (1 L x 2). The organic layer is concentrated *in vacuo* and the residue is azeotropically dried with toluene (300 mL x 3). The syrupy residue (855 g, quantitative yield) contains one *tert*-butyl group (¹H NMR, δ 1.05, s, 9H) and two phenyl groups (δ, 7.40, m, 6H; 7.67, d, 4H), anomeric proton (δ 5.90, narrow doublet, 1H), also, H-2 and H-3 are observed (δ 4.59, apparent s, 1H; and δ 4.42, apparent s, 1H). The H-4 and H-5,5' signals appear at δ 4.04 (m, 1H) and δ 3.81 (m, 2H), respectively. This syrup apparently consists of only the β-anomer as judged by ¹H NMR.

### EXAMPLE 40

### 5-O-tert-Butyldiphenylsilyl-1,2-O-isopropylidenen-β-L-arabinofuranose (38, R¹ = tBuPh₂Si, R' = R" = CH₃)

To a solution of the above syrupy residue (855 g, 2.2 mol) in acetone (5 L) are added anhydrous copper sulfate (500 g) and then concentrated sulfuric acid (50 mL), and the mixture is stirred overnight at room temperature. Solid materials are filtered, and the filtrate is neutralized by addition of solid sodium hydrogen carbonate (200 g). After stirring overnight at room temperature, the mixture is filtered, and the solvent removed *in vacuo* to a syrup which is dissolved in diethyl ether (2 L), and the filtrate concentrated *in vacuo* to give 5-O-*tert*-butyldiphenylsilyl-1,2-O-isopropylidenen-β-L-arabinofuranose as a syrup (38, R¹ = *t*-BuPh₂Si, R' = R" = CH₃) (940 g, quantitative): ¹H-NMR, δ 1.02 (3H, s, *t*-Bu), 1.04 (3H, s, *t*-Bu), 1.06 (3H, s, *t*-Bu), 1.32 (3H, s, *i*-Pr), 1.53 (3H, s, *i*-Pr), 3.77 (2H, m, H-5,5'), 4.08 (1H, apparent t, H-4), 4.20 (1H, s, H-3), 4.56 (1H, s, H-2), 5.92 (1H, s, H-1), 7.40 (6H, m, Ph), 7.78 (4H, m, Ph).

### EXAMPLE 41

### 1,2-O-Isopropylidenen-β-L-arabinofuranose (39, R' = R'' = CH₃)

The above syrup (940 g, 2.2 mol) is dissolved in ethyl acetate (3 L), and to this solution is added 75% aqueous tetrabutylammonium fluoride. The mixture is stirred at room temperature for 3 hours, and then diluted with water (1 L). The aqueous layer is separated, the organic layer is washed with water (2 x 1L), the combined aqueous layers is washed with ethyl acetate (2 x 1L), and then condensed *in vacuo* below 40 °C. The solid residue is recrystallized from ethanol and diethyl ether to give 1,2-O-isopropylidene-β-L-arabinofuranose (**39**, R' = R" = CH₃) (222 g, 53% overall yield from L-arabinose). Mp. 117-118°C. ¹H-NMR δ 1.53 (6H, s, 2 x CH₃), 3.77 (2H, m, H-5,5'), 4.10 (1H, m, H-4), 4.26 (1H, brs, H-3), 4.58 (1H, d, H-2, J_{2,3}=4.1 Hz), 5.94 (1H, d, H-1, J_{1,2}=4.1 Hz).

### EXAMPLE 42

### 3,5-Di-O-benzyl-1,2-O-isopropylidene-β-L-arabinofuranose (40, R² = benzyl, R' = R" =CH₃)

To a solution of 1,2-O-isopropylidene-β-L-arabinofuranose (190 g, 1 mol) in *N,N*-dimethylformamide (500 mL) is added portionwise sodium hydride (60 g) while stirring. After 30 minutes, benzyl bromide (360 g, 2.1 mol) is added, and the mixture is stirred overnight at room temperature, concentrated to a syrup which is dissolved diethyl ether (500 mL), filtered from insoluble materials, and then condensed *in vacuo* to give 3,5-di-O-benzyl-1,2-O-isopropylidene-β-L-arabinofuranose (40, R² = benzyl, R' = R" = CH₃) (370 g, 100 %) as a syrup. ¹H-NMR δ 1.26 (3H, s, CH₃), 1.42 (3H, s, CH₃), 3.62 (2H, m, H-5,5'), 4.24 (1H, m, H-4), 4.46-4.64 (6H, m, H-2,3, 2 x PhCH₂), 5.90 (1H, d, H-1, J_{1,2} = 2.88 Hz), 7.25-7.40 (10H, m, 2 x Ph).

### EXAMPLE 43

### Methyl 3,5-di-O-benzyl-L-arabinofuranoside (41, R = CH₃, R² = benzyl)

To a solution of 3,5-di-O-benzyl-1,2-O-isopropylidene-β-L-arabinofuranose (370 g) in methanol (2 L) is added concentrated sulfuric acid (100 g), and then is refluxed for 30 minutes. The mixture is neutralized with 10N sodium hydroxide (110 mL). The mixture is concentrated *in vacuo*, and the residue dissolved in methylene chloride (2 L), filtered from solid inorganic materials. The filtrate apparently contains anomers of 41 (R = CH₃, R² = benzyl) in about 2:1 ratio. An aliquot from the filtrate is concentrated to dryness for ¹H-NMR characterization. ¹H- NMR: δ 3.44 and 3.49 for glycoside methyl (a total of 3Hs), 4.89 and 5.00 (anomeric doublet and singlet), 7.30-7.40 (10H, m, Ph)

### EXAMPLE 44

### Methyl 3,5-di-O-benzyl-2-O-triflyl-L-arabinofuranoside (42, R = CH₃, R² = benzyl, R³ = SO₂CF₃)

The above filtrate is cooled to -78 °C. To the mixture are added trifluoroacetic anhydride (315 g) and 2,6-lutidine (161 g) while stirring. After stirring for 5 hours at -78 °C, the reaction is quenched by addition of 2M citric acid solution (I L). The organic layer is separated, washed with cold water (2 x 1 L), passed through a pad of silica gel (ca. 10 cm thick), and concentrated *in vacuo* to give methyl 3,5-di-O-benzyl-2-O-triflyl-L-arabinofuranoside (42, R = CH₃, R² = benzyl, R³ = SO₂CF₃) (390 g, 84% overall yield from 1,2-O-isopropylidene-β-L-arabino-furanose).

### EXAMPLE 45

### Methyl 2-acetylthio-3,5-di-O-benzyl-2-deoxy-L-ribofuranoside (43, R = CH₃, R² = benzyl, R'''= Ac)

To a solution of methyl 3,5-di-O-benzyl-2-O-triflyl-L-arabinofuranoside (4.8 g) in *N*-methyl-2-pyrrolidinone (100 mL) is added potassium thioacetate (1.7 g), and the mixture is stirred for 4 hours at 50 °C, and then concentrated *in vacuo*. The residue is dissolved in methylene chloride (50 mL), filtered, and the filtrate is evaporated to dryness *in vacuo* to give methyl 2-acetylthio-3,5-di-O-benayl-2-deoxy-L-ribofuranoside (43, R = CH₃, R² = benzyl, R'''= Ac) (4.0 g). ¹H-NMR shows it is a 12:1 mixture of β and α anomers. These anomers are separated on a silica gel column: ¹H-NMR β anomer, δ 2.41 (3H, s, SAc), 3.39 (3H, s, OCH₃), 3.60 (2H, m, H-5,5'), 4.20 (1H, m, H-4), 4.25 (1H, m, H-3), 4.35 (1H, m, H-2), 4.92 (1H, s, H-1), 7.24-7.40 (10H, m, Ph); α anomer, δ, 2.40 (3H, s, SAc), 3.40 (2H, m, H-5,5'), 3.45 (3H, s, OCH₃), 4.00 (1H, m, H-4), 4.12 (1H, m, H-3), 4.28 (1H, m, H-2), 5.08 (1H, d, H-1), 7.25-7.40 (10H, m, Ph).

### EXAMPLE 46

### 1-(3,5-Di-O-acetyl-α-D-glyceropento-4-enofuranosyl)thymine (55, B=thymine, R'=R"=Ac)

A mixture of **54** (B = thymine, R' = Ac) (Pfitzner, K. E.; Moffatt, J. G. *J. Am. Chem. Soc*. **1965,** 87, 5661) (2.8 g, 0.01 mol), anhydrous potassium carbonate (5.5 g, 0.04 mol) and acetic anhydride (50 mL) is heated at 80 minutes for 1 hour. Excess acetic anhydride is removed *in vacuo*, and to the residue is stirred with chloroform (250 mL), filtered and the solid is washed with chloroform (2 x 50 mL). The combined filtrate and washings are evaporated *in vacuo*, and the residue is chromatographed on a silica gel column using methylene chloride-methanol (19:1 v/v) as the eluent. After concentration of the appropriate fractions, 55 (B = thymine, R' = R'' = Ac) is obtained as a foam, 3.7 g (56 %). ¹H-NMR δ 1.86 (3H, s, 5-CH₃), 2 06 (3H, s, 3'-OAc), 2.15 (3H, s, 5'-OAc), 2.43 (1H, q, H-2', J_{2',2"} = 13.0, J_{1',2'} = 6.6 Hz), 2.75 (1H, q, J_{2',2"} = 13.0, J_{1',2"} = 6.7 Hz), 4.58 (1H, m, H-4'), 5.65 (1H, m, H-3'), 5.78 (1H, t, H-1', J1',2' = J1',2" = 6.6 Hz), 6.92 (1H, s, H-5'), 7.75 (1H, s, H-6).

### EXAMPLE 47

### 1-(3,5-Di-O-acetyl-β-D-threopentofuranosyl)thymine (56, B=thymine, R'=R"=Ac)

Compound 55 (B=thymine, R'=R"=Ac) (3.2 g, 0.01 mol) is dissolved ethanol (250 mL), and hydrogenated over 10% Pd-C catalyst in a Parr apparatus at an initial pressure of 4 atm. for 3 hours. The catalyst is removed by filtration, and the filtrate is concentrated in vacuo. The residue is chromatographed on a silica gel column using methylene chloride-methanol (19:1 v/v). The major UV-absorbing fraction is concentrated *in vacuo* to obtain **56** (B=thymine, R'=R"=Ac), 2.6 g (81 %). ¹H-NMR δ 1.85 (3H, s, 5-CH₃), 2 06 (3H, s, 3'-OAc), 2.13 (3H, s, 5'-OAc), 2.41 (1H, q, H-2', J_{2',2"} = 13.0, J_{1',2'} = 6.6 Hz), 2.69 (1H, q, J_{2',2"} = 13.0, J_{1',2"} = 6.7 Hz), 4.23-4.38 (2H, m, H-5',5"), 4.58 (1H, m, H-4'), 5.65 (1H, m, H-3'), 5.75 (1H, t, H-1', J_{1',2'} = J_{1',2"} = 6.6 Hz), 7.75 (1H, s, H-6).

### EXAMPLE 48

### Methyl β-L-arabinopyranoside

A mixture of L-arabinose (100 g) in methanol containing 1.5% of hydrogen chloride (1 L) is gently refluxed for 3 hours. After cooling to room temperature solid sodium hydrogen carbonate (100 g) is added portion-wise with stirring. The mixture is kept in a refrigerator overnight and filtered. The filtrate is concentrated in vacuo to a thin syrup, which is allowed to crystallize. About 30 g of crude methyl-β-L-arabino-pyranoside is obtained which can be purified by an extraction with hot ethyl acetate. The residue is recrystallized from ethanol, mp 169 °C. From the mother liquor additional amount is obtained.

### EXAMPLE 49

### Benzyl 3,4-O-isopropylidene-β-L-arabinopyranoside

L-Arabinose (200 g) is dissolved in benzyl alcohol saturated with hydrogen chloride at 0 °C (1 L), and the mixture is stirred at room temperature overnight. Ethyl acetate (1.5 L) is added slowly while stirring, and the mixture kept in a refrigerator for 2 hours, and then filtered. The solid is treated with 2,2-dimethoxypropane (400 mL) in acetone (2.5 L) in the presence of p-toluenesulfonic acid monohydrate (5 g) for 2 hours at room temperature. After neutralization with triethylamine, the mixture is concentrated in vacuo. The residue is placed on top of a silica gel pad (20 cm x 10 cm-diameter) and washed with a 3:1 mixture of n-hexane and ethyl acetate. Benzyl 3,4-O-isopropylidene-β-L-arabinopyranoside (340 g) is obtained as a white solid, mp 52 °C. ¹H NMR (CDCl₃) δ 7.39-7.30 (m, 5H, CH₂Ph), 4.94 (d, 1H, H-1, J1,2 = 3.6 Hz), 4.76 (d, 1H, CH₂Ph, J = 11.7 Hz), 4.55 (d, 1H, CH₂Ph, J= 11.7 Hz), 4.25-4.21 (m, 1H, H-2), 4.21 (q, 1H, H-3, J = 6.1 Hz), 4.01 (dd, 1H, H-5', J5,5' = 13.2 Hz, JS',4 = 2.4 Hz), 3.94 (dd, 1H, H-5, J5,5' =13.2 Hz, J5,4 =1.1 Hz), 3.80 (broad d, 1H, H-4, J = 3.2 Hz), 2.28 (broad s, 1H, OH), 1.53 (s, 3H, CH3), 1.36 (s, 3H, CH3).

### EXAMPLE 50

### Benzyl 3,4-O-isopropylidene-2-O-phenoxythiocarbonyl-β-L-arabino-pyranoside.

To a stirred solution of thiophospgene (40 mL) in methylene chloride (500 mL) is slowly added (over 30 minutes) a solution of phenol (55 mL) and pyridine (60 mL) in methylene chloride (250 mL) at 0 °C. The resulting dark red solution is stirred for 30 minutes at room temperature. To this solution is added a solution of benzyl 3,4-O-isopropylidene-β-L-arabino-pyranoside (100 g) in a mixture of pyridine (60 mL) and methylene chloride (250 mL) over 30 minutes. The resulting dark green solution is stirred for 1 hour at room temperature, and diluted with methylene chloride (1 L) and washed with water (100 mL x 5), saturated sodium bicarbonate solution and brine. The organic layer is dried (MgSO₄), filtered, and concentrated in vacuo to give crude benzyl 3,4-O-isopropylidene-2-O-phenoxythiocarbonyl-β-L-arabino-pyranoside, which is used in the next step without further purification.

### EXAMPLE 51

### Benzyl 3,4-O-isopropylidene-2-deoxy-β-L-erythropentopyranoside.

To a refluxing solution of crude benzyl 3,4-O-isopropylidene-2-O-phenoxythiocarbonyl-β-L-arabino-pyranoside prepared above in toluene (1.5 L) is added a solution of tri-n-butyltin hydride (115 mL) and AIBN (12 g) in toluene over 1 hour. After addition, the brown solution is stirred for additional 30 minutes under reflux. The mixture is concentrated in vacuo, and the residue is placed on top of a silica gel pad (20 cm x 20 cm-diameter) and eluted with 6:1 mixture of n-hexane and ethyl acetate. The eluent is washed with 5% sodium hydroxide to remove phenol, the with water and brine, and dried (MgS04). After evaporation of the solvent, 90 g of benzyl 3,4-O-isopropylidene-β-L-*erythro*pentopyranoside is obtained. The product is sufficiently pure to be used in the next step. ¹H NMR (CDCl₃) δ 7.47-7.35 (m, 5H, CH2Ph), 5.08 (t, 1H, H-1, Jl,2a = Jl,2b = 5.3 Hz), 4.88 (d, 1H, CH2Ph, J_{gem} = 11.9 Hz), 4.60 (d, 1H, CH2Ph, J_{gem} = 11.9 Hz), 4.56 (q, 1H, H-3, J = 5.7 Hz), 4.25 (dt, 1H, H-4, J4,3 = 6.5, J4,5a = 2.6 Hz), 4.00 (dd, 1H, H-5a, J5a,5b = 12.9, J5a,4 = 2.6 Hz), 3.87 (dd, 1H, H-5b, J5a,5b =12.9 Hz, J5b,4 = 2.6 Hz), 2.27 (dt, 1H, H-2a, J2a,2b = 14.7 Hz, J2a,1 = J2a,3 = 4.6 Hz), 1.96 (ddd, 1H, H-2b, J2b,2a = 14.7 Hz, J2b,1 = 6.0 Hz, J2b,3 = 5.7 Hz), 1.61 (s, 3H, CH3), 1.44 (s, 3H, CH3).

### EXAMPLE 52

### 2'-Deoxy-α-cytidine (53, R = R' = R'' = H, X = NH₂)

2'-Deoxycytidine **(61,** R = R' = R'' = H, X = NH₂) (4.5 g, 0.02 mol) is dissolved in pyridine (50 mL), and acetic anhydride (10 mL) is added. The mixture is stirred overnight, and then diluted with ethanol (20 mL). After stirring the mixture for 30 minutes, the mixture is concentrated *in vacuo*. Traces of acetic acid and pyridine are removed by several azeotropic distillation with ethanol and toluene, and the residue dissolved in N-methylpyrrolidinone (100 mL). To the mixture is added bis(trimethylsilyl)acetamide (2 mL) and trimethylsilyl trifluoromethanesulfonate (4 g, 0.02 mol), and the mixture is stirred overnight at room temperature. The solvent is removed *in vacuo*, and the residue is partitioned between chloroform (50 mL) and cold, saturated sodium bicarbonate solution (50 mL). The aqueous layer is washed with chloroform (50 mL). The combined chloroform layers are dried over sodium sulfate and concentrated *in vacuo* to give a crude mixture of 61 and 52 (X = NHAc, R = H, R''' = CH₃). After evaporation of the solvent *in vacuo*, the residue is dissolved in methanolic ammonia (100 mL), and the mixture kept standing overnight at room temperature. The mixture is flash evaporated, and the residue which contains 61 (R = H, X = NH₂, R³ = R⁴ = H) and 53 (R = H, X = NH₂, R' = R'' = H) is dissolved in 15 mL of 30% methanol applied to a column (3 x 25 cm) of Bio-Rad AGI 2X (OH) pre-equilibrated with 30% aqueous methanol. The column is eluted with 30% methanol, and two UV absorbing fractions are collected. Each fraction is concentrated *in vacuo*, and the residue is crystallized from methanol. The first fraction contains 2'-deoxycytidine (61, R = R³ = R⁴ = H, X = NH₂), which is isolated by evaporation, followed by crystallization of the residue from ethanol, 1.6 g, (36 %), mp 200-202 °C. From the second fraction, 2'-deoxy-α-cytidine (53, X = NH₂, R = R' = R''= H) (2.0 g, 44 %) is obtained, mp 195-197 °C. The melting point reported for 2'-deoxy-α-cytidine is 192-193 °C.(Fox, J. J.; Yung, N. C.; Wempen, I.; Hoffer, M. *J*. *Am. Chem. Soc.* **1961**, *83*, 4066). ¹H-NMR shows a distinct double doublet for H-1' at δ 6.15 (J_{1',2'} = 2.3, J_{1',2"} = 7.4 Hz).

This invention has been described with reference to its preferred embodiments. Variations and modifications of the invention, will be obvious to those skilled in the art from the foregoing detailed description of the invention.

Further embodiments of the present invention are as follows :
1. A process for the preparation of a 2'-deoxy-β-L-nucleoside comprising the steps of:
   a) selectively activating a 2'-hydroxyl of a β-L-nucleoside to form an activated nucleoside substituted at the 2'-position with a substituent selected from the group consisting of the following:

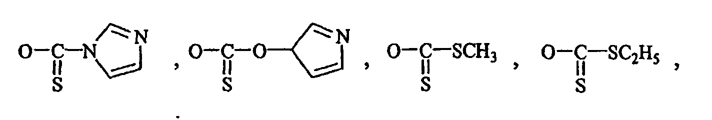

      O-S(=O)ₙ-R⁵ or O-C(=O)-R⁵;

      wherein n is 1 or 2 and R⁵ is a hydrogen, an alkyl or aryl moiety; and
   b) reducing the product of step a with a reducing agent to form a 2'-deoxy-L-nucleoside.
2. The process of embodiment 1 wherein the reducing agent is tri-butyltin-hydride.
3. A process for the preparation of a 2'-deoxy-L-nucleoside comprising the steps of:
   a) preparing a 2'-halo-L-nucleoside of the following formula: 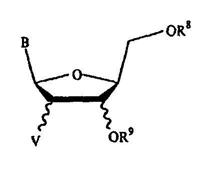 wherein B is a heterocyclic or heteroaromatic base,
      R⁸ and R⁹ are independently hydrogen or a suitable protecting group,
      V is a halogen; and
   b) reducing the 2'-halo-L-nucleoside to a 2'-deoxy-L-nucleoside.
4. The process of embodiment 3 wherein the preparation of the 2'-halo-L-nucleoside comprises the steps of:
   a) selectively activating a 2'-hydroxyl of a L-nucleoside to form an activated nucleoside substituted at the 2'-position with a substituent selected from the group consisting of the following:

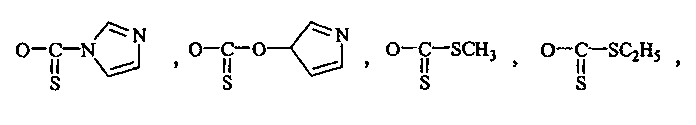

      O-S(=O)ₙ-R⁵ or O-C(=O)-R⁵;

      wherein n and R⁵ are previously defined; and
   b) substituting the 2'-moiety with a halide to give the 2'-halo-L-nucleoside.
5. The process of embodiment 3 wherein the synthesis of the 2'-halo-L-nucleoside further comprises the following steps:
   a) preparing from a suitably protected and activated L-nucleoside an anhydro-L-nucleoside of the following formula: 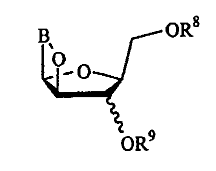 wherein B, R⁸ and R⁹ are previously defined; and
   b) substituting the 2'-moiety with a halide to give a 2'-halo-L-nucleoside.
6. The process of embodiment 5 wherein the synthesis of the anhydro-L-nucleoside further comprises the following steps:
   a) selectively activating a 2'-hydroxyl of a L-nucleoside to form an activated nucleoside substituted at the 2'-position with a substituent selected from the group consisting of the following:

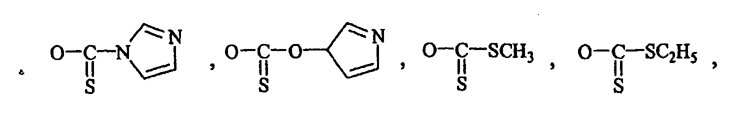

      O-S(=O)ₙ-R⁵ or O-C(=O)-R⁵;

      wherein n and R⁵ are previously defined; and
   b) intra-molecularly cyclizing the nucleoside with the heterocyclic or heteroaromatic base to form the anhydro-L-nucleoside.
7. The process of embodiment 3 wherein, the reduction of the 2'-halo-L-nucleoside comprises reducing via hydrogenolysis to obtain the 2'-deoxy-L-nucleoside.
8. A process for the preparation of a 2'-deoxy-L-nucleoside comprising the steps of:
   a) preparing from a suitably protected and activated L-nucleoside a 2'-S-substituted-L-nucleoside of the following formula: 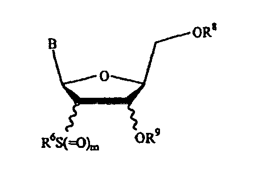 wherein B, R⁸ and R⁹ are previously defined,
      R⁶ is an alkyl or aryl, and m is 0, 1 or 2; and
   b) reducing the 2'-S-substituted-L-nucleoside to a 2'-deoxy-L-nucleoside.
9. The process of embodiment 8 wherein, the synthesis of the 2'-S-substituted-L-nucleoside further comprises the steps of:
   a) selectively activating a 2'-hydroxyl of a L-nucleoside to form an activated nucleoside substituted at the 2'-position with a substituent selected from the group consisting of the following:

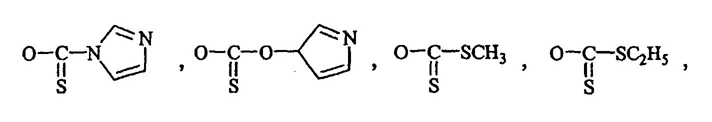

      O-S(=O)ₙR⁵ or O-C(=O)-R⁵;

      wherein n and R⁵ are previously defined; and
   b) substituting the 2'-moiety with a ⁻S(=O)ₘR⁶ or ⁻S(=O)ₘR⁶ equivalent to give the 2'-S-substituted-L-nucleoside.
10. The process of embodiment 9 wherein ⁻S(=O)ₘR⁶ is thioacylate or thiobenzoate.
11. The process of embodiment 9 wherein ⁻S(=O)ₘR⁶ is thioacetate.
12. The process of embodiment 8 wherein, the preparation of 2'-S-substituted-L-nucleoside further comprises the steps of:
   a) selectively activating a 2-hydroxyl of a L-furanose to form an activated furanose substituted at the 2'-position with a substituent selected from the group consisting of the following:

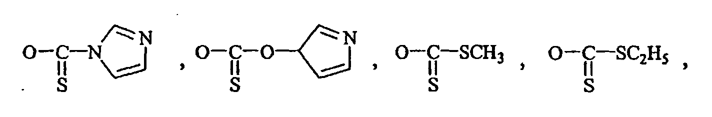

      O-S(=O)ₙ-R⁵ or O-C(=O)-R⁵;

      wherein n and R⁵ are previously defined;
   b) substituting the 2-moiety with ⁻S(=O)ₘR⁶ or ⁻S(=O)ₘR⁶ equivalent to obtain a 2-S-substituted-L-furanose; and
   c) coupling the appropriately activated 2-S-substituted-L-furanose with a heterocyclic or heteroaromatic base to form a 2'-S-substituted-L-nucleoside
13. The process of embodiment 12 wherein ⁻S(=O)ₘR⁶ is thioacylate or thiobenzoate.
14. The process of embodiment 12 wherein ⁻S(=O)ₘR⁶ is thioacetate.
15. The process of embodiment 12 wherein the preparation of the suitably protected 2-hydroxyl-L-furanose does not comprise using mercury amalgam.
16. The process of embodiment 15 wherein the preparation of the suitably protected L-furanose is the synthesis of a suitably protected L-arabinose which further comprises the following steps:
   a) preparing a 5-O-silylated-L-arabinose;
   b) reacting the 5-O-silylated-L-arabinose with acetone and acid, optionally with a drying agent such as anhydrous copper sulfate, to obtain a 5-O-silylated-1,2-O-isopropylidene-L-arabinose;
   c) deprotection of the 5-O-silylated-1,2-O-isopropylidene-L-arabinose at the 5-position using fluoride ion to obtain a 1,2-O-isopropylidene-L-arabinose;
   d) protecting the 4 and 5 position of 1,2-O-isopropylidene-L-arabinose to obtain a 1,2-O-isopropylidene-4-O-protected-5-O-protected'-L-arabinose; and
   e) reaction of 1,2-O-isopropylidene-4-O-protected-5-O-protected'-L-arabinose with an alcohol to obtain a 1-O-protected"-4-O-protected-5-O-protected'-L-arabinose with a free 2'-hydroxyl.
17. The process of embodiment 8 wherein the preparation of 2'-S-substituted-L-nucleoside further comprises the following steps:
   a) preparing from a suitably protected and activated L-nucleoside an anhydro-L-nucleoside of the following formula: 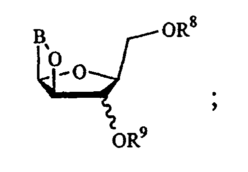 wherein B, R⁸ and R⁹ are previously defined; and
   b) substituting the 2'-moiety with ⁻S(=O)ₘR⁶ or ⁻S(=O)ₘR⁶ equivalent to obtain a 2'-S-substituted-L-nucleosides.
18. The process of embodiment 17 wherein the preparation of the anhydro-L-nucleoside further comprises the following steps:
   a) selectively activating a 2'-hydroxyl of a L-nucleoside to form an activated nucleoside substituted at the 2'-position with a substituent selected from the group consisting of the following:

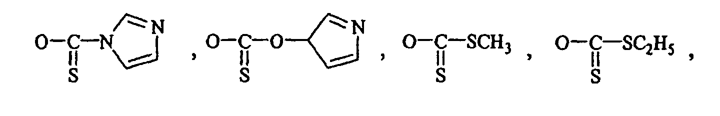

      O-S(=O)ₙ-R⁵ or O-C(=O)-R⁵;

      wherein n and R⁵ are previously defined; and
   b) intra-molecular cyclizing of the nucleoside with the heterocyclic or heteroaromatic base to form the anhydro-L-nucleoside.
19. The process of embodiment 17 wherein ⁻S(=O)ₘR⁶ is thioacylate or thiobenzoate.
20. The process of embodiment 17 wherein ⁻S(=O)ₘR⁶ is thioacetate.
21. The process of embodiment 8 wherein, the reduction of the cyclonucleoside comprises the step of reducing via desulfurization with Raney Nickel to obtain a 2'-deoxy-L-nucleoside.
22. A process for the preparation of a 2'-deoxy-L-nucleoside comprising the following steps:
   a) preparing from a suitably protected and activated L-furanose a 2-S-substituted-2-deoxy-L-furanose of the following formula: 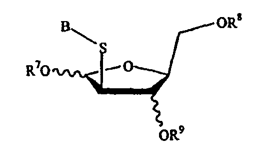 wherein B, R⁸ and R⁹ are previously defined;
      R⁷ is a suitable protecting group;
   b) cyclizing the 2-S-substituted-2-deoxy-L-furanose to form a cyclonucleoside of the following formula: 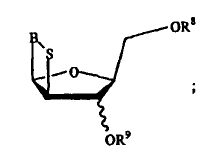 and
   c) reducing the cyclonucleoside to a 2'-deoxy-L-nucleoside.
23. The process of embodiment 22 wherein the preparation of the 2-S-substituted-2-deoxy-L-furanose comprises the following step:
   a) reacting an appropriately protected and activated L-furanose with a thio-heterocyclic or thio-heteroaromatic base.
24. The process of claim 22 wherein the preparation of the 2-S-substituted-2-deoxy-L-furanose further comprises the following steps:
   a) preparing from a suitably protected and activated L-furanose a 2-thiol-2-deoxy-L-furanose of the following formula: 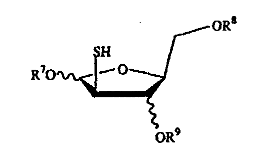 wherein B, R⁷, R⁸ and R⁹ are previously defined; and
   b) coupling the 2-thiol-2-deoxy-L-furanose with a halo-hetercyclic or halo-heteroaromatic base to form a 2-S-substituted-2-deoxy-L-furanose of the following formula: 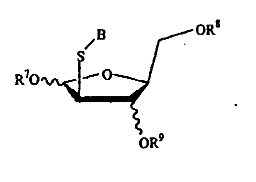
25. The process of embodiment 22 wherein, the reduction of the cyclonucleoside comprises the step of reducing via desulfurization with Raney Nickel to obtain the 2'-deoxy-L-nucleoside.
26. A process for the preparation of a 2'-deoxy-L-nucleoside comprising the steps of:
   a) preparing from a suitably protected and activated L-nucleoside a 2'-carbonyl-L-nucleoside of the following formula: 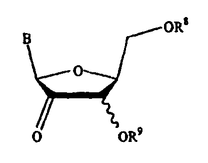 wherein B, R⁸ and R⁹ are previously defined; and
   b) reducing the 2'-carbonyl-L-nucleoside to a 2'-deoxy-nucleoside.
27. The process of embodiment 26 wherein, the reduction of the 2'-carbonyl-L-nucleoside comprises using hydrazine hydrate and hydroxide as the reducing agent.
28. The process of claim 26 wherein, the reduction of the 2'-carbonyl-L-nucleoside comprises the step of using tosylhydrazine followed by a borane or borohydride and optionally with an acetate as the reducing agent.
29. The process of embodiment 28 wherein the borane is catechol borane reacted with sodium acetate.
30. The process of embodiment 28 wherein the borohydride is sodium borohydride.
31. The process of embodiment 28 wherein the borohydride is NaBH₃CN.
32. A process for the preparation of a 2'-deoxy-L-nucleoside comprising the steps of:
   a) preparing a suitably protected 2'-deoxy-α-D-nucleoside;
   b) oxidizing the 2'-deoxy-α-D-nucleoside to give an aldehyde of the following formula: 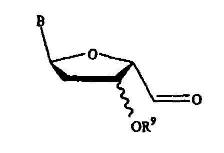 wherein B and R⁹ are previously defined;
   c) converting the aldehyde to an enolacetate or enamine of the following formula: 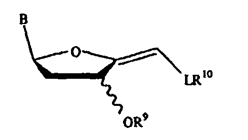 wherein L is O or N; R¹⁰ is -C(=O)R¹¹ if L is O or R¹¹R¹² if L is N; and R¹¹ and R¹² are independently an alkyl or aryl group;
   d) hydrogenating the enolactate or enamine to obtain a 2'-deoxy-β-L-nucleoside of the following formula: 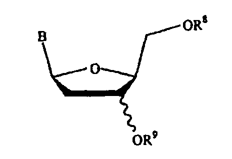 wherein B, R⁸ and R⁹ are previously defined; and
   e) optionally epimerizing the 3' position.
33. The process of embodiment 32 wherein the preparation of the 2'-deoxy-α-D-nucleoside further comprises epimerizing a corresponding, optionally protected, 2'-deoxy-β-D-nucleoside.
34. The process of embodiment 32 wherein the preparation of the 2'-deoxy-α-D-nucleoside further comprises the following steps:
   a) selectively activating a 2'-hydroxyl of a α-D-nucleoside to form an activated nucleoside substituted at the 2'-position with a substituent selected from the group consisting of the following:

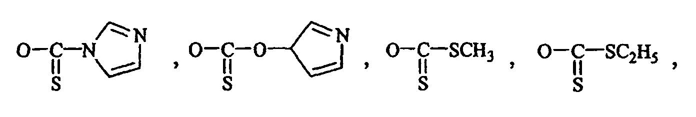

      O-S(=O)ₙR⁵ or O-C(=O)-R⁵;

      wherein n and R⁵ are previously defined; and
   b) reducing the 2'-moiety with a hydride to give the 2'-deoxy-α-D-nucleoside.
35. The process of embodiment 34 wherein the hydride is generated from tri-butyltinhydride.
36. The process of embodiment 32 wherein the preparation of the 2'-deoxy-α-D-nucleoside further comprises the steps of:
   a) selectively activating a 2'-hydroxyl of a α-D-nucleoside to form an activated nucleoside substituted at the 2'-position with a substituent selected from the group consisting of the following:

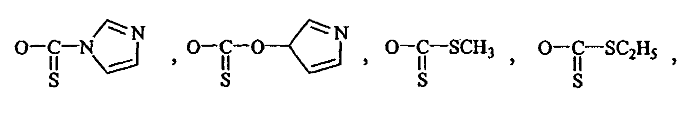

      O-S(=O)ₙR⁵ or O-C(=O)-R⁵;

      wherein n and R⁵ are previously defined;
   b) substituting the 2'-moiety with a halide to give a 2'-halo-α-D-nucleoside; and
   c) reducing the 2'-halo-nucleoside to give the 2'-deoxy-α-D-nucleoside.
37. The process of embodiment 36 wherein the reduction is accomplished via hydrogenolysis.
38. The process of embodiment 32 wherein the preparation of the 2'-deoxy-α-D-nucleoside further comprises the following steps:
   a) selectively activating a 2'-hydroxyl of a α-D-nucleoside to form an activated nucleoside substituted at the 2'-position with a substituent selected from the group consisting of the following:

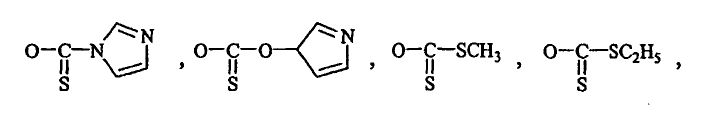

      O-S(=O)ₙR⁵ or O-C(=O)-R⁵;

      wherein n and R⁵ are previously defined;
   b) substituting the 2'-moiety with a ⁻S(=O)ₘR⁶ or ⁻S(=O)ₘR⁶ equivalent, where R⁶ is an alkyl or aryl moiety, to give a 2'-S-substituted-α-D-nucleoside; and
   c) reducing the 2'-S-substituted-α-D-nucleoside to a 2'-deoxy-α-D-nucleoside.
39. The process of embodiment 38 wherein ⁻S(=O)ₘR⁶ is thioacylate or thiobenzoate.
40. The process of embodiment 38 wherein ⁻S(=O)ₘR⁶ is thioacetate.
41. The process of embodiment 38 wherein the reduction is accomplished via desulfurization using Raney nickel to obtain the 2'-deoxy-α-D-nucleoside.
42. A process for the preparation of a 2'-deoxy-L-nucleoside comprising epimerizing the C-4' position of a pyrimidine α-L-nucleoside.
43. A process for the preparation of a 2'-deoxy-L-nucleoside containing a purine comprising base exchange with a pyrimidine β-L-nucleoside with a purine.
44. The process of embodiment 1, 3 or 8 wherein the preparation of a compound of the following formula (A): 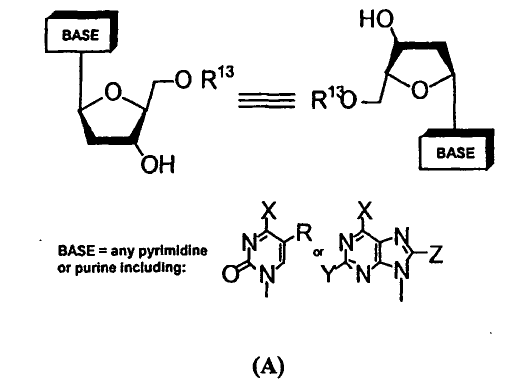 wherein
   X and Y are independently H, OH, OR, SH, SR¹, NH₂, NHR¹ or NR¹R²;
   Z is hydrogen, halogen, CN or NH₂;
   R is hydrogen, lower alkyl, aralkyl, halogen, NO₂, NH₂, NHR³, NR³R⁴, OH, OR³, SH, SR³, CN, CONH₂, CSNH₂, CO₂H, CO₂R³, CH₂CO₂H, CH₂CO₂R³, CH=CHR³, CH₂CH=CHR³ or C≡CR³;
   R¹, R², R³ and R⁴ are independently a lower alkyl, e.g., methyl, ethyl, propyl, butyl, and alkyl possessing 6 or less carbons, in cyclic, branched or straight chains, unsubstituted or substituted wherein the alkyl bears one, two, or more substituents, including but not limited to, amino, carboxyl, hydroxy and phenyl;
   R¹³ is hydrogen, alkyl, acyl, phosphate (monophosphate, diphosphate, triphosphate, or stabilized phosphate) or silyl; and
   further comprising condensing 2-O-acetyl-1,3,5-tri-O-benzoyl-β-L-ribofuranose with a purine or pyrimidine base, followed by selective halogenation or thiocarhonylation at the 2'-OH group and subsequent reduction.
45. The process of embodiment 8 wherein the preparation of the compound of the above formula (A) further comprises converting L-ribose to a 2-deoxy-2-S-acetyl-2-thio-L-ribose derivative which is then condensed with a purine or pyrimidine base to obtain only the desired β-nucleoside followed by desulfurization.
46. The process of embodiment 22 wherein the preparation of the compound of the above formula (A) further comprises synthesizing a 2-thiol-L-arabinose derivative from L-ribose, then linking a purine or pyrimidine base to the sulfur, forming a glycosyl C-N bond between the sugar and the base to obtain only the desired β-anomer, and reducing by desulfurization.
47. The process of embodiment 1, 3 or 8 wherein the preparation of the compound of the above formula (A) further comprises condensing a 2,3,5-tri-O-protected-L-xylose derivative followed by removal of the 2'-OH group by either halogenation or thiocarbonylation procedure. The 3'-OH group is then of epimerized to obtain the desired 2'-deoxy-β-L-nucleosides.
48. The process of embodiment 5 or 17 wherein the preparation of the compound of the above formula (A) containing a pyrimidine base further comprises condensing a 2,3,5-tri-O-protected-L-ribose with a pyrimidine, followed by deoxygenation of 2'-OH by way of 2,2'-anhydronucleoside formation.
49. The process of embodiment 8 or 22 wherein the preparation of the compound of the above formula (A) containing a purine base further comprises condensing a 2,3,5-tri-O-protected-L-xylose with a purine, followed by deoxygenating the 2'-OH by substitution with sulfur and reducing by desulfurization.
50. The process of embodiment 26 wherein, the preparation of the compound of the above formula (A) containing a purine base further comprises condensing a 2,3,5-tri-O-protected-L-xylose with a purine, oxygenating the 2'-OH into a keto group and followed by removing the keto group by the Wolf-Kischner reduction or a similar modification.
51. The process of embodiment 26 wherein the preparation of the compound of the above formula (A) containing a pyrimidine base further comprises condensing a 2,3,5-tri-O-protected-L-xylose with a pyrimidine, oxygenating the 2'-OH into a keto group and followed by removing the keto group by the Wolf-Kischner reduction or a similar modification..
52. The process of embodiment 3, 5 or 8 wherein the preparation of the compound of the above formula (A) comprises condensing a 2,3,5-tri-O-protected-L-arabinose with a purine or pyrimidine; followed by deoxygenating the 2'-OH via substitution of the OH or thiocarbonylation and subsequent reduction.
53. The process of embodiment 15 wherein the preparation further comprises synthesizing a crystalline 3,5-di-O-(p-methylbenzoyl)-2-deoxy-β-L-ribofuranosyl chloride though a novel process from L-arabinose.
54. The process of embodiment 32 wherein the preparation of the compound of the above formula (A) containing a purine base further comprises condensing a 2,3,5-tri-O-protected-D-arabinose with a purine to obtain the corresponding β-D-nucleoside, then converting it into the desired β-L-arabino-nucleoside by inversion of the 4'-hydroxymethyl group.
55. The process of embodiment 32 wherein the preparation of the compound of the above formula (A) further comprises synthesizing the L-nucleoside from a natural β-D-nucleoside by successive anomerization and C-4'epimerization.

## Claims

1. A process for the preparation of a 2'-deoxy-L-nucleoside comprising the following steps:
a) preparing from a suitably protected and activated L-furanose a 2-S-substituted-2-deoxy-L-furanose of the following formula: 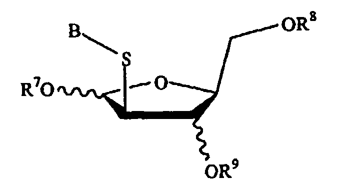 wherein B is a heterocyclic or heteroaromatic base, R⁸ and R⁹ are independently hydrogen or a suitable protecting group; R⁷ is a suitable protecting group;
b) cyclizing the 2-S-substituted-2-deoxy-L-furanose to form a cyclonucleoside of the following formula: 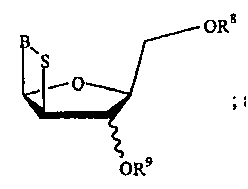 and
c) reducing the cyclonucleoside to a 2'-deoxy-L-nucleoside.

2. The process of claim 1 wherein the preparation of the 2-S-substituted-2-deoxy-L furanose comprises the following step:
a) reacting an appropriately protected and activated L-furanose with a thio-heterocyclic or thio-heteroaromatic base.

3. The process of claim 1 wherein the preparation of the 2-S-substituted-2-deoxy-L furanose further comprises the following steps:
a) preparing from a suitably protected and activated L-furanose a 2-thiol-2-deoxy-L-furanose of the following formula: 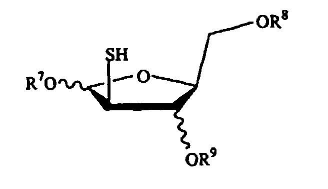 wherein B, R⁷, R⁸ and R⁹ are previously defined; and
b) coupling the 2-thiol-2-deoxy-L-furanose with a halo-hetercyclic or halo-heteroaromatic base to form a 2-S-substituted-2-deoxy-L-furanose of the following formula:

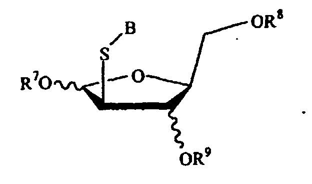

4. The process of claim 1 wherein, the reduction of the cyclonucleoside comprises the step of reducing via desulfurization with Raney Nickel to obtain the 2'-deoxy-L-nucleoside.

5. A process for the preparation of a 2'-deoxy-L-nucleoside containing a purine comprising base exchange with a pyrimidine β-L-nucleoside with a purine.

6. The process of claim 1 wherein the preparation of the compound of the above formula (A) further comprises synthesizing a 2-thiol-L-arabinose derivative from L-ribose, then linking a purine or pyrimidine base to the sulfur, forming a glycosyl C-N bond between the sugar and the base to obtain only the desired β-anomer, and reducing by desulfurization.

7. The process of claim 1 wherein the preparation of the compound of the above formula (A) containing a purine base further comprises condensing a 2,3,5-tri-O-protected-L-xylose with a purine, followed by deoxygenating the 2'-OH by substitution with sulfur and reducing by desulfurization.
